(19) Europäisches Patentamt
European Patent Office
Office européen des brevets

(11) Publication number: 0 264 773 A1

(12) EUROPEAN PATENT APPLICATION

(21) Application number: 87114904.3

(22) Date of filing: 13.10.87

(51) Int. Cl.4: C07D 487/04 , A61K 31/495 , //(C07D487/04,239:00,231:00)

(30) Priority: 16.10.86 US 919730
16.10.86 US 919731

(43) Date of publication of application: 27.04.88 Bulletin 88/17

(84) Designated Contracting States: AT BE CH DE ES FR GB GR IT LI NL SE

(71) Applicant: AMERICAN CYANAMID COMPANY
1937 West Main Street P.O. Box 60
Stamford Connecticut 06904-0060(US)

(72) Inventor: Tseng, Shin Shyong
232 Windmill Court
Bridgewater New Jersey 08807(US)
Inventor: Dusza, John Paul
24 Convent Road
Nanuet New York 10954(US)
Inventor: Brabander, Herbert Joseph
10 Robert Place
Nanuet New York 10954(US)
Inventor: Epstein, Joseph William
19, Briarwood Avenue
Monroe New York 10954(US)

(74) Representative: Wächtershäuser, Günter, Dr.
Tal 29
D-8000 München 2(DE)

(54) 4,5-dihydro and 4,5,6,7-tetrahydropyrazolo[1,5-a]pyrimidines.

(57) A compound of the formula:

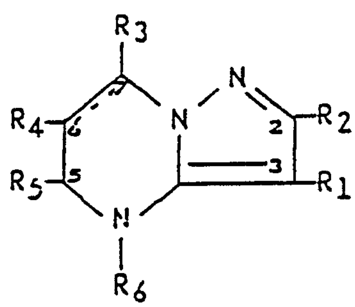

Ia or Ib

wherein ---may represent the presence of a double bond between the $C_6$ and $C_7$ position, Ia, or the absence of a double bond between the $C_6$ and $C_7$ position, Ib; $R_1$ is hydrogen, bromo, chloro, carbamoyl, carboxamido, ethylcarboxylate, carboxyl, carboxyalkoxyl where alkoxy is ($C_1$-$C_3$), cyano, -CO-$CF_3$, COONa,

EP 0 264 773 A1

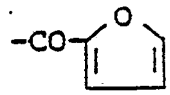,

$-CO-C(CH_3)_3$, or

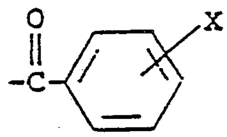

where X is hydrogen, cyano, halogen or nitro; $R_2$, $R_4$ and $R_5$ are hydrogen or lower alkyl($C_1$-$C_3$); $R_3$ ia hydrogen, alkyl($C_1$-$C_3$),

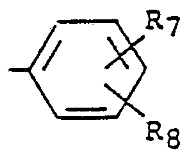

where $R_7$ and $R_8$ may be the same or different and are hydrogen, halogen, alkyl($C_1$-$C_3$), nitro, alkoxy($C_1$-$C_3$), trifluoromethyl, N-alkyl($C_1$-$C_3$)-N-alkanoxyl($C_1$-$C_3$)-amino, acetylamino or N-alkylacetylamino where alkyl is ($C_1$-$C_3$), and $R_3$ may be a monovalent radical of 3-thienyl, 2-pyridinyl, 3-pyridinyl or 4-pyridinyl, either of said pyridinyl radicals being optionally substituted with an alkyl radical $R_9$, where alkyl is ($C_1$-$C_4$), and the structures of the monovalent 2-pyridinyl, 3-pyridinyl and 4-pyridinyl moieties are depicted respectively as:

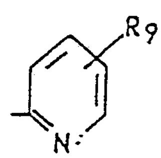 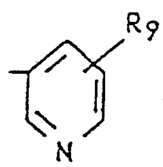 and 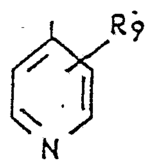 ;

$R_6$ is hydrogen, alkyl ($C_1$-$C_3$) or

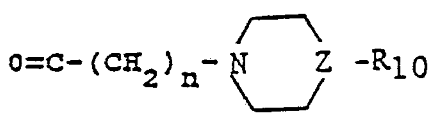

, where n is an integer from 1 to 4 inclusive; and Z is CH-, N-and -O-such that when Z is -CH, $R_{10}$ is hydrogen, ethoxycarbonyl, phenyl, phenylmethyl, phenylmethyl($C_1$-$C_6$)alkylenyl, when z is -O-, $R_{10}$ is nil and when Z is N-, $R_{10}$ is hydrogen, alkyl-($C_1$-$C_3$), alkenyl($C_2$-$C_3$), alkynyl($C_2$-$C_3$), cycloalkyl($C_3$-$C_6$), dimethylaminoalkyl($C_1$-$C_3$), hydroxyalkyl($C_1$-$C_3$), ethylcarboxylate, alkyl($C_1$-$C_3$)carbonyl, phenyl, benzyl, mono-or disubstituted benzyl [wherein the substituents are halogen, alkoxy($C_1$-$C_3$), alkyl ($C_1$-$C_3$), and trifluoromethyl], benzoyl, 4-chlorophenylmethyl, 1,3-benzodioxol-5-yl-methyl, 1,3-benzodioxol-5-yl, 2-furanyl-carboxyl, 2-pyrimidinyl, 2-pyridinyl, 4-morpholinyl-2-oxoethyl, N-(1-methylethyl)-2-oxoethyl, 1-pyrrolidinyl-2-oxoethyl, bis(4-fluorophenyl)-methyl, 2-cyclohexylethyl, phenylcarboxamido, mono-and di-substituted phenylcarboxamido [wherein the substituents are trifluoromethyl, halogen and alkyl ($C_1$-$C_3$)], adamantanoyl, 3-phenoxypropyl, mono-and disubstituted phenyl [wherein the substituents are halogen, trifluoromethyl, alkoxy($C_1$-$C_3$) and alkyl($C_1$-$C_3$)], 5-chloro-2-methoxy phenylacetamide and (2-oxo-1-pyrrolidinyl)-2-butynyl and the pharmacologically acceptable acid addition salts thereof.

# 4,5-DIHYDRO AND 4,5,6,7-TETRAHYDROPYRAZOLO[1,5-a]-PYRIMIDINES

## BRIEF SUMMARY OF THE INVENTION

The invention relates to new organic compounds and more particularly is concerned with novel 4,5-dihydro and 4,5,6,7-tetrahydropyrazolo[1,5-a]pyrimidines useful as anxiolytic agents, antihypertensive agents and as agents for the treatment of senile dementia, cognitive and related neural behavioral problems, antidepressant agents, in mammals, or as intermediates. The compounds of the present invention may be represented by the following formula:

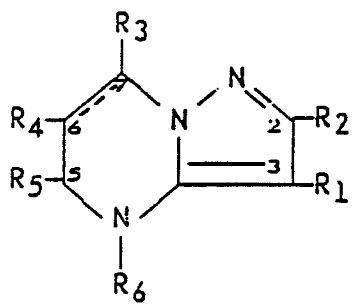

Ia or Ib

wherein ---may represent the presence of a double bond between the $C_6$ and $C_7$ position, Ia, or the absence of a double bond between the $C_6$ and $C_7$ position, Ib; $R_1$ is hydrogen, halogen, carbamoyl, carboxamido, ethyl carboxylate, carboxyl, carboxyalkoxyl where alkoxyl is ($C_1$-$C_3$), cyano, -CO-$CF_3$, COONa,

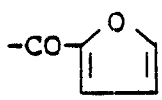

,

-CO-$C(CH_3)_3$, or

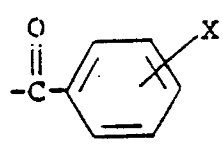

where X is hydrogen, cyano, halogen or nitro; $R_2$, $R_4$ and $R_5$ may be hydrogen or lower alkyl ($C_1$-$C_3$); $R_3$ is hydrogen, alkyl($C_1$-$C_3$),

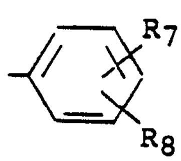

wherein $R_7$ and $R_8$ may be the same or different and are hydrogen, halogen, alkyl($C_1$-$C_3$), nitro, alkoxy($C_1$-$C_3$), trifluoromethyl, N-alkyl($C_1$-$C_3$)-N-alkanoyl($C_1$-$C_3$)-amino, acetylamino or N-alkylacetylamino where alkyl is ($C_1$-$C_3$), and $R_3$ may be a monovalent radical of 3-thienyl, 2-pyridinyl, 3-pyridinyl and 4-pyridinyl, either of said pyridinyl radicals being optionally substituted with an alkyl radical $R_9$, where alkyl is ($C_1$-$C_4$), and the structures of the monovalent 2-pyridinyl, 3-pyridinyl and 4-pyridinyl moieties are depicted respectively as:

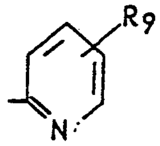

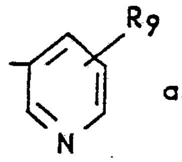

and

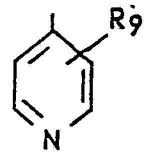

; $R_6$ is hydrogen, alkyl($C_1$-$C_3$) or

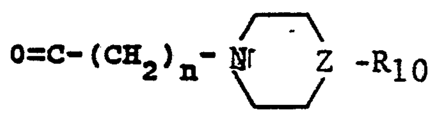

, where n is an integer from 1 to 4 inclusive; and Z is CH-, N-and -O-such that when Z is -CH, $R_{10}$ is hydrogen, ethoxycarbonyl, phenyl, phenylmethyl, phenylmethyl($C_1$-$C_6$)alkylenyl, when Z is -O-, $R_{10}$ is nil and when Z is N-, $R_{10}$ is hydrogen, alkyl-($C_1$-$C_3$), alkenyl($C_2$-$C_3$), alkynyl($C_2$-$C_3$), cycloalkyl($C_3$-$C_6$), dimethylaminoalkyl($C_1$-$C_3$), hydroxyalkyl($C_1$-$C_3$), ethyl carboxylate, alkyl($C_1$-$C_3$)carbonyl, phenyl, benzyl, mono-or disubstituted benzyl [wherein the substituents are halogen, alkoxy($C_1$-$C_3$), alkyl($C_1$-$C_3$), and trifluoromethyl], benzoyl, 4-chlorophenylmethyl, 1,3-benzodioxol-5-yl-methyl, 1,3-benzodioxol-5-yl, 2-furanyl-carboxyl, 2-pyrimidinyl, 2-pyridinyl, 4-morpholinyl-2-oxoethyl, N(1-methylethyl)-2-oxoethyl, 1-pyrrolidinyl-2-oxoethyl, bis(4-fluorophenyl)-methyl, 2-cyclohexylethyl, phenylcarboxamido, mono-and di-substituted phenylcarboxamido [wherein the substituents are trifluoromethyl, halogen and alkyl ($C_1$-$C_3$)], adamantanoyl, 3-phenoxypropyl, mono-and disubstituted phenyl [wherein the substituents are halogen, trifluoromethyl, alkoxy($C_1$-$C_3$) and alkyl($C_1$-$C_3$)], 5-chloro-2-methoxy phenylacetamide and (2-oxo-1-pyrrolidinyl)-2-butynyl and the pharmacologically acceptable acid addition salts thereof.

The invention includes 4,5-dihydro and 4,5,6,7-tetrahydropyrazolo[1,5-a]pyrimidines of the formula:

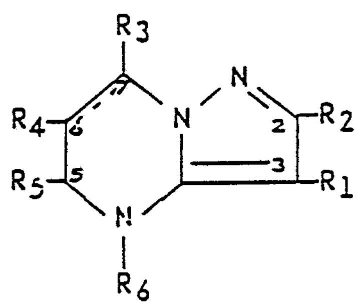

Ia or Ib

wherein ---may represent the presence of a double bond between the $C_6$ and $C_7$ position, Ia, or the absence of a double bond between the $C_6$ and $C_7$ position, Ib; $R_1$ is hydrogen, bromo, chloro, carbamoyl, carboxyl, carboxyalkoxyl where alkoxyl is ($C_1$-$C_3$), cyano, -CO-$CF_3$, COONa,

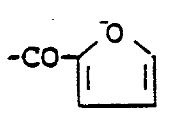,

-CO-C($CH_3$)$_3$, or

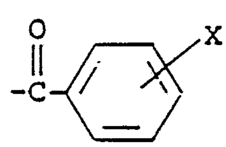

where X is hydrogen, cyano, halogen or nitro; $R_2$, $R_4$ and $R_5$ may be hydrogen or lower alkyl($C_1$-$C_3$); $R_3$ is hydrogen, alkyl($C_1$-$C_3$),

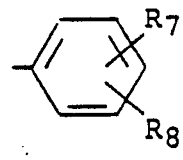

where $R_7$ and $R_8$ may be the same or different and are hydrogen, halogen, alkyl($C_1$-$C_3$), nitro, alkoxy($C_1$-$C_3$), trifluoromethyl, acetylamino or N-alkylacetylamino where alkyl is ($C_1$-$C_3$), and $R_3$ may also be a monovalent radical of 3-thienyl, 2-pyridinyl, 3-pyridinyl or 4-pyridinyl, either of said pyridinyl radicals being optionally substituted with an alkyl radical $R_9$, where alkyl is ($C_1$-$C_4$), and the structures of the monovalent 2-pyridinyl, 3-pyridinyl and 4-pyridinyl moieties are depicted respectively as:

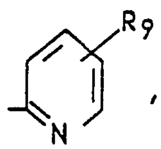 , 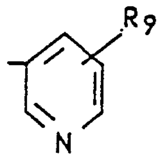 and 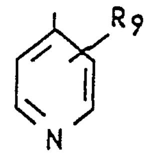

, $R_6$ is hydrogen or alkyl($C_1$-$C_3$) which are useful as anxiolytic agents, anti-hypertensive agents, antidepressant agents and intermediates for 4-[(substituted)alkylcarbonyl]4,5-dihydro and -4,5,6,7-tetrahydro-7-[-(substituted)-phenyl]pyrazolo[1,5-a]pyrimidine-3-carbonitriles.

The present invention also includes novel compositions of matter containing the above-defined compounds which are useful as anxiolytic agents, antihypertensive agents or antidepressant agents in mammals and the methods for meliorating axiety, treating hypertensive, alleviating depression in mammals and as agents for the treatment of senile dementia, cognitive and related neural behavioral problems in mammals.

The invention also comprises processes for the preparation of compounds within the scope of formulae Ia and Ib and 4-[(substituted)alkylcarbonyl]-4,5-dihydro and -4,5,6,7-tetrahydro-7-[(substituted)-phenyl]-pyrazolo-[1,5-a]pyrimidine-3-carbonitriles.

## Detailed Description of the Invention

The novel 4,5-dihydro and 4,5,6,7-tetrahydropyrazolo[1,5-a]pyrimidines of the invention are obtainable as colorless to yellow crystalline materials having characteristic melting points and absorption spectra. They are generally soluble in organic solvents such as lower alkanols, chloroform, tetrahydrofuran, N,N-dimethylformamide, dichloromethane, acetone and the like but are generally insoluble in water.

The novel 4,5-dihydro and 4,5,6,7-tetrahydropyrazolo[1,5-a]pyrimidines of the invention may be readily prepared as set forth in the following reaction schemes and Examples 1-63.

**SCHEME 1**

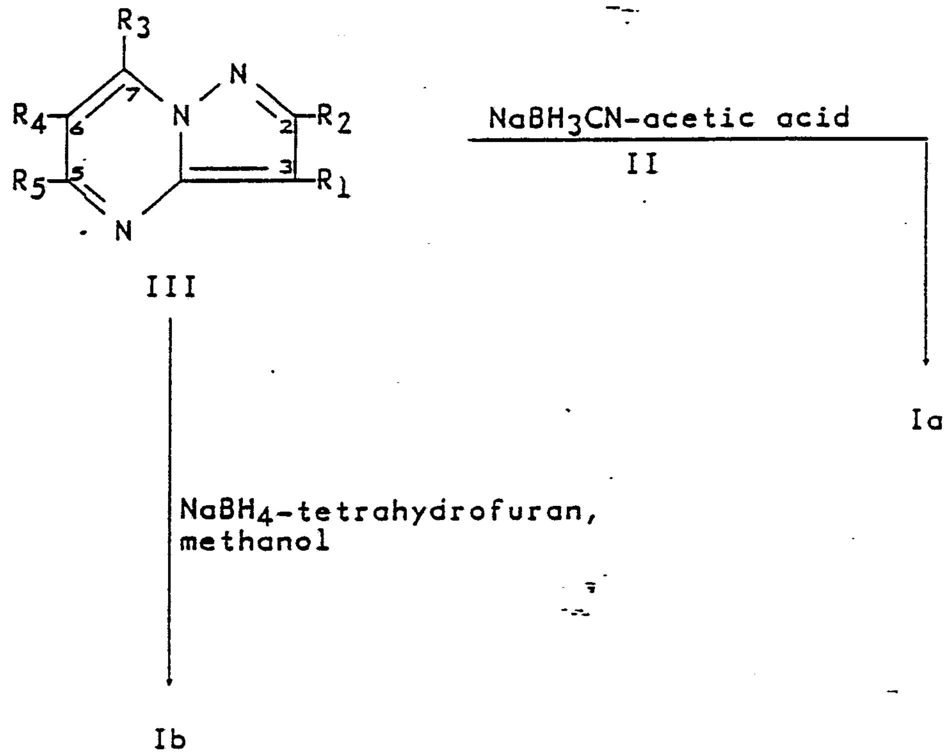


III

Ia

Ib

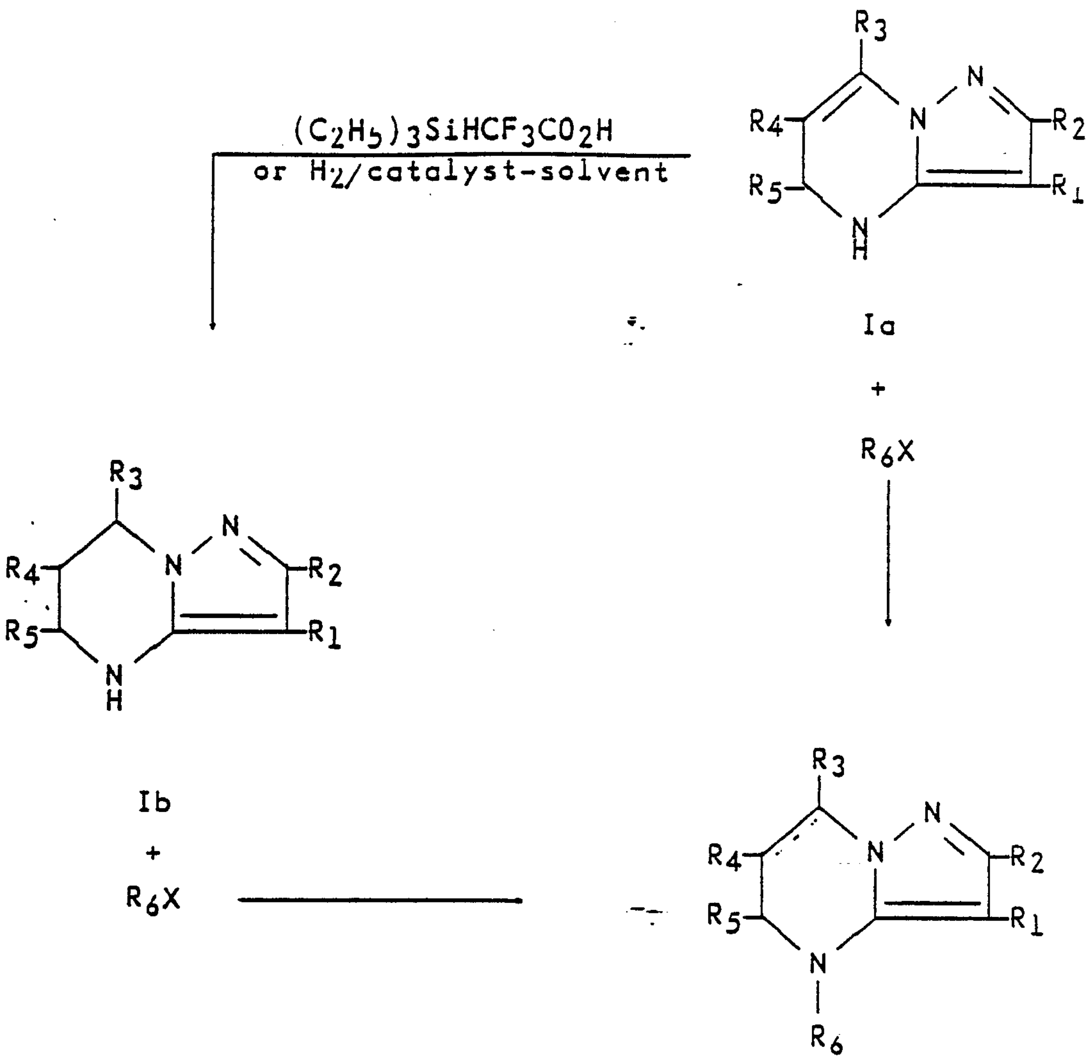


Ia

+

$R_6X$

Ib

+

$R_6X$

wherein $R_1$, $R_2$, $R_3$, $R_4$, $R_5$ and $R_6$ are as hereinabove defined and X is halogen.

As shown hereinabove (scheme 1) a pyrazolo[1,5-a]-pyrimidine III, with a hydrogen, phenyl, substituted phenyl, or heteroaryl group in the C-7 position and an electronwithdrawing group in the C-3 position is reacted with sodium cyanoborohydride II by stirring in glacial acetic acid under nitrogen in an ice bath for approximately one hour, then at room temperature for from 1-12 hours. The resulting precipitate is collected and washed with water, then is dissolved in an inert solvent such as dichloromethane or acetonitrile and the like and washed with saturated sodium bicarbonate. Separation and evaporation of the organic phase gives the crude dihydro product Ia which is recrystallized from a solvent such as isopropyl alcohol or acetonitrile and the like or from a mixture of solvents such as either-hexane, chloroform-methanol or N,N-dimethylformamide-acetonitrile and the like.

The dihydro product Ia is reduced with triethylsilane in trifluoroacetic acid at 60°C for 1-24 hours according to the procedure of Lanzilotti, et al., J. Org. Chem., 44 ,4809 (1979). The reaction mixture at ambient temperature is made slightly basic (pH 9) with aqueous potassium hydroxide to precipitate the product Ib which is then isolated and purified by crystallization or chromatography.

The reaction of the dihydro product Ia or the tetrahydro product Ib, when dissolved in a solvent such as N, N-dimethylformamide and the like, with an alkylating agent such as methyl iodide or dimethyl sulfate and the like in the presence of sodium hydride provides the corresponding product where $R_6$ is alkyl.

The pyrazolo[1,5-a]pyrimidines III are disclosed in U.S. Patents 4,178,449; 4,236,005 and 4,281,000 and in pending application, Serial No. 612,812, filed May 24, 1984 and allowed on January 10, 1985. They are prepared by condensation of 3-aminopyrazoles and substituted 3-amino-pyrazoles with 1,3-dicarbonyl compounds as described in J. Med. Chem., 18, 645 (1974); J. Med. Chem., 18, 460 (1975); J. Med. Chem., 20, 386 (1977; Synthesis, 637 (1982); and references contained therein.

The 7-aryl and 7-heteroaryl[1,5-a]pyrimidines which contain a 3-aroyl group, are synthesized by condensation of 1-aryl or 1-heteroaryl-1,3-dicarbonyl compounds with 3-amino-4-aroylpyrazoles.

It has been found that the use of sodium cyanoborohydride in acetic acid offers a simple, convenient, regioselective means for the reduction of pyrazolo[1,5-a]pyrimidines and derivatives thereof, bearing functional groups such as halogens, nitriles, amides amidines, esters, carboxylic acids and aryl ketones without reducing these groups and providing the final products in higher yield then obtained with other reducing agents. In fact, certain of the above described functional groups are known to be affected by the use of other reducing agents, with mixtures of products being formed which required the employment of time consuming separation techniques to obtain the desired product.

In rare instances the reduction of pyrazolo[1,5-a]pyrimidine derivatives with sodium cyanoborohydride in glacial acetic acid results in complete reduction to the tetrahydro form, whereas predominately the reduction procedure provides the dihydro product.

Another effective means for the reduction of pyrazolo[1,5-a]pyrimidines is concerned with the utilization of sodium borohydride in glacial acetic acid. This method although effective does not provide yields commensurate with those obtained by the use of sodium cyanoborohydride. This difference could be due to the fact that sodium cyanoborohydride is more stable in glacial acetic acid then is sodium borohydride. It has also been discovered that when sodium borohydride is reacted with a pyrazolo[1,5-a]pyrimidine derivative such as 7-(4-chlorophenyl)-5-methylpyrazolo[1,5-a]pyrimidine-3-carbonitrile or 7-(3-chlorophenyl)-5-methylpyrazolo[1,5-a]pyrimidine-3-carbonitrile in tetrahydrofuran:methanol (1:1) by stirring at room temperature for 24 hours or at 55°C for six hours the corresponding tetrahydro compound is obtained.

Still another means for the reduction of pyrazolo[1,5-a]pyrimidines or further reduction of 4,5-dihydropyrazolo[1,5-a]pyrimidines to the 4,5,6,7-tetrahydro form resides with the catalytic hydrogenation of the compound by shaking in a suitable apparatus, such as a Parr shaker, with a solvent such as ethyl acetate, N,N-dimethylformamide, or the like in the presence of a catalyst such as 10% palladium on carbon under an initial hydrogen pressure of from 5-30 lbs. until the uptake of hydrogen is complete followed by separation and purification of the reduction product by conventional means.

Certain of the novel 4,5-dihydro and 4,5,6,7-tetrahydropyrazolo[1,5-a]-pyramidines of the invention possess central nervous system activity at nontoxic doses and as such are useful as anxiolytic agents. They produce certain responses in standard tests with laboratory animals which are known to correlate well with relief of anxiety in human beings. The compounds, when tested pharmacologically, are found to have a desirable wide spread between doses producing anxiolytic activity and toxic symptoms.

The antianxiety properties of the novel 4,5-dihydro and 4,5,6,7-tetrahydropyrazolo[1,5-a]-pyramidines of the invention have been established in a test which indicates anxiolytic activity by the measure of protection from convulsions resulting from the administration of pentylenetetrazole. Single or graded dose levels of the test compounds were administered orally or intraperitoneally in a 2% starch vehicle containing 0.5% v/v polyethylene glycol and one drop of polysorbate 80, or distilled water and one drop of polysorbate 80 to groups of at least 4 rats. At 30 or 60 minutes, the rats were treated intravenously with pentylenetetrazole at a dose of 23 mg/kg of body weight. This dose is estimated to cause clonic seizures in 99% of unprotected rats. The test compounds are considered active if they protect 50% or more of the rats from clonic seizures. It has been reported [R. T. Hill and D. H. Tedeschi, "Animal Testing and Screening Procedures in Evaluating Psychotropic Drugs" in "An Introduction to Psychopharmacology", pp. 237-288 (Eds. R. R. Rech and K. E. Moore, Raven Press, New York, 1971) that there is a high degree of correlation between antagonism of pentylenetetrazole seizures in rats and antianxiety effects in higher warm-blooded animals. The results of this *in vivo* tests on representative compounds of the present invention are shown in Table I.

TABLE I

Protection Against Clonic Seizures Caused By Pentylenetetrazole In Rats

| Compound | Dose (mg/kg) | % of Rats Protected |
|---|---|---|
| 4,5-Dihydro-7-[3-(trifluoromethyl)-phenyl]pyrazolo[1,5-a]pyrimidine-3-carbonitrile | 25 | 100<br>50 |
| 4,5-Dihydro-7-phenylpyrazolo[1,5-a]-pyrimidine-3-carboxamide | 25 | 50 |
| [4,5-Dihydro-7-[3-(trifluoromethyl)-phenyl]pyrazolo[1,5-a]pyrimidin-3-yl]-phenylmethanone | 25 | 75 |

Another test used to assess antianxiety effects is a non-conditioned passive avoidance procedure described by J. R. Vogel, B. Beer and D. E. Cody, "A Simple and Reliable Conflict Procedure for Testing Antianxiety Agents", Psychopharmacologia, 21 :1-7 (1971). A conflict situation is induced in rats by a modification of this method.

Groups of 8 naive, Wistar strain male rats weighing 200-240 g each were deprived by water for 48 hours. The test compounds were administered in single or graded, oral doses, suspended in 2% starch with 5% polyethylene glycol in distilled water and one drop of polysorbate 80. Control animals received the vehicle alone. At 60 minutes each rat was placed in an individual clear plastic chamber. Tap water was available ad libitum from a nipple located in a black box off the main chamber. A 0.7 milliampere AC shocking current was established between the stainless steel grid floor and the tap. After 20 licks of non-shocked drinking, a 2 second shocking current was administered to the rat. This ratio of 20 licks of non-shocked drinking followed by a 2 second shock was contained for a total of 3 minutes. The number of shocks taken by each rat during the 3 minute interval was recorded and compared to a control group. The test compounds are considered active if the number of shocks received by the test group is significantly higher than the control group by the Mann-Whitney U test. That is, the test compounds are considered active if they result in the treated rat taking slightly more than double the number of shocks that the untreated rat will take. Results of this in vivo test on a representative compound of the present invention are given in Table II.

TABLE II

Conflict Procedure In Rats

| Compound | Dose (mg/kg) | Result (no. of shocks per 3 min.) |
|---|---|---|
| [4,5-Dihydro-7-[3-(trifluoromethyl)phenyl]pyrazolo[1,5-a]pyrimidin-3-yl]phenylmethanone | 25 | 19.1 |
| 4,5-Dihydro-4-methyl-7-[3-(trifluoromethyl)phenyl]pyrazolo-[1,5-a]pyrimidine-3-carbonitrile | 25 | 19.0 |

Still another test utilized for the determination of anxiolytic activity is the measurement of the ability of a test compound to inhibit the binding of tritiated benzodiazepines to brain-specific receptors of mammals. A modification of the method described by R. F. Squires, et al., Nature, 266 , No. 21:732 (April, 1977) and H. Mohler, et al ., Science, 198:849 (1977) was employed.

Male albino rats (Wistar strain, weighing 150-200 g each) were used. The test compounds were solubilized in dimethylformamide, acetic acid, ethanol or hydrochloric acid.

Whole cortex of rats was homogenized gently in 20 volumes of ice-cold 0.32 M sucrose, centrifuged twice at 1000 g for 10 minutes and then recentrifuged at 30,000 g for 20 minutes to produce a crude $P_2$-synaptosomal fraction. The $P_2$-fraction was either: (1) resuspended in twice the original volume in hypotonic 50 mM Tris.HCl (pH 7.4), or (2) resuspended in one-half the original volume in hypotonic 10 mM Tris.HCl (pH 7.4) and frozen (-20°C) until time of use. Frozen $P_2$ preparations were thawed and resuspended in four times the original homogenizing volume at time of assay.

The binding assay consisted of 300 µl of the $P_2$-fraction suspension (0.2-0.4 mg protein), 100 µl of test drug and 100 µl of $^3$H-diazepam (1.5 nM, final concentration) or $^3$H-flunitrazepam (1.0 nM, final concentration) which was added to 1.5 ml of 50 nM Tris.HCl (pH 7.4). Non-specific binding controls and total binding controls received 100 µl of diazepam (3 µM final concentration) and 100 µl of deionized water, respectively, in place of the test compound. Incubation for 30 minutes proceeded in ice and was terminated by filtration, under vacuum, through glass fiber filters. The filters were washed twice with 5 ml of ice-cold 50 mM Tris.HCl (pH 7.4) and placed in scintillation vials. After drying at 50-60°C for 30 minutes, 10 ml of diluent was added and the radioactivity determined in a scintillation counter.

Inhibition of binding was calculated by the difference between total binding and binding in the presence of test compound, divided by the total binding, X 100. Physiological activity can be shown by a test compound that inhibits $^3$H-benzodiazepine binding by 12% or more. Such in vitro activity is biologically relevant when the test compound also demonstrates statistically significant anxiolytic activity through in vivo studies.

The result of this in vitrotest on a representative compound of this invention is given in Table III.

TABLE III

Inhibition of the Binding of $^3H$ Benzodiazepine to Brain-Specific Receptors of Rats

| Compound | % Inhibition |
|---|---|
| 4,5-Dihydro-7-[3-(trifluoromethyl)phenyl]-pyrazolo[1,5-a]pyrimidine-3-carbonitrile | 15 |
| 4,5-Dihydro-7-phenylpyrazolo[1,5-a]pyrimidine-3-carbonitrile | 20 |
| 4,5-Dihydro-7-phenylpyrazolo[1,5-a]pyrimidine-3-carboxylic acid, ethyl ester | 54 |
| 4,5-Dihydro-7-[3-(trifluoromethyl)phenyl]-pyrazolo[1,5-a]pyrimidine-3-carboxylic acid, ethyl ester | 90 |
| [4,5-Dihydro-7-[3-(trifluoromethyl)phenyl]-pyrazolo[1,5-a]pyrimidin-3-yl]phenylmethanone | 76 |
| Phenyl[4,5,6,7-tetrahydro-7-(4-pyridinyl)-pyrazolo[1,5-a]pyrimidin-3-yl]methanone | 22 |
| 4,5-Dihydro-4-methyl-7-[3-(trifluoromethyl)-phenyl]pyrazolo[1,5-a]pyrimidine-3-carbonitrile | 12 |
| 2,2-Dimethyl-1-(4,5,6,7-tetrahydropyrazolo-[1,5-a]pyrimidin-3-yl]-1-propanone | 28 |
| 7-(3-Fluorophenyl)-4,5-dihydropyrazolo-[1,5-a]pyrimidine-3-carboxamide | 39 |
| 3-Chloro-4,5-dihydro-7-[3-(trifluoromethyl)-phenyl]pyrazolo[i,5-a]pyrimidine | 63 |
| 4,5-Dihydro-7-[3-(trifluoromethyl)phenyl]-pyrazolo[1,5-a]pyrimidine | 48 |
| 7-(3,4-Dichlorophenyl)-4,5-dihydropyrazolo-[1,5-a]pyrimidine-3-carbonitrile | 12 |

Certain of the novel 4,5-dihydro and 4,5,6,7-tetrahydropyrazolo[1,5-a]pyrimidines of the invention are active hypotensive agents at nontoxic doses when administered to mammals. These compounds were tested for hypotensive activity by the method of P.S. Chan and D.W. Poorvin, Clinical and Experimental Hypertension, 1 (6), 817-830 (1979). Male, 16 week old, spontaneously hypertensive rats of the Okamoto strain having an average mean arterial blood pressure of 160±1.5 mm of mercury are used in the best. One to three rats are used per test compound. A rat is dosed by gavage with a test compound, suspended in 2% pre-boiled starch at a concentration of 50 mg/ml, at a dose of 100 mg/kg of body weight or less, with 0.9% sodium chloride loading at a dose of 25 ml/kg of body weight. A second idential dose of the test compound, without sodium chloride loading is given 24 hours later. At 28 hours after the initial dose the mean arterial blood pressure is measured by the method of Chan and Poorvin vide supra. The procedure is repeated in a second and third rat when necessary.

The results of this test on representative compounds of the present invention appear below in Table IV.

TABLE IV

Reduction of Mean Arterial Blood Pressure in Spontaneously Hypertensive Rats

| Compound | MABP/mm Hg (no. of rats) |
|---|---|
| 4,5-Dihydro-7-[3-(trifluoromethyl)phenyl]-pyrazolo[1,5-a]pyrimidine-3-carboxamide | 108(1) |
| 7-(2,5-Dichlorophenyl)-4,5-dihydro-2-methylpyrazolo[1,5-a]pyrimidine-3-carboxamide | 95(1) |
| [4,5-Dihydro-7-[3-(trifluoromethyl)phenyl]-pyrazolo[1,5-a]pyrimidin-3-yl]phenylmethanone | 122(2) |
| Phenyl[4,5,6,7-tetrahydro-7-(4-pyridinyl)-pyrazolo[1,5-a]pyrimidin-3-yl]methanone | 125(1) |
| 4,5,6,7-Tetrahydro-7-[3-(trifluoromethyl)-phenyl]pyrazolo[1,5-a]pyrimidine-3-carbonitrile | 128(1) |
| 4,5,6,7-Tetrahydropyrazolo[1,5-a]pyrimidine-3-carboxamide | 137(1) |
| 2,2-Dimethyl-1-(4,5,6,7-tetrahydropyrazolo-[1,5-a]pyrimidin-3-yl)-1-propanone | 114(1) |

The 4,5-dihydro and 4,5,6,7-tetrahydropyrazolo-[1,5-a]pyrimidines of this invention are active as antidepressant agents in warm-blooded animals as evidenced by their results when tested in the Stress Induced Immobility Test. In this test, rats were confined on a warm (44.5°C), but aversive, surface for 15 minutes. After 5-6 minutes, control rats began to show episodes of behavior where they remained flat and immobile for periods of time. The total duration of this behavior was timed. The average duration of immobility in the control rats was 200 seconds. The test compounds were administered intraperitoneally at 25 mg/kg of body weight. A compound was considered active if the maximum duration time was <100 seconds (less than 50% of the control value) in more than 50% of the rats.

The results of this test on representative compounds of the present invention appear in Table V.

TABLE V

Stress Induced Immobility Test

| Compound | No. of Rats Responding / No. of Rats Tested | Mean Duration (Seconds) |
|---|---|---|
| 4,5-Dihydro-6-methyl-7-[3-(trifluoromethyl)-phenyl]pyrazolo[1,5-a]-pyrimidine-3-carbonitrile | 4/5 | 55.0 |
| 7-(3-Chlorophenyl)-4,5,6,7-tetrahydro-5-methylpyrazolo[1,5-a]-pyrimidine-3-carbonitrile | 4/5 | 75.4 |
| 7-(3-Chlorophenyl)-4,5-dihydro-6-methyl-pyrazolo[1,5-a]pyrimidine-3-carbonitrile | 2/3 | 66.7 |
| 7-(4-Chlorophenyl)-4,5,6,7-tetrahydro-5-methylpyrazolo[1,5-a]-pyrimidine-3-carbonitrile | 3/3 | 4.7 |

The novel 4,5-dihydro and 4,5,6,7-tetrahydropyrazolo[1,5-a]pyrimidines of the invention which are effective for meliorating anxiety in warm-blooded animals are administered in amounts ranging from about 0.1 mg to about 35.0 mg/kg of body weight per day. A preferred dosage regimen for optimum results would be from about 0.5 mg to about 20.0 mg/kg of body weight per day and such dosage units are employed that a total of from about 35 mg to about 1.4 g of the active compound for a subject of about 70 kg of body weight are administered in a 24 hour period.

Certain of the novel 4,5-dihydro and 4,5,6,7-tetrahydropyrazolo[1,5-a]pyrimidines of the invention have been found to be highly useful for lowering elevated blood pressure of alleviating depression in mammals when administered in amounts of ranging from about 2.5 mg to about 100 mg/kg of body weight per day. A preferred dosage regimen for optimum results would be from about 50 mg to about 750 mg per dose. Such dosage units are employed that a total of from about 200 mg to about 3.0 g of the active compound for a subject of about 70 kg of body weight are administered in a 24 hour period.

The dosage regimen for the above-described utilities may be adjusted to provide the optimum therapeutic response. For example, several divided doses may be administered daily or the dose may be proportionally reduced as indicated by the exigencies of the therapeutic situation. A decided practical advantage is that these active compounds may be administered in any convenient manner such as by the oral, intravenous, intramuscular or subcutaneous routes.

The active compounds may be orally administered, for example, with an inert diluent or with an assimilable edible carrier. They may be enclosed in hard or soft shell gelatin capsules or compressed into tablets. They also may be incorporated directly with the food of the diet. For oral therapeutic administration, these active compounds may be incorporated with excipients and used in the form of ingestible tablets, buccal tablets, troches, capsules, elixirs, suspensions, syrups, wafers and the like. Such compositions and preparations should contain at least 0.1% of active compound. The percentage of the compositions and preparation may, of course, be varied and may conveniently be between about 2% to about 60% of the

weight of the unit. The amount of active compound in such therapeutically useful compositions is such that a suitable dosage will be obtained. Preferred compositions or preparations according to the present invention are prepared so that an oral dosage unit form contains between about 5 and 200 mg of active compound.

The tablets, troches, pills, capsules and the like may also contain the following: A binder such as gum tragacanth, acacia, corn starch or gelatin; excipients such as dicalcium phosphate; a disintegrating agent such as corn starch, potato starch, alginic acid and the like; a lubricant such as magnesium stearate; and a sweetening agent such as sucrose, lactose or saccharin may be added or a flavoring agent such as peppermint, oil of wintergreen or cherry flavoring. When the dosage unit form is a capsule, it may contain, in addition to materials of the above type, a liquid carrier. Various other materials may be present as coatings or to otherwise modify the physical form of the dosage unit. For instance, tablets, pills or capsules may be coated with shellac, sugar or both. A syrup or elixir may contain the active compound, sucrose as a sweetening agent, methyl and propylparabens as preservatives, a dye and flavoring such as cherry or orange flavor. Of course, any material used in preparing any dosage unit form should be pharmaceutically pure and substantially non-toxic in the amounts used. In addition, these active compounds may be incorporated into sustained-release preparations and formulations.

These active compounds may also be administered parenterally or intraperitoneally. Solutions or suspensions of these active compounds as a free base or pharmacologically acceptable salt can be prepared in water suitably mixed with a surfactant such as sodium lauryl sulfate or an emulsifier or stabilizer such as hydroxypropylcellulose. Dispersons can also be prepared in glycerol, liquid polyethylene glycols, and mixtures thereof in oils. Under ordinary conditions of storage and use, these preparations contain a preservative to prevent the growth of microorganisms.

The pharmaceutical forms suitable for injectable use include sterile aqueous solutions or dispersions and sterile powders for the extemporaneous preparation of sterile injectable solutions or dispersions. In all cases, the form must be sterile and must be fluid to the extent that easy syringability exists. It must be stable under the conditions of manufacture and storage and must be preserved against the contaminating action of microorganisms such as bacteria and fungi. The carrier can be a solvent or dispersion medium containing, for example, water, ethanol, polyol (e.g. glycerol, propylene glycol and liquid polyethylene glycol), suitable mixtures thereof, and vegetable oils.

The 4,5-dihydro and 4,5,6,7-tetrahydropyrazolo[1,5-a]pyrimidines of the invention will be described in greater detail in conjunction with the following non-limiting Examples 1-63.

Example 1

7-[3-(Trifluoromethyl)phenyl]pyrazolo[1,5-a]-pyrimidine-3-carboxamide

A mixture of 3.0 g of 7-(α,α,α-trifluoro-m -tolyl)pyrazolo[1,5-a]pyrimidine-3-carbonitrile (prepared as described in U. S. Patent 4,236,005) and 150 ml of concentrated sulfuric acid was stirred at room temperature for 4 hours. The solution was then carefully poured into ice water with stirring. The white precipitate formed was collected, washed with water and then with saturated sodium bicarbonate until it was neutral. The solid was heated with one liter of isopropyl alcohol and filtered. The white solid was dried in vacuo and gave the product of the example as a colorless solid, mp 256-258°C.

Example 2

7-(2,5-Dichlorophenyl)-2-methylpyrazolo[1,5-a]pyrimidine-3-carboxamide

A mixture of 31.0 g of 2′,5′-dichloroacetophenone and 25 ml of N,N-dimethylformamide dimethyl acetal was heated on a steam bath for 6 hours, then evaporated to dryness invacuo. The residue was slurried with hexane, filtered, and gave 35.3 g of 2′,5′-dichloro-3-dimethylaminoacrylophenone as orange crystals, mp 83-85°C.

A mixture of 12.2 g of 3-amino-4-cyano-5-methylpyrazole and 24.4 g of 2′,5′-dichloro-3-dimethylaminoacrylophenone in 250 ml of glacial acetic acid was heated on a steam bath for 4 hours. The mixture was cooled and filtered and gave 21.28 g of 7-(2,5-dichlorophenyl)-2-methylpyrazolo[1,5-a]-pyrimidine-3-carbonitrile as off-white crystals.

The preceding product 21.28 g was dissolved in concentrated sulfuric acid and stirred for 5 hours. The solution was carefully poured onto ice. The precipitate which formed was collected by filtration, washed with water and air dried to give the product of the example as colorless crystals, mp 234-236°C.

Additional pyrazolo[1,5-a]pyrimidine-4-carboxamides which were prepared from the corresponding pyrazolo[1,5-a]pyrimidine-3-carbonitriles in the manner described in Example 1 are listed in Table VI.

The pyrazolo[1,5-a]pyrimidine-3-carboxamides which were prepared from the corresponding pyrazolo-[1,5-a]pyrimidine-3-carbonitriles in the manner described in Example 1 are listed in Table VI.

The pyrazolo[1,5-a]pyrimidine-3-carbonitriles were prepared by the procedures described in U. S. Patents 4,178,449, 4,236,005 and 4,281,000 by reacting the appropriate 3-(dimethylamino)acrylophenone intermediate with an appropriately substituted 3-aminopyrazole-4-carbonitrile.

TABLE VI

Pyrazolo[1,5-a]pyrimidine-3-carboxamides

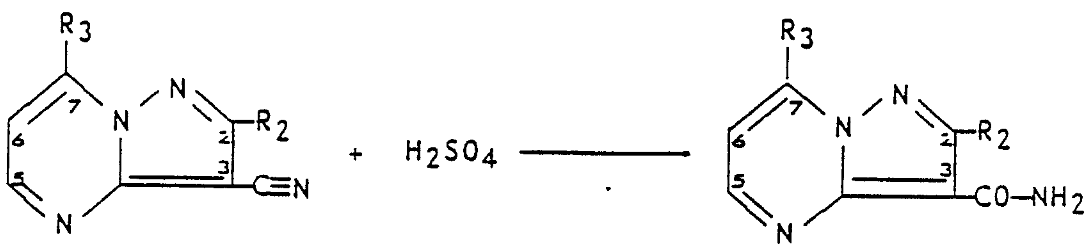


| Ex. | Compound | $R_2$ | $R_3$ | MP°C |
|---|---|---|---|---|
| 3 | 7-Phenylpyrazolo[1,5-a]pyrimidine-3-carboxamide | H | 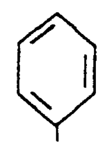 | 236-238.5 |
| 4 | 2-Methyl-7-phenylpyrazolo-[1,5-a]pyrimidine-3-carboxamide | $CH_3$ | 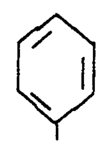 | 233-235 |
| 5 | 7-(3-Pyridinyl)pyrazolo[1,5-a]-pyrimidine-3-carboxamide | H | 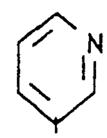 | 285-286 |
| 6 | 7-(4-Pyridinyl)pyrazolo[1,5-a]-pyrimidine-3-carboxamide | H | 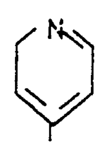 | 394-396 |
| 7 | 7-(3-Fluorophenyl)pyrazolo-[1,5-a]pyrimidine-3-carboxamide | H | 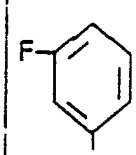 | 247-249 |

Example 8

Phenyl[7-[3-(trifluoromethyl)phenyl]pyrazolo[1,5-a]pyrimidin-3-yl]methanone

A reaction mixture of 1.87 g of (3-amino-1H -pyrazol-4-yl)phenyl-methanone and 2.43 g of 3-dimethylamino-1-[3-(trifluoromethyl)phenyl]-2-propen-1-one in 25 ml of glacial acetic acid was refluxed for 6 hours and then the solvent was removed in vacuo giving a crystalline residue. This residue was partitioned between saturated aqueous sodium bicarbonate and dichloromethane. The organic layer was dried with anhydrous sodium sulfate and then passed through a short pad of hydrous magnesium silicate. The addition of hexane to the refluxing eluate induced crystallization. After cooling, the desired product was collected as crystals, mp 148-150°C.

Phenyl[7-(4-pyridinyl)pyrazolo[1,5-a]pyrimidin-3-yl]methanone

Following the general procedure of Example 8 and reacting (3-amino-1 H-pyrazol-4-yl)phenyl-methanone with 3-dimethylamino-1-(4-pyridinyl)-2-propen-1-one gave the desired product, mp 185-186°C.

Example 10

7-(3-Chlorophenyl)pyrazolo[1,5-a]pyrimidine-3-carbonitrile

A mixture of 50.0 g of 3'-chloro-3-dimethylaminoacrylophenone, 25.0 g of 3-aminopyrazole-4-carbonitrile and 500 ml of glacial acid was heated at reflux for 2 hours. The mixture went into solution upon heating and a precipitate formed after one hour of refluxing. The reaction mixture was filtered and the crystals collected were triturated with saturated aqueous sodium bicarbonate, filtered and washed with water and then dried to give 49.0 g of the product of the example as colorless crystals, mp 238-240°C.

Example 11

7-(3-Nitrophenyl)pyrazolo[1,5-a]pyrimidine-3-carbonitrile

A mixture of 24.4 g of 3-dimethylamino-3'-nitroacrylophenone, 13.0 g of 3-aminopyrazole-4-carbonitrile and 120 ml of glacial acetic acid was heated at reflux for 7 hours, then was stirred at room temperature for 16 hours. The precipitate was collected, triturated with saturated aqueous sodium bicarbonate, filtered and washed with water. The solid was then triturated with acetonitrile, filtered and dried and gave the desired product, mp 244-246°C.

Example 12

N-[3-(3-Cyanopyrazolo[1,5-a]pyrimidin-7-yl)phenyl]-N-methylacetamide

A mixture of 540 mg of 3-aminopyrazole-4-carbonitrile, 1.23 g of N-[3-[3-(dimethylamino)-1-oxo-2-propenyl]phenyl]-N-methylacetamide and 50 ml of glacial acetic acid was heated at reflux for 8 hours then the solvent was removed. The residue was partitioned between saturated aqueous sodium bicarbonate and dichloromethane. The organic layer was separated, dried, passed through a pad of hydrous magnesium silicate and hexane was added to the refluxing filtrate to crystallize the product. The mixture was cooled and the solid collected, giving the desired product, mp 195-197°C.

Example 13

N-[3-(3-Cyanopyrazolo[1,5-a]pyrimidin-7-yl)phenyl]acetamide

A mixture fo 6.0 g of 3-aminopyrazole-4-carbonitrile, 13.0 g of N-[3-[3-(dimethylamino)-1-oxo-2-prophenyl]phenyl]acetamide and 100 ml of glacial acetic acid was heated at reflux for 4 hours. On standing at room temperature a precipitate formed. The precipitate was isolated, washed with hexane, then ether and dried. The solid was recrystallized from acetonitrile-N,N-dimethylformamide and gave the product of the example, mp 252-254°C.

Example 14

7-(3-Chlorophenyl)-5-methylpyrazolo[1,5-a]pyrimidine-3-carbonitrile

A mixture of 50.0 g of 3-chloroacetophenone and 75.0 ml of N,N-dimethylacetamide dimethyl acetal was stirred and heated at reflux for 16 hours. The mixture was evaporated in vacuo to give an oil. The oil was triturated with hexane while cooling in an ice bath. Scratching induced crystal formation. The crystals were collected by filtration and gave 60.2 g of 1-(3-chlorophenyl)-N-(dimethylamino)-2-buten-1-one as red crystals, mp 38-40°C.

A mixture of 30.0 g of the preceding compound, 14.48 g of 3-aminopyrazole-4-carbonitrile and 200 ml of glacial acetic acid was stirred and heated at reflux for 2 hours, with formation of a solid. The mixture was allowed to stand at room temperature for 16 hours, then was filtered. The white crystalline precipitate was triturated with sodium bicarbonate to neutralize, then was filtered. The crystals were copiously washed with water, then dried to give the product of the example as white crystals, mp 218-220°C.

Example 15

7-(4-Chlorophenyl)-5-methylpyrazolo[1,5-a]pyrimidine-3-carbonitrile

A mixture of 25.0 g of 4-chloroacetophenone and 40 ml of N,N-dimethylacetamide dimethyl acetal was stirred and heated at reflux for 3 hours. The mixture was evaporated in vacuo to give a red solid. The solid was triturated with n-hexane and filtered. The solid was washed with n-hexane and dried to give 15.6 g of 1-(4-chlorophenyl)-3-(dimethylamino)-2-buten-1-one as red crystals, mp 103-105°C.

A mixture of 15.6 g of the preceding compound, 7.53 g of 3-aminopyrazole-4-carbonitrile and 150 ml of glacial acetic acid was stirred and heated at reflux for 15 minutes. The mixture went into solution and then a pre cipitate formed during reflux. The mixture was filtered to yield a light brown solid. The solid was triturated with aqueous saturated sodium bicarbonate until pH 7-8 was achieved, then was collected by filtration and dried to give 17.2 g of crude product. A one-gram-portion of product was triturated with water and stirred for 2 hours at room temperature. The mixture was filtered and the solid was dried and gave the desired product as brown crystals, mp 285-287°C.

Example 16

5-Methyl-7-[3-(trifluoromethyl)phenyl]pyrazolo[1,5-a]pyrimidine-3-carbonitrile

A mixture of 20.0 g of 3-trifluoromethylacetophenone and 20 ml of N,N-dimethylacetamide dimethyl acetal was stirred and heated at reflux for 3 hours. The mixture was evaporated in vacuo to give a red solid. The solid was triturated with n-hexane and filtered. The solid was washed with n-hexane and dried to give 18.0 g of 3-(dimethylamino)-1-[3-(trifluoromethyl)phenyl]-2-buten-1-one as red crystals, mp 71-73°C.

A mixture of 18.0 g of the preceding compound, 7.56 g of 3-aminopyrazole-4-carbonitrile and 150 ml of glacial acetic acid was stirred and heated at reflux for 16 hours. The reaction mixture was evaporated to dryness in vacuo to give yellow-white crystals. The crystals were triturated with aqueous saturated sodium bicarbonate solution, then filtered. The crystals were washed with water, then dried. The solid was dissolved in 800 ml of hot ethanol and cooled in an ice bath with scratching to induce crystallization. The product was collected by filtration and dried to give the product as pale cream crystals, mp 153-155°C.

Example 17

7-(3-Chlorophenyl)-6-methylpyrazolo[1,5-a]pyrimidine-3-carbonitrile

A mixture of 75.0 g of m-chloropropiophenone and 200 ml of N,N-dimethylformamide dimethyl acetal was stirred and heated at reflux for 16 hours. The mixture was evaporated in vacuo to give a black oil. The oil was subjected to Kugelrohr distillation at 0.5 mm of mercury and the fraction that boils at 110°C was removed. The residue from the distillation was collected to give 35.0 g of 3′-chloro-3-dimethylamino-2-methylacrylophenone as a black oil.

A mixture of 25.0 g of the preceding compound, 11.88 g of 3-aminopyrazole-4-carbonitrile and 250 ml of glacial acetic acid was stirred and heated at reflux for 3 hours. The reaction mixture was evaporated to dryness in vacuo to give a dark brown solid. The solid was triturated with aqueous saturated sodium bicarbonate solution, then filtered. The solid was then triturated with methanol and filtered and gave the product of the example as white crystals, mp 206-208°C.

Example 18

7-(4-Chlorophenyl)-6-methylpyrazolo[1,5-a]pyrimidine-3-carbonitrile

A mixture of 50.0 g of p-chloropropiophenone and 200 ml of N,N-dimethylformamide dimethyl acetal was stirred and heated at reflux for 28 hours. The reaction mixture was evaporated in vacuo to give a yellow oil. The oil was then subjected to Kugelrohr distillation. The residual oil from the distillation was collected and triturated with hexane to provide crystals. The solid was collected by filtration and gave 21.5 g of 4′-chloro-3-dimethylamino-2-methylacrylophenone as yellow crystals, mp 45-47°C.

A mixture of 21.5 g of the preceding compounding 10.368 g of 3-aminopyrazole-4-carbonitrile and 250 ml of glacial acetic acid was stirred and heated at reflux for 24 hours. The mixture was evaporated to dryness in vacuo to give brown crystals. The solid was triturated with satur ated sodium bicarbonate solution to obtain pH 7-8 then the mixture was filtered. The solid was washed with water and dried and gave the desired product as light brown crystals, mp 154-157°C.

Example 19

4,5-Dihydro-7-[3-(trifluoromethyl)phenyl]pyrazolo[1,5-a]pyrimidine-3-carbonitrile

To a solution of 6.0 g of 7-(α,α,α-trifluoro-m-tolyl)pyrazolo[1,5-a]pyrimidine-3-carbonitrile (Ex. 3, U. S. Patent 4,236,005) in 100 ml of glacial acetic acid, was added in portions, 3.0 g of sodium cyanoborohydride at room temperature under nitrogen. The mixture was stirred at room temperature for 2 hours. The precipitate was collected by filtration, washed with water, then dissolved in dichloromethane. The organic solution was neutralized with a saturated solution of sodium bicarbonate, then dried over anhydrous sodium sulfate and filtered. The filtrate was evaporated in vacuo and gave the desired product as a white solid. The product was recrystallized from isopropyl alcohol, mp 175-177°C.

Following the general procedure of Example 19 and reacting the appropriate pyrazolo[1,5-a]pyrimidine derivative with sodium cyanoborohydride, the dihydro products of Examples 20-38, listed in Table VII, were obtained.

TABLE VII

| Ex. | Pyrazolo[1,5-a]pyrimidine Derivative | Dihydro-product | MP°C |
|---|---|---|---|
| 20 | 7-Phenylpyrazolo[1,5-a]pyrimidine-3-carbonitrile | 4,5-Dihydro-7-phenylpyrazolo[1,5-a]-pyrimidine-3-carbonitrile | 169-172 |
| 21 | 7-Phenylpyrazolo[1,5-a]pyrimidine-3-carboxylic acid, ethyl ester | 4,5-Dihydro-7-phenylpyrazolo[1,5-a]-pyrimidine-3-carboxylic acid, ethyl ester | 109-111 |
| 22 | 7-[3-(Trifluoromethyl)phenyl]pyrazolo[1,5-a]pyrimidine-3-carboxylic acid, ethyl ester | 4,5-Dihydro-7-[3-(trifluoromethyl)phenyl]pyrazolo[1,5-a]pyrimidine-3-carboxylic acid, ethyl ester | 108-110 |
| 23 | 7-(m-Fluorophenyl)pyrazolo[1,5-a]-pyrimidine-3-carbonitrile | 7-(3-Fluorophenyl)-4,5-dihydropyrazolo-[1,5-a]pyrimidine-3-carbonitrile | 196-198 |
| 24 | 7-[3-(Trifluoromethyl)phenyl]pyrazolo[1,5-a]pyrimidine-3-carboxamide | 4,5-Dihydro-7-[3-(trifluoromethyl)-phenyl]pyrazolo[1,5-a]pyrimidine-3-carboxamide | 157-160 |
| 25 | 7-(2,5-Dichlorophenyl)-2-methyl-pyrazolo[1,5-a]pyrimidine-3-carboxamide | 7-(2,5-Dichlorophenyl)-4,5-dihydro-2-methylpyrazolo[1,5-a]pyrimidine-3-carboxamide | 93-96 |
| 26 | 7-Phenylpyrazolo[1,5-a]pyrimidine-3-carboxamide | 4,5-Dihydro-7-phenylpyrazolo[1,5-a]-pyrimidine-3-carboxamide | 149-152 |

TABLE VII (continued)

| Ex. | Pyrazolo[1,5-a]pyrimidine Derivative | Dihydro-product | MP°C |
|---|---|---|---|
| 27 | 2-Methyl-7-phenylpyrazolo[1,5-a]-pyrimidine-3-carboxamide | 4,5-Dihydro-2-methyl-7-phenylpyrazolo-[1,5-a]pyrimidine-3-carboxamide | 275-277 |
| 28 | 7-(3-Pyridyl)pyrazolo[1,5-a]pyrimidine-3-carboxamide | 4,5-Dihydro-7-(3-pyridinyl)pyrazolo-[1,5-a]pyrimidine-3-carboxamide | 182-184 |
| 29 | Phenyl[7-[3-(trifluoromethyl)phenyl]pyrazolo[1,5-a]pyrimidin-3-yl]-methanone | 4,5-Dihydro-7-[3-(trifluoromethyl)-phenyl]pyrazolo[1,5-a]pyrimidine-3-yl]-phenylmethanone | 115-117 |
| 30 | 7-(3-Chlorophenyl)pyrazolo[1,5-a]-pyrimidine-3-carbonitrile | 7-(3-Chlorophenyl)-4,5-dihydropyrazolo-[1,5-a]pyrimidine-3-carbonitrile | 183-186 |
| 31 | 7-(3-Nitrophenyl)pyrazolo[1,5-a]-pyrimidine-3-carbonitrile | 4,5-Dihydro-7-(3-nitrophenyl)pyrazolo-[1,5-a]pyrimidine-3-carbonitrile | 230-233 |
| 32 | N-[3-(3-Cyanopyrazolo[1,5-a]pyrimidin-7-yl)phenyl]-N-methylacetamide | N-[3-(3-Cyano-4,5-dihydropyrazolo-[1,5-a]pyrimidinyl-7-yl)phenyl]-N-methylacetamide | 68-71 |
| 33 | N-[3-(3-Cyanopyrazolo[1,5-a]pyrimidin-7-yl)phenyl]acetamide | N-[3-(3-Cyano-4,5-dihydropyrazolo-[1,5-a]pyrimidin-7-yl)phenylacetamide | 224-227 |

TABLE VII (continued)

| Ex. | Pyrazolo[1,5-a]pyrimidine Derivative | Dihydro-product | MP°C |
|---|---|---|---|
| 34 | 7-(3-Chlorophenyl)-6-methylpyrazolo-[1,5-a]pyrimidine-3-carbonitrile | 7-(3-Chlorophenyl)-4,5-dihydro-6-methylpyrazolo[1,5-a]pyrimidine-3-carbonitrile | 206-208 |
| 35 | 7-(4-Chlorophenyl)-6-methylpyrazolo-[1,5-a]pyrimidine-3-carbonitrile | 7-(4-Chlorophenyl)-4,5-dihydro-6-methylpyrazolo[1,5-a]pyrimidine-3-carbonitrile | 203-205 |
| 36 | 5-Methyl-7-[3-(trifluoromethyl)-phenyl]pyrazolo[1,5-a]pyrimidine-3-carbonitrile | 4,5-Dihydro-5-methyl-7-[3-(trifluoromethyl)phenyl]pyrazolo[1,5-a]pyrimi-dine-3-carbonitrile | 160-163 |
| 37 | 6-Methyl-7-[3-(trifluoromethyl)-phenyl]pyrazolo[1,5-a]pyrimidine-3-carbonitrile | 4,5-Dihydro-6-methyl-7-[3-(trifluoromethyl)phenyl]pyrazolo[1,5-a]pyrimi-dine-3-carbonitrile | 178-182 |
| 38 | 7-(3-Fluorophenyl)pyrazolo[1,5-a]-pyrimidine-3-carboxamide | 7-(3-Fluorophenyl)-4,5-dihydropyrazolo-[1,5-a]pyrimidine-3-carboxamide | 122-125 |

In a manner similar to the preceeding examples, the following compounds may be prepared:
4,5-Dihydro-2-methyl-7-[3-(trifluoromethyl)phenyl]pyrazolo[1,5-a]pyrimidine-3-carbonitrile;
4,5-Dihydro-2-methyl-7-[3-(trifluoromethyl)phenyl]pyrazolo[1,5-a]pyrimidine-3-carboxamide;
4,5-Dihydro-7-(3-methoxyphenyl)pyrazolo[1,5-a]pyrimidine-3-carbonitrile;

4,5-Dihydro-7-[2-(trifluoromethyl)phenyl]pyrazolo[1,5-a]pyrimidine-3-carbonitrile;
2-Ethyl-4,5-dihydro-6-methyl-7-[3-(trifluoromethyl)phenyl]pyrazolo[1,5-a]pyrimidine-3-carbonitrile;
4,5-Dihydro-7-(3-tolyl)pyrazolo[1,5-a]pyrimidine-3-carboxylic acid, ethyl ester;
2-Ethyl-4,5-dihydro-7-phenylpyrazolo[1,5-a]pyrimidine-3-carbonitrile;
4,5-Dihydro-7-(3,4-xylyl)pyrazolo[1,5-a]pyrimidine-3-carboxylic acid;
4,5-Dihydro-7-(3,4-dimethoxyphenyl)pyrazolo[1,5-a]pyrimidine-3-carboxylic acid, ethyl ester;
7-(4-Ethylphenyl)-4,5-dihydropyrazolo[1,5-a]pyrimidine-3-carboxylic acid, ethyl ester;
4,5-Dihydro-7-phenylpyrazolo[1,5-a]pyrimidine-3-yl, trifluoromethyl ketone;
4,5-Dihydro-7-(3-pyridinyl)pyrazolo[1,5-a]pyrimidine-3-carbonitrile;
2-Ethyl-4,5-dihydro-7-(3-pyridinyl)pyrazolo[1,5-a]pyrimidine-3-carbonitrile;
4,5-Dihydro-7-(3-pyridinyl)pyrazolo[1,5-a]pyrimidine-3-carboxylic acid, ethyl ester;
2-Ethyl-4,5-dihydro-7-(3-pyridinyl)pyrazolo[1,5-a]pyrimidine-3-carboxylic acid, ethyl ester;
4,5-Dihydro-7-(3-thienyl)pyrazolo[1,5-a]pyrimidine-3-carbonitrile;
4,5-Dihydro-2,6-dimethyl-7-(3-pyridinyl)pyrazolo[1,5-a]pyrimidine-3-carbonitrile;
3-Chloro-2-ethyl-4,5-dihydro-7-(3-pyridinyl)pyrazolo[1,5-a]pyrimidine;
4,5-Dihydro-7-(6-methyl-2-pyridinyl)pyrazolo[1,5-a]pyrimidine-3-carbonitrile;
4,5-Dihydro-7-[3-(trifluoromethyl)phenyl]pyrazolo[1,5-a]pyrimidine;
3-Chloro-4,5-dihydro-7-[3-(trifluoromethyl)phenyl]pyrazolo[1,5-a]pyrimidine;
3-Chloro-4,5-dihydro-7-(3-pyridinyl)pyrazolo[1,5-a]pyrimidine; and the like.

## Example 39

### 4,5-Dihydro-4-methyl-7-[3-(trifluoromethyl)phenyl]pyrazolo[1,5-a]pyrimidine-3-carbonitrile

A mixture of 1.5 g of 4,5-dihydro-7-[3-(trifluoromethyl)phenyl]pyrazolo[1,5-a]pyrimidine-3-carbonitrile (Ex. 19) and 220 mg of 60% sodium hydride dispersed in mineral oil in 50 ml of N,N-dimethylformamide was stirred at room temperature under nitrogen for 3 1/2 hours. Then one ml of iodomethane was added and the reaction mixture was stirred under nitrogen for 16 hours. The mixture was evaporated *in vacuo* and gave an oil. The oil crystallized upon trituration in n-hexane to give the produce which was recrystallized from ethyl acetate:n-hexane (1:4) and this gave the desired product as yellow crystals, mp 120-122°C.

## Example 40

### 4,5,6,7-Tetrahydro-7-[3-(trifluoromethyl)phenyl]pyrazolo[1,5-a]pyrimidine-3-carboxamide

A solution of 3.44 g of 4,5-dihydro-7-[3-(trifluoromethyl)phenyl]pyrazolo[1,5-a]pyrimidine-3-carboxamide (Ex. 24) in 40 ml of trifluoroacetic acid was stirred under nitrogen and heated to 60°C in an oil bath. Then 5.0 ml of triethylsilane was added and the mixture was stirred at 60°C for 24 hours. The reaction mixture was cooled and carefully poured into a beaker containing a 25% aqueous solution of potassium hydroxide and cracked ice. The pro duct which precipitated was extracted into chloroform. The chloroform was washed with water, dried over anhydrous sodium sulfate and filtered. The filtrate was evaporated *in vacuo* and gave crystals which were recrystallized from toluene hexane to give the desired product, mp 152-154°C.

## Example 41

### 4,5,6,7,-Tetrahydro-7-[3-(trifluoromethyl)phenyl]-pyrazolo[1,5-a]pyrimidine-3-carbonitrile

A mixture of 1.0 g of 4,5,6,7-tetrahydro-7-[3-(trifluoromethyl)phenyl]pyrazolo[1,5-a]pyrimidine-3-carboxamide, 0.44 g of potassium carbonate and 10 ml of ethyl chloroformate 30 ml of p-dioxane was heated at reflux for 3 hours. The mixture was evaporated to dryness *in vacuo*. The residue was extracted with dichloromethane and the combined extracts were filtered, then evaporated and gave 0.6 g of an oil. The oil was triturated with etherhexane and gave 250 mg of a white solid. The solid was subjected to preparation thick layer chromatography, using chloroform:ethanol(10:1). The product at Rf 0.64 was collected and separated and gave the product of the example as a white solid, mp 183-185°C.

Example 42

Phenyl[4,5,6,7-tetrahydro-7-(4-pyridinyl)pyrazolo[1,5-a]pyrimidin-3-yl]methanone

To a 20.0 g portion of phenyl[7-(4-pyridinyl)pyrazolo[1,5-a]pyrimidin-3-yl]methanone (prepared as described in Ex. 9) suspended and stirred in 100 ml of glacial acetic acid under nitrogen was added in portions 8.5 g of sodium cyanoborohydride, during this addition another 50 ml of glacial acetic was also added. After 3 hours the solution was evaporated to dryness in vacuo. The residue was dissolved in dichloromethane and treated with saturated sodium bicarbonate until basic. The organic layer was separated and dried over anhydrous sodium sulfate. Evaporation gave a gummy solid. The solid was recrystallized from isopropyl alcohol and gave the desired product as a light yellow solid, mp 186-189°C.

Example 43

7-(3-Fluorophenyl)-4,5,6,7-tetrahydropyrazolo[1,5-a]pyrimidine-3-carbonitrile

To a 5.0 g portion of 7-(3-fluorophenyl)-4,5-dihydropyrazolo[1,5-a]pyrimidine-3-carbonitrile (prepared as described in Ex. 23) in 50 ml of trifluoroacetic acid, stirred under nitrogen and heated to 60°C was added 7.2 ml of triethylsilane. Heating was continued for 4 hours, then the mixture was stirred at room temperature for 16 hours. The mixture was carefully poured into an ice cooled beaker containing 150 ml of water and 40 g of potassium hydroxide. The precipitate formed was collected, washed with water and dried. The solid was dissolved in dichloromethane and filtered through hydrous magnesium silicate. Evaporation of the filtrate gave a white solid. Recrystallization from isopropyl alcohol gave the desired product, mp 146-148°C.

Example 44

7-(3-Chlorophenyl)-4,5,6,7-tetrahydropyrazolo[1,5-a]pyrimidine-3-carbonitrile

To an 8.0 g portion of 7-(3-chlorophenyl)-4,5-dihydropyrazolo[1,5-a]pyrimidine-3-carbonitrile (prepared as described in Ex. 30) in 65 ml of trifluoroacetic acid, stirred under nitrogen and heated to 55°C was added 7.3 ml of triethylsilane. The reaction mixture was stirred and heated at 60°C for 7 1/2 hours. The mixture was then carefully poured into an ice cooled beaker containing 100 ml of 25% aqueous potassium hydroxide. The precipitate which formed was collected by filtration and dissolved in chloroform. The chloroform solution was washed with saturated sodium chloride solution, dried over anhydrous sodium sulfate and filtered. The filtrate was concentrated in vacuo to give 6.8 g of white crystals. The crystals were triturated with either. The ether was collected and crystals formed. The crystals were washed with ether and give 2.8 g of white crystals. The crude product, 2.8 g, was chromatographed on a liquid chromatograph using a silica gel column and eluting with 5% ethyl acetate/chloroform. The fraction containing the desired product was collected and evaporated in vacuo and gave crystals, mp 148-150°C.

Example 45

7-(3-Chlorophenyl)-4,5,6,7-tetrahydro-5-methylpyrazolo[1,5-a]pyrimidine-3-carbonitrile

To an unweighed amount of 7-(3-chlorophenyl)-5-methylpyrazolo[1,5-a]pyrimidine-3-carbonitrile (prepared as described in Ex. 14) and in a stirred mixture of one liter of tetrahydrofuran and one liter of methyl alcohol, under nitrogen was added 20.0 g of sodium borohydride, portionwise. The mixture was stirred for 8 hours at room temperature, then allowed to stand for 3 days providing two layers. The top layer was separated, dried over anhydrous sodium sulfate and filtered. The filtrate was evaporated in vacuo to give a yellow oil. The oil was dissolved in 250 ml of chloroform and filtered through hydrous magnesium silicate and evaporated to a yellow oil. The oil was triturated with ether, forming crystals on scratching. The crystals were collected to give the desired product as white crystals, mp 192-195°C.

Example 46

7-(4-Chlorophenyl)4,5,6,7-tetrahydro-5-methylpyrazolo[1,5-a]pyrimidine-3-carbonitrile

To a 17.2 g portion of 7-(4-chlorophenyl)-5-methylpyrazolo[1,5-a]pyrimidine-3-carbonitrile (prepared as described in Ex. 15) in a stirred mixture of one liter of tetrahydrofuran and one liter of methyl alcohol was added under nitrogen 10.0 g of sodium borohydride, portionwise. The mixture was heated to 55°C for 6 hours, then evaporated in vacuo to give a gummy solid. The solid was triturated with ether then collected by filtration. The solid was recrystallized from 800 ml of methyl alcohol and collected by filtration to give the desired product as white crystals, mp 215-217°C.

Example 47

4,5,6,7-Tetrahydro-7-[3-(trifluoromethyl)phenyl]pyrazolo[1,5-a]pyrimidine-3-carbonitrile

To stirred mixture of 14.0 g of 5-methyl-7-[3-(trifluoromethyl)phenyl]pyrazolo [1,5-a]pyrimidine-3-carbonitrile (prepared as described in Ex. 16) in 250 ml of glacial acetic acid, under nitrogen was added portionwise 29.5 g of sodium cyanoborohydride. The mixture was heated at 55-60°C in a water bath for 16 hours then was evaporated in vacuo to give a white solid. The solid was triturated with saturated sodium bicarbonate to neutralize to pH 7-8. The crystals were collected by filtration and dissolved in 200 ml of absolute ethanol by heating on a steam bath. The solution was evaporated in vacuo and gave 10.1 g of white crystals. The crude product was chromatographed on a Waters Prep. 500A Liquid Chromatograph using a silica gel column and eluting with ethyl acetate: hexane (35:65) and collecting fractions for two peaks. The second peak fraction was evaporated in vacuo and gave the product of the example as white crystals, mp 192-194°C.

Example 48

4,5,6,7-Tetrahydro-7-[3-(trifluoromethyl)phenyl]pyrazolo[1,5-a}pyrimidine-3-carbonitrile

A mixture of 1.5 parts of 4,5-dihydro-7-[3-(trifluoromethyl)phenyl]pyazolo[1,5-a]pyrimidine-3-carbonitrile, 50 parts of ethyl acetate, 10 parts of acetic acid and 0.2 parts of 5% palladium-on-carbon catalyst was shaken on a Parr hydrogenator under about 30 pounds of hydrogen pressure of 4.5 hours. The reaction mixture was filtered, and the mother liquor was concentrated to remove the solvent. The yellow oil which was obtained was crystallized by trituration in ether. The crystalline product was recrystallized from ethyl acetate by addition of hexane. The product 4,5,6,7-tetrahydro-7-[3-(trifluoromethyl)-phenyl]pyrazolo[1,5-a]-pyrimidine-3-carbonitrile melted at 181-183°C.

Example 49

4,5,6,7-Tetrahydropyrazolo[1,5-a]pyrimidine-3-carboxamide

To a stirred mixture of 4.0 g of pyrazolo[1,5-a]pyrimidine-3-carbonitrile in 200 ml of methyl alcohol under nitrogen at, room temperature, was added, one at a time, 3 pellets of sodium borohydride. The mixture was stirred for several hours and then was allowed to stand at room temperature. The product was collected by filtration to give 1.75 g of 4,5,6,7-tetrahydropyrazolo[1,5-a]pyrimidine-3-carbonitrile, mp 206-207°C.

The preceding product, 1.75 g, was dissolved in concentrated sulfuric acid and stirred for 16 hours. The reaction mixture was carefully poured onto ice and made slightly basic with ammonium hydroxide. The precipitate was collected by filtration to give the product of the example as colorless crystals, mp 247-248°C.

Example 50

4,5,6,7,-Tetrahydro-2-methylpyrazolo[1,5-a]-pyrimidine-3-carbonitrile

A stirred mixture of 7.32 g of 5-amino-3-methylpyrazole-4-carbonitrile and 10.0 g of malonaldehyde bis-(dimethylacetal) in 50 ml of glacial acetic acid was heated at reflux for 8 hours. The reaction mixture was cooled and evaporated in vacuo. The residue was partitioned between saturated sodium bicarbonate and dichloromethane. The organic layer was dried over anhydrous sodium sulfate and passed through hydrous magnesium silicate. The eluent was evaporated to give 7.65 g of 2-methylpyrazolo[1,5-a]pyrimidine-3-carbonitrile, mp 179-181°C.

To a stirred mixture of 5.0 g of the preceding product and 250 ml of methyl alcohol under nitrogen was added sodium borohydride. The mixture was stirred at room temperature for several hours, then was concentrated. The residue was recrystallized from dichloromethane/n-hexane to give the desired product as colorless crystals, mp 170-172°C.

Example 51

4,5,6,7-Tetrahydro-2-methylpyrazolo[1,5-a]pyrimidine-3-carboxamide

A 1.0 g portion of 4,5,6,7-tetrahydro-2-methylpyrazolo[1,5-a]pyrimidine-3-carbonitrile was dissolved with stirring in 5.0 ml of concentrated sulfuric acid. The mixture was stirred for 6 hours at room temperature then poured onto ice. This mixture was made basic with concentrated ammonium hydroxide and then was filtered to give the product of the example as a colorless solid, mp 234-236°C.

Example 52

4,5,6,7-Tetrahydro-2-methyl-5,7-dipropylpyrazolo[1,5-a]pyrimidine-3-carbonitrile

A stirred mixture of 4.88 g of 5-amino-3-methylpyrazole-4-carbonitrile and 6.25 g of 4,6-nonanedione in 50 ml of glacial acetic acid was heated at reflux for 10 hours. The reaction mixture after reflux was worked up as described in Example 50 to give 7.80 of 2-methyl-5,7-dipropylpyrazolo[1,5-a]pyrimidine-3-carbonitrile, mp 73-74°C.

To a 2.0 g portion of the preceding compound in 80 ml of methyl alcohol under nitrogen was added 0.33 g of sodium borohydride, portionwise, while the solution was stirred. After one hour the solvent was evaporated and the residue was partitioned between saturated sodium bicarbonate and dichloromethane and purified as hereinabove described to give the product of the example, mp 98-99°C.

Example 53

4,5,6,7-Tetrahydropyrazolo[1,5-a]-pyrimidine-3-carboxylic acid, ethyl ester

A stirred mixture of 10.88 g of 5-amino-4-pyrazolecarboxylic acid, ethyl ester, 11.51 g of malonaldehyde bis(dimethyl acetal) and 100 ml of glacial acetic acid was heated at reflux for 16 hours, then worked up as described in Example 50 to give 7.8 g of pyrazolo[1,5-a]pyrimidine-3-carboxylic acid, ethyl ester.

To a stirred mixture of 5.0 g of the preceding product in 200 ml of ethyl alcohol plus 100 ml of methyl alcohol, under nitrogen at room temperature was added about 1.0 g of sodium borohydride, portionwise. The reaction mixture was stirred for 16 hours and then was evaporated in vacuo. The residue was partiioned between saturated sodium bicarbonate solution and dichloromethane. The organic layer was passed through a short column of hydrous magnesium silicate and n-hexane was added to the eluent to crystallize the product. Recrystallization from dichloromethane/hexane gave the desired product, mp 158-159°C.

Example 54

Phenyl(4,5,6,7-tetrahydropyrazolo[1,5-a]pyrimidin-3-yl)methanone

A mixture of 1.87 g of (3-amino-1H-pyrazol-4-yl)phenylmethanone (prepared as described in U. S. Patent application Serial No. 612,811, filed May 24, 1984), 1.64 g of malonaldehyde bis(dimethyl acetal) and 25 ml of glacial acetic acid was heated at reflux for 6 hours. The solvent was evaporated and the solid was worked up as described in Example 50 and gave 1.70 g of phenylpyrazolo[1,5-a]pyrimidin-3-yl-methanone, mp 174-175°C.

A mixture of 3.07 g of the preceding compound (prepared in the manner described above), 60 ml of N,N -dimethylformamide and 600 mg of 10% palladium-on-carbon catalyst was hydrogenated with an initial pressure of 15 lbs. of hydrogen until no additional hydrogen was absorbed. The resulting mixture was filtered to remove the catalyst and then evaporated to dryness. The crude product was partitioned between dichloromethane and saturated aqueous sodium bicarbonate solution and after separation, the organic layer was dried over anhydrous sodium sulfate and passed through a short column of hydrous magnesium silicate. The eluent was refluxed on a steam bath, with the gradual addition of n-hexane. When crystallization was noted, the solution was cooled and the solid was collected by filtrtion to give the desired product, mp 173-174°C.

Example 55

2-Furanyl(4,5,6,7-tetrahydropyrazolo[1,5-a]pyrimidin-3-yl)methanone

A mixture of 7.08 g of (3-amino-1H-pyrazol-4-yl)-2-furanylmethanone (prepared as described in U.S. Patent application Serial No. 612,811, filed May 24, 1984), 8.20 g of malonaldehyde bis(dimethyl acetal) and 100 ml of glacial acetic acid was heated at reflux for 8 hours. The solvent was evaporated and the solid was worked up as described in Example 50 to give 4.92 g of 2-furanylpyrazolo[1,5-a]pyrimidin-3-yl-methanone, mp 222-224°C.

A mixture of 1.0 g of the preceding compound 100 ml of N,N-dimethylformamide and 500 mg of 10% palladium-on-carbon catalyst was hydrogenated and isolated as described in Example 54 to obtain 2-furanyl(4,5,6,7-tetrahydropyrazolo[1,5-a]pyrimidin-3-yl)methanone, mp 138-139°C.

Example 56

2,2-Dimethyl-1-(4,5,6,7-tetrahydropyrazolo[1,5-a]pyrimidin-3-yl)-1-propanone

A mixture of 25.0 g of pivaloyl acetonitrile in 50 ml of N,N-dimethylformamide dimethyl acetal was stirred at room temperature for 8 hours under argon and then the solvent was evaporated and n-hexane was added with stirring. The product was collected by filtration to give 34.6 g of 2-[(dimethylamino)methylene]-4,4-dimethyl-3-oxopentanenitrile.

A mixture of 18.0 g of the preceding product, 15.0 g of aminoguanidine nitrate, 250 ml of ethyl alcohol and 11.0 ml of 10N sodium hydroxide was heated at reflux for 8 hours. The solvent was evaporated and water was added to yield an oily precipitate which crystallized with scratching. The precipitate was partitioned between saturated sodium bicarbonate and dichloromethane and worked up as described in Example 50 to give 9.68 g of product. A 1.33 g amount of this material was recrystallized from dichloromethane/hexane to give 1.02 g of 1-(3-amino-1H-pyrazol-4-yl)-2,2-dimethyl-1-propanone, mp 159-160°C.

A stirred mixture of 3.34 g of the preceding product (prepared in the manner described above), 3.50 g of malonaldehyde bis(dimethyl acetal) and 100 ml of glacial acetic acid was heated at reflux for 8 hours. The reaction mixture was cooled and evaporated in vacuo and then partitioned and worked up as described hereinabove to give 3.19 g of 2,2-dimethyl-1-pyrazolo[1,5-a]pyrimidin-3-yl-l-propanone, mp 130-131°C.

A mixture of 2.0 g of the preceding compound, 40 ml of N,N-dimethylformamide and 400 mg of 10% palladium-on-carbon catalyst was hydrogenated and isolated as described in Example 54 to obtain the compound 2,2-dimethyl-1-(4,5,6,7-tetrahydropyrazolo[1,5-a]pyrimidin-3-yl)-1-propanone, mp 169-170°C.

Example 57

4,5-Dihydro-7-[3-(trifloromethyl)phenyl]pyrazolo[1,5-a]pyrimidine-3-carboxylic acid

A 3.37 g amount of 4,5-dihydro-7-[3-(trifluoromethyl)phenyl]pyrazolo[1,5-a]pyrimidine-3-carboxylic acid, ethyl ester (prepared as described in Ex. 22) was added to a solution of 1.4 g of potassium hydroxide in 100 ml of ethanol. This mixture was heated at reflux for 5 hours, cooled to room temperature and evaporated to dryness in vacuo. The residue was dissolved in water and the solution was neutralized to pH 7.0 with concentrated hydrochloric acid. The white precipitate formed was collected by filtration, washed with water and dried in vacuo to give 2.5 g of the desired product as a whtie solid, mp 175-177°C.

Example 58

7-(2,5-Dichlorophenyl)-4,5-dihydropyrazolo[1,5-a]pyrimidine-3-carbonitrile

A mixture of 98.3 g of 2′,5′-dichloro-3-dimethylaminoacrylophenone and 43.5 g of 3-aminopyrazole-4-carbonitrile in one liter of glacial acetic acid was heated at reflux for 6 3/4 hours. The reaction mixture was cooled and diluted with water to provide a precipitate which was collected, washed with water and dried in vacuo to give 107.6 g of 7-(2,5-dichlorophenyl)pyrazolo[1,5-a]pyrimidine-3-carbonitrile as pale yellow crystals, mp 202-204°C.

To a stirred mixture of 59.7 g of the preceding product in 600 ml of glacial acetic acid, under nitrogen was added portionwise about half of 25.9 g of sodium cyanoborohydride. The mixture was then warmed on a steam bath with continued stirring until solution occured. The heat was removed and the remainder of the sodium cyanoborohydride was added under nitrogen and the reaction mixture was stirred for an additional 2 1/2 hours. The yellow precipitate that formed was collected by filtration and washed with water and then neutralized by washing with a saturated solution of sodium bicarbonate. The precipitate was dried in vacuo to give 41.1 g of the product of the example as a light yellow solid, mp 222-223°C.

Example 59

7-(4-Chlorophenyl)-4,5-dihydropyrazolo[1,5-a]pyrimidine-3-carbonitrile

A mixture of 87.0 g of 4′-chloro-3-dimethylaminoacrylophenone and 44.9 g of 3-aminopyrazole-4-carbonitrile in 500 ml of glacial acetic acid was heated at reflux for 5 1/2 hours and then the precipitated product was collected by filtration, washed with water and dried to give 87.7 g of solid. The above filtrate was diluted with water to precipitate additional 13.6 g of solid. The combined product 101.3 g was combined with one liter of absolute ethanol and was heated to boiling and then the mixture was filtered hot to give 79.7 g of 7-(4-chlorophenyl)pyrazolo[1,5-a]pyrimidine-3-carbonitrile, mp 244-245°C.

To a stirred mixture of 46.8 g of 7-(4-chlorophenyl)pyrazolo[1,5-a]pyrimidine-3-carbonitrile in 1.2 liters of glacial acetic acid under nitrogen and heated on a steam bath was added portionwise 30.0 g of sodium cyanoborohydride, heating and stirring was continued until all the solid was dissolved. The heat was removed and stirring was continued. A solid mass formed and was collected by filtration. The solid was washed with water, then with aqueous ammonia, followed by water again. The solid was dried in vacuo to give 29.1 g of the product of the example as a white solid, mp 208-209°C.

Example 60

3-Chloro-4,5-dihydro-7-(3-(trifluoromethyl)phenylpyrazolo[1,5-a]pyrimidine

To a stirred mixture of 13.3 g of 3-chloro-7-[3-(trifluoromethyl)phenyl]pyrazolo[1,5-a]pyrimidine in 150 ml of glacial acetic acid, under nitrogen at room temperature was added portionwise 7.02 g of sodium cyanoborohydride. The mixture was stirred for 24 hours then was evaporated in vacuo to give an oil. The oil was dissolved in dichloromethane and washed with saturated sodium bicarbonate. The organic layer was separated, dried over anhydrous sodium sulfate, filtered and the filtrate was evaporated in vacuo to give an oil which was then triturated with hexane. The crystals that formed were collected by filtration to give 6.7 g of the desired product as yellow crystals, mp 89-92°C.

Example 61

4,5-Dihydro-7-[3-(trifluoromethyl)phenyl]pyrazolo[1,5-a]pyrimidine

To a stirred mixture of 25.0 g of 7-[3-(trifluoromethyl)phenyl]pyrazolo[1,5-a]pyrimidine in 250 ml of glacial acetic acid, at room temperature, under nitrogen was added portionwise 14.92 g of sodium cyanoborohydride. The resultant solution was stirred for 24 hours, and then was evaporated in vacuo to give an oil. The oil was dissolved in chloroform and washed with satured sodium bicarbonate. The organic layer was separated, dried over anhydrous sodium sulfate and filtered. The filtrate was evaporated in vacuo to give a semi-solid which was chromatographed on a silica gel column using a mixture of 25% ethyl acetate and 75% hexane as the solvent system. The product fraction was collected and evaporated to give 6.2 g of the desired product as orange crystals, mp 61-64°C.

Example 62

7-(2,5-Dichlorophenyl)-4,5,6,7-tetrahydro-6-methylpyrazolo[1,5-a]pyrimidine-3-carbonitrile

A mixture of 50.0 g of 2,5-dichloroacetophenone and 50 ml of N,N-dimethylacetamide dimethyl acetal was stirred and heated at reflux for 12 1/2 hours. The mixture was then cooled and evaporated in vacuo to give an oil which crystallized on scratching to give a dark red solid. This was collected and washed with hexane/ether to give 58.8 g of 1-(2,5-dichlorophenyl)-3-(dimethylamino)-2-buten-1-one as a red solid.

A mixture of 58.8 g of the preceding product and 24.5 g of 3-aminopyrazole-4-carbonitrile in 400 ml of glacial acetic acid was heated at reflux for about 5 hours. The reaction mixture was cooled and was diluted with water and the precipitate that formed was collected by filtration. This was washed several times with water and dried to give 68.5 g of 7-(2,5-dichlorophenyl)-5-methylpyrazolo[1,5-a]pyrimidine-3-carbonitrile as a light yellow solid, mp 229-230°C.

To a stirred mixture of 68.5 g of the above product in one liter of glacial acetic acid warmed on a steam bath was added, under nitrogen, portionwise, 28.4 g of sodium cyanoborohydride. Heating and stirring was continued for about 6 hours, then an additional 21.6 g of sodium cyanoborohydride was added and stirring and heating was continued for 5 1/2 hours. The reaction mixture was filtered hot to remove insoluble material totaling 23.1 g (A). The filtrate was cooled in an ice bath to crystallize a solid which was collected by filtration and washed with water to yield 21.9 g (B). The filtrate from (B) was diluted with water to give an additional precipitate which was collected after standing and gave 19.07 g (C).

A mixture comprised of 10.0 g of (A), 10.0 g of (B) and 10.0 g of (C) was stirred in 150 ml of methanol and 500 ml of tetrahydrofuran, under nitrogen and then 12.44 g of sodium borohydride was added portionwise. The mixture was heated under reflux for 7 hours and then filtered hot. The filtrate was cooled to room temperature and water was added to separate and oil which was extracted into chloroform. The organic layer was separated, dried over anhydrous sodium sulfate and filtered. The filtrate was evaporated in vacuo to give an off-white solid which was triturated with ether/hexane to give 19.4 g of the product of the example as a white solid, mp 184-185°C.

Example 63

7-(3,4-Dichlorophenyl)-4,5-dihydropyrazolo[1,5-a]pyrimidine-3-carbonitrile

A mixture of 50.0 g of 3,4-dichloroacetophenone and 75 ml of N,N-dimethylformamide dimethyl acetal was heated under reflux for 6 hours, then allowed to cool for 16 hours. The crystalline precipitate was collected by filtration, washed with hexane and dried. The addition of hexane to the above filtrate precipitated some additional product which was collected by filtration. The precipitates were combined, washed with hexane and dried in vacuo to give 55.0 g of 3′,4′-dichloro-3-dimethylaminoacrylophenone as bright yellow crystals.

A mixture of 55.0 g of the preceding product and 24.3 g of 3-aminopyrazole-4-carbonitrile in 500 ml of glacial acetic acid was heated at reflux for 5 hours. The reaction mixture was allowed to stand at room temperature for 16 hours. The solid that separated was collected by filtration, washed with water, then aqueous ammonia, followed by additional water. The product was dried in vacuo to give 62.5 g of 7-(3,4-dichlorophenyl)pyrazolo[1,5-a]pyrimidine-3-carbonitrile as light yellow crystals.

A 25.0 g portion of the preceding product was then combined with one liter of acetic acid and to this mixture was added excess sodium cyanoborohydride in portions. The mixture was stirred for two days under nitrogen while the temperature was maintained by a steam bath. The solid that precipitated from solution was recrystallized from ethanol to give 12.0 g of 7-(3,4-dichlorophenyl)-4,5-dihydropyrazolo[1,5-a]-pyrimidine-3-carbonitrile as a light yellow solid, mp 254-256°C.

The 4,5-dihydro and 4,5,6,7-tetrahydropyrazolo[1,5-a]pyrimidines are useful intermediates in the synthesis of compounds of the invention 4-[(substituted)alkylcarbonyl]-4,5-dihydro and -4,5,6,7-tetrahydro-7-[-(substituted)phenyl]pyrazolo[1,5-a]pyrimidine-3-carbonitriles represented by the following formula:

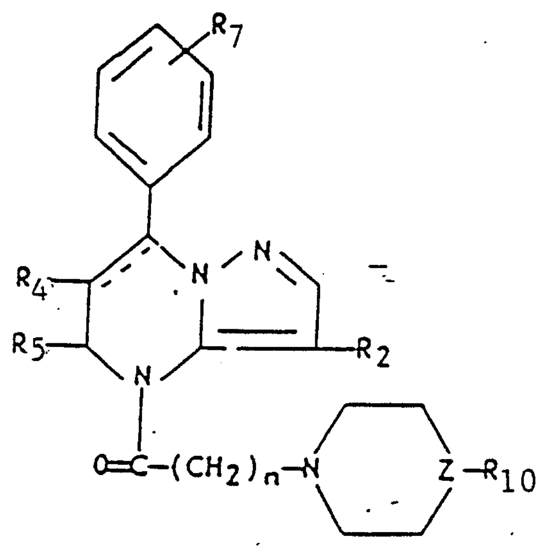

wherein N, $R_2$, $R_4$, $R_5$, $R_7$ and $R_{10}$ are as defined in formula Ia or Ib, above.

The novel 4-[(substituted)alkylcarbonyl]-4,5-dihydro and -4,5,6,7-tetrahydro-7-[(substituted)phenyl]-pyrazolo[1,5-a]pyrimidine-3-carbonitrile compounds of the present invention may be readily prepared as set forth in the following reaction scheme:

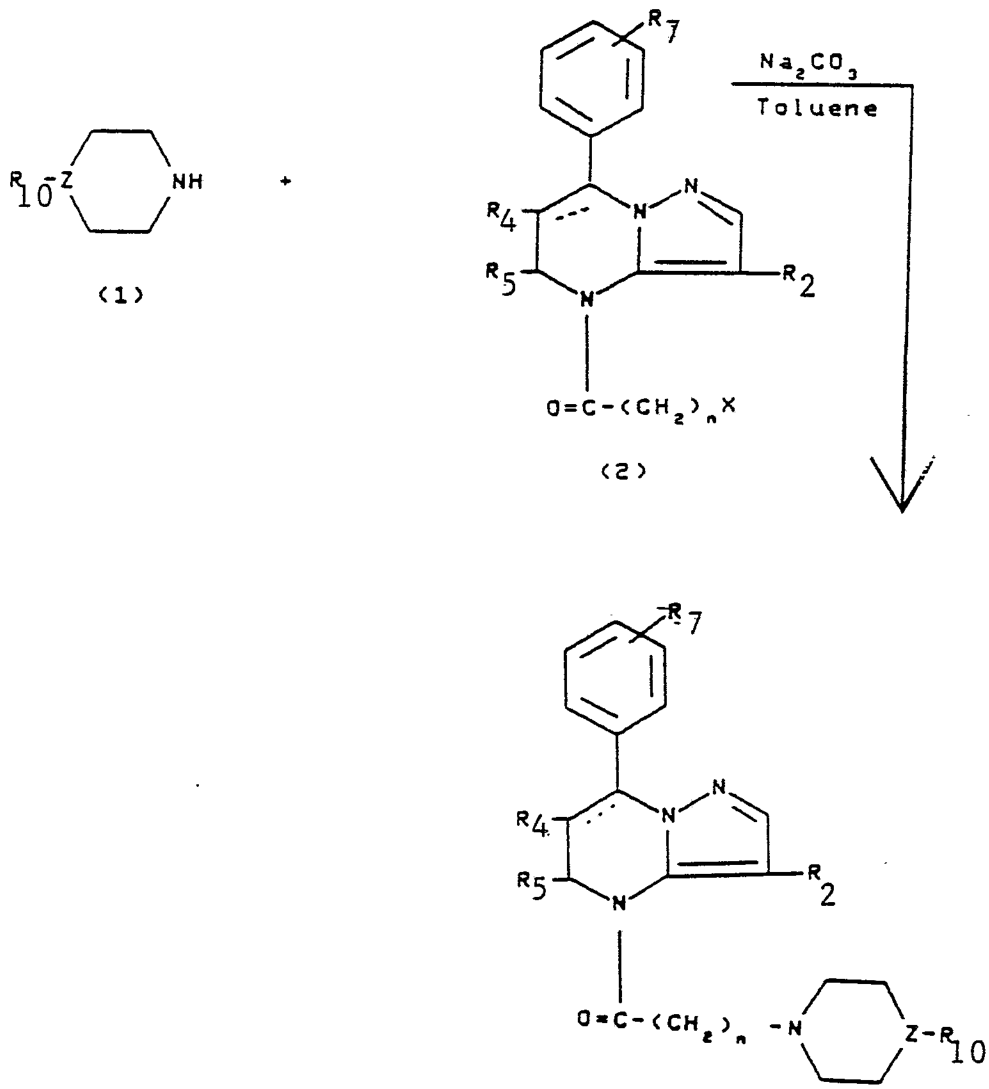


(1)

(2)

(3)

In accordance with the reaction scheme, above, an amine (1) where Z and $R_{10}$ are as described above is reacted with a haloalkanoyl-4,5-dihydro or -4,5,6,7-tetrahydro-7-substituted phenyl pyrazolo[1,5-a]-pyrimidine-3-carbonitrile (2) wherein n, $R_7$, $R_2$, $R_4$ and $R_5$ are as described above, X is a halide such as chloro or iodo, and an alkaline carbonate in toluene at reflux giving the products (3).

The pharmacological activity of these compounds of this invention is demonstrated in the following tests.

A test used to assess antianxiety effects is non-conditioned passive avoidance procedure described by J. R. Vogel, B. Beer and D. E. Clody, "A Simple and Reliable Conflict Procedure for Testing Antianxiety Agents", Psychlopharmacologia, 21, 1-7 (1971). A conflict situation is induced in rats by a modification of this method as described for Table II, above. Results of this in vivo test on representative active compounds of the present invention are given in Table VI.

TABLE VI

Conflict Procedure in Rats

| Compound | Dose (mg/kg) | Result (No. of shocks per 3 min.) |
|---|---|---|
| 4,5-Dihydro-4-[(4-methyl-1-piperazinyl)acetyl]-7-[3-(trifluoromethyl)phenyl]pyrazolo-[1,5-a]pyrimidine-3-carbonitrile, dihydrochloride | 25 | 17.4 |
| 4-[[4-(2-Cyclohexylethyl)-1-piperazinyl]acetyl]-4,5-dihydro-7-[3-(trifluoromethyl)phenyl]-pyrazolo[1,5-a]pyrimidine-3-carbonitrile | 25 | 18.0 |

Another test utilized for the determination of anxiolytic activity is the measurement of the ability of a test compound to inhibit the binding of tritiate benzodiazepines to brain-specific receptors of mammals. A modification of the method described by R. F. Squares, et al., Nature, 266 (21), 732 (April, 1977) and H. Mohler, et al., Science, 198, 849 (1977) was employed.

Male albino rates (Wistar strain, weighting 150-200 g each) were obtained from Royalhart Farms. $^3$H-Methyldiazepam (79.9 Ci/mmol) and $^3$H-methyl-flunitrazepam (84.3 Ci/mmol were obtained from New England Nuclear. The test compounds were solubilized in dimethylformamide, acetic acid, ethanol or hydrochloric acid. The procedure described for Table III, above, was followed.

The results of this *in vitro* test on representative compounds of this invention are given in Table VII.

TABLE VII

Inhibition of the Binding of $^3H$-Benzodiazepine to Brain-Specific Receptors of Rats

| Compound | % Inhibition |
|---|---|
| 4,5-Dihydro-4-[(4-methyl-1-piperazinyl)-acetyl]-7-[3-(trifluoromethyl)phenyl]-pyrazolo[1,5-a]pyrimidine-3-carbonitrile | 41 |
| 4,5-Dihydro-4-[[4-(phenylmethyl)-1-piperazinyl]acetyl]-7-[3-(trifluoromethyl)phenyl]-pyrazolo[1,5-a]pyrimidine-3-carbonitrile | 17 |
| 4[[4-(3,4-Dichlorophenyl)-1-piperazinyl]-acetyl]-4,5-dihydro-7-[3-(trifluoromethyl)-phenyl]pyrazolo[1,5-a]pyrimidine-3-carbonitrile, hydrochloride | 12 |
| 4-[(Dihexylamino)acetyl]-4,5-dihydro-7-[3-(trifluoromethyl)phenyl]pyrazolo[1,5-a]-pyrimidine-3-carbonitrile | 24 |
| 4,5-Dihydro-4-[[4-[2-(4-morpholinyl)-2-oxoethyl]-1-piperazinyl]acetyl]-7-[3-(trifluoromethyl)phenyl]pyrazolo[1,5-a]pyrimidine-3-carbonitrile | 41 |
| 4-[2-[3-Cyano-7-[3-(trifluoromethyl)-phenyl]pyrazolo[1,5-a]pyrimidin-4(5H)-yl]-2-oxoethyl]-N-(1-methylethyl)-1-piperazine-acetamide | 41 |
| 4,5-Dihydro-4-[[4-[2-oxo-2-(1-pyrrolidinyl)-ethyl]-1-piperazinyl]acetyl]-7-[3-(trifluoromethyl)phenyl]pyrazolo[1,5-a]pyrimidine-3-carbonitrile | 33 |
| 4-[[4-(2-Cyclohexylethyl)-1-piperazinyl]-acetyl]-4,5-dihydro-7-[3-(trifluoromethyl)-phenyl]pyrazolo[1,5-a]pyrimidine-3-carbonitrile, dihydrochloride | 14 |
| 4-[2-[3-Cyano-7-[3-(trifluoromethyl)phenyl]-pyrazolo[1,5-a]pyrimidin-4(5H)-yl]-2-oxo-ethyl]-1-piperazinecarboxylic acid, ethyl ester | 45 |

TABLE VII (continued)

| Compound | % Inhibition |
|---|---|
| 4-[[4-(2-Furanylcarbonyl)-1-piperazinyl]-acetyl]-4,5-dihydro-7-[(3-trifluoromethyl)-phenyl]pyrazolo[1,5-a]pyrimidine-3-carbonitrile, hydrochloride | 12 |
| 4,5-Dihydro-4-(1-piperazinylacetyl)-7-[3-(trifluoromethyl)phenyl]pyrazolo[1,5-a]-pyrimidine-3-carbonitrile | 25 |
| 4-[2-[3-Cyano-7-[3-(trifluoromethyl)phenyl]-pyrazolo[1,5-a]pyrimidin-4(5H)-yl]-2-oxoethyl]-N-phenyl-1-piperazinecarboxamide | 24 |
| N-(5-Chloro-2-methoxyphenyl)-4-[2-[3-cyano-7-[3-(trifluoromethyl)phenyl]pyrazolo-[1,5-a]pyrimidin-4(5H)-yl]-2-oxoethyl]-1-piperazineacetamide | 15 |
| 4,5,6,7-Tetrahydro-4-[[4-phenylmethyl-1-piperazinyl]acetyl]-7-[3-(trifluoromethyl)-phenyl]pyrazolo[1,5-a]pyrimidine-3-carbonitrile | 14 |
| 4,5,6,7-Tetrahydro-4-[[4-(phenylmethyl)-1-piperazinyl]acetyl]-7-[3-(trifluoromethyl)-phenyl]pyrazolo[1,5-a]pyrimidine-3-carbonitrile, dihydrochloride | 12 |

Certain of the novel compounds of the present invention are active hypotensive agents at nontoxic doses when administered to mammals. These compounds were tested for hypotensive activity by the method of P. S. Chan and D. W. Poorvin, Clinical and Experimental Hypertension, 1 (6), 817-830 (1979) as described for Table IV, above.

The results of this test on representative compounds of the present invention appear below in Table VIII.

TABLE VIII

Reduction of Mean Arterial Blood Pressure in Spontaneously Hypertensive Rats

| Compound | MABP/mmHg |
|---|---|
| 4,5-Dihydro-4-[[4-(phenylmethyl)-1-piperazinyl]acetyl]-7-[3-(trifluoromethyl)phenyl]-pyrazolo[1,5-a]pyrimidine-3-carbonitrile | 120 |
| 4,5-Dihydro-4-[[4-(phenylmethyl)-1-piperazinyl]acetyl]-7-[3-(trifluoromethyl)phenyl]-pyrazolo[1,5-a]pyrimidine-3-carbonitrile, dihydrochloride | 137 |
| 4-[[4-[(4-Chlorophenyl)phenylmethyl]-1-piperazinyl]acetyl]-4,5-dihydro-7-[3-(trifluoromethyl)phenyl]pyrazolo[1,5-a]pyrimidine-3-carbonitrile | 134 |
| 4,5-Dihydro-4-[(4-phenyl-1-piperazinyl)-acetyl]-7-[3-(trifluoromethyl)phenyl]pyrazolo-[1,5-a]pyrimidine-3-carbonitrile | 137 |
| 4-[[4-(3-Chlorophenyl)-1-piperazinyl]acetyl]-4,5-dihydro-7-[3-(trifluoromethyl)phenyl]-pyrazolo[1,5-a]pyrimidine-3-carbonitrile | 124 |
| 4,5-Dihydro-4-[[4-(2-pyrimidinyl)-1-piperazinyl]acetyl]-7-[3-(trifluoromethyl)phenyl]-pyrazolo[1,5-a]pyrimidine-3-carbonitrile | 127 |

TABLE VIII (continued)

| Compound | MABP/mmHg |
|---|---|
| 4-[[4-(4-Fluorophenyl)-1-piperazinyl]acetyl]-4,5-dihydro-7-[3-(trifluoromethyl)phenyl]-pyrazolo[1,5-a]pyrimidine-3-carbonitrile | 130 |
| 4-[3-[4-[(4-Chlorophenyl)methyl]-1-piperazinyl]-1-oxopropyl]-4,5-dihydro-7-[3-(trifluoromethyl)phenyl]pyrazolo[1,5-a]pyrimidine-3-carbonitrile | 138 |
| 4,5-Dihydro-4-[[4-[2-(4-morpholinyl)-2-oxoethyl]-1-piperazinyl]acetyl]-7-[3-(trifluoromethyl)phenyl]pyrazolo[1,5-a]pyrimidine-3-carbonitrile | 129 |
| 4-[2-[3-Cyano-7-[3-(trifluoromethyl)phenyl]-pyrazolo[1,5-a]pyrimidin-4(5H)-yl]-2-oxoethyl]-N-(1-methylethyl)-1-piperazineacetamide | 129 |
| 4-[[4-[Bis(4-fluorophenyl)methyl]-1-piperazinyl]acetyl]-4,5-dihydro-7-[3-(trifluoromethyl)phenyl]pyrazolo[1,5-a]pyrimidine-3-carbonitrile | 121 |
| 4-[2-[3-Cyano-7-[3-(trifluoromethyl)phenyl]-pyrazolo[1,5-a]pyrimidin-4(5H)-yl]-2-oxoethyl]-N-phenyl-1-piperazinecarboxamide | 125 |
| N-(5-Chloro-2-methoxyphenyl)-4-[2-[3-cyano-7-[3-(trifluoromethyl)phenyl]pyrazolo[1,5-a]-pyrimidin-4(5H)-yl]-2-oxoethyl]-1-piperazine-acetamide | 139 |
| 4,5-Dihydro-4-[[4-(2-propenyl)-1-piperazinyl]-acetyl]-7-[3-(trifluoromethyl)phenyl]pyrazolo-[1,5-a]pyrimidine-3-carbonitrile | 139 |
| 4-[(4-Cyclobutyl-1-piperazinyl)acetyl]-4,5-dihydro-7-[3-(trifluoromethyl)phenyl]pyrazolo-[1,5-a]pyrimidine-3-carbonitrile | 124 |
| 4,5-Dihydro-4-[[4-(1-methylethyl)-1-piperazinyl]acetyl]-7-[3-(trifluoromethyl)phenyl]-pyrazolo[1,5-a]pyrimidine-3-carbonitrile | 139 |

The novel compounds of the present invention possess the ability to enhance neural function in warm blooded animals affected by behavioral neurological problems, including the cognitive deterioration associated with decreased neural function which occurs with cerebral insufficiency, aging, dementia, and similar conditions.

A useful in vivo test associated with decreased neural function in mammals is the Passive Avoidance Anoxic Induced Amnesia Test. This test is used to determine the attenuation of anoxic induced amnesia in mice treated with drug, as compared to saline treated control animals with no drug.

A shock-motivated, single trial, step-through passive avoidance procedure is used. Groups of 25 Swiss-Webster, middle-aged mice (9 months of age) are place singly in the front chamber of a 2-chamber box and are allowed to voluntarily cross into the rear chamber. As soon as the mouse enters the rear chamber, a door automatically traps the animal and a mild electric shock (0.4 mA for 4 seconds) is delivered to its feet. Following the foot shook, the mice are initially placed in the anoxic environment (0% oxygen) for 12 seconds, which quickly induces unconsciousness. They are then placed in an hypoxic environment (15% oxygen) for four minutes which prolongs the oxygen deprived state, maintaining unconsciousness. All testing is performed 24 hours later, and in all cases the mice appear fully recovered from the previous anoxic/hypoxic treatment. All test compounds are administered intraperitoneally at a dose of 10-200 mg/kg, 30 minutes prior to training the testing. Control animals are injected intraperitoneally only with saline at 0.01 ml/g of body weight.

The latent period prior to entering the rear chamber is recorded for both training and testing sessions. The greater the memory of being shocked, the greater the reluctance to enter into the rear chamber and the higher will be the latent period prior to re-entry. An improvement (lengthened latency) of 30% over saline control scores in considered active. The result of this test on a representative compound of the present invention appears in Table IX.

TABLE IX

Passive Avoidance Anoxic Induced Amnesia Test

| Compound | Dose (mg/kg) | % Improvement |
|---|---|---|
| 4,5-Dihydro-4-[[4-(phenylmethyl)-1-piperazinyl]acetyl]-7-[3-(trifluoromethyl)phenyl]pyrazolo-[1,5-a]pyrimidine-3-carbonitrile | 100<br>200 | 65<br>93 |

The compounds of this invention are useful as antidepressant agents in warm-blooded animals as verified in the following tests. First, the antidepressant properties of these compounds were tested by measuring their ability to counteract depression induced in animals by the administration of tetrabenazine methanesulfonate. Each test compound was administered orally to 10 mice at a dose of 25 mg/kg of body weight. Thirty minutes later, tetrabenazine methanesulfonate was administered intraperitoneally to each mouse at a dose of 39 mg/kg of body weight, which dose in known to depress markedly the exploratory behavior of normal mice. Thirty minutes later the mice were tested for their exploratory behavior was described by E. Greenblatt and A. C. Osterberg, Toxicology and Applied Pharmacology, 7, 566-578 (1965). A compound is considered active if 3 or more of 10 mice are protected against the tetrabenazine-induced effects.

The results of this test on representative compounds of this invention appear in Table X.

## TABLE X

### Tetrabenazine-Induced Depression

| Compound | Result |
|---|---|
| 4,5-Dihydro-4-[[4-(phenylmethyl)-1-piperazinyl]-acetyl]-7-[3-(trifluoromethyl)phenyl]pyrazolo-[1,5-a]pyrimidine-3-carbonitrile | Active |
| 4,5-Dihydro-4-[[4-[(3-methoxyphenyl)methyl]-1-piperazinyl]acetyl]-7-[3-(trifluoromethyl)phenyl]-pyrazolo[1,5-a]pyrimidine-3-carbonitrile | Active |
| 4,5-Dihydro-4-[[4-[(3-methoxyphenyl)methyl]-1-piperazinyl]acetyl]-7-[3-(trifluoromethyl)phenyl]-pyrazolo[1,5-a]pyrimidine-3-carbonitrile, dihydrochloride | Active |
| 4-[[4-(1,3-Benzodioxol-5-ylmethyl)-1-piperazinyl]-acetyl]-4,5-dihydro-7-[3-(trifluoromethyl)-phenyl]pyrazolo[1,5-a]pyrimidine-3-carbonitrile | Active |

The activity of the compound of this invention as antidepressant agents was further verified in the following test which measures the ability of a test compound to inhibit $^3$H-imipramine binding to human platelet membranes.

Platelet membranes were obtained using the procedure described by Wennogle, L. P., et al., Pharmac. Biochem. Behav., 15, 975 (1981). Fresh human platelet concentrates (less than 48 hours old) were obtained from the New York Blood Center. These concentrates were prepared in a citrate-dextrose anticoagulant using standard techniques. Platelets were washed twice by centrifugation (2500 x G, 10 minutes) in 50 volumes of an antiprotease buffer containing 0.005M Tris potassium chloride, 0.12M sodium chloride, 0.05M Tris pH 7.5, 0.025 Units/ml aprotinin, 0.05 µg/m pepstatin, 2 x $10^{-5}$ bacitracin, 3mM ethylenediaminetetraacetic acid and 1.0mM ethyleneglycol-bis (β-aminoethyl ether)-N,N'-tetraacetic acid. Antiproteases have been shown to inhibit the breakdown of the $^3$H-imipramine receptor [Wennogle, L. P., et al . (vide supra)]. All procedures were conducted at 0°C using plastic laboratory-ware throughout the platelet membrane preparation. Platelets were resuspended in 20 volumes of buffer and sonicated 3 times using 10 second bursts of a Branson sonifier (cell disrupter 350) at setting 6 (standard 3/4 inch horn), keeping the sample on ice thoroughout the procedure. Platelet membranes were then washed twice at 18,000 x G for 20 minutes with 50 volumes of the antiprotease buffer and resuspended to 3.0 mg. protein/ml using the Bradford protein analysis with bovine gamma globulin as standard. Membranes were used immediately or stored frozen in liquid nitrogen.

The displacement of $^3$H-imipramine binding to human platelet membranes was performed essentially as described by Paul, S. M., et al., Life Sci., 26, 953 (1980) with the following modifications. Membranes (0.3 mg protein) were suspended in antiprotease buffer with both 3.0 mM $^3$H-imipramine (New England Nuclear) and displacing drug or buffer in a total volume of 250 µl in glass test tubes. Samples were incubated for 90 minutes at 0°C, then diluted to 4 ml in 0.12M sodium chloride, 0.005M potassium chloride, 0.05M Tris pH 7.5 (wash buffer) and immediately filtered through GF/B Whatman Filters and washed twice with 5 ml of wash buffer. Filters were counted in a liquid scintillation counter after addition of Beckman HP Scintillant.

Non-specific binding of $^3$H-imipramine was defined as that fraction (generally 35%) of radioactivity that was not displaced by 10 M desmethylimipramine. Specific binding was determined by subtraction of this non-specific level from values for total $^3$H-imipramine binding which were measured by incubating $^3$H-imipramine in the absence of displacing drug. Compounds were tested in duplicate test tubes at a concentration of 10 M. Compounds which inhibited binding by more than 50% were considered to be active.

The results of this test on representative compounds of this invention appear in Table XI.

TABLE XI

$^3$H -Imipramine Binding to Human Platelet Membranes

| Compound | % Inhibition |
|---|---|
| 4,5-Dihydro-4-[[4-[(3-methoxyphenyl)methyl]-1-piperazinyl]acetyl]-7-[3-(trifluoromethyl)phenyl]pyrazolo[1,5-a]pyrimidine-3-carbonitrile | 78 |
| 4-[[4-(1,3-Benzodioxol-5-ylmethyl)-1-piperazinyl]acetyl]-4,5-dihydro-7-[3-(trifluoromethyl)phenyl]pyrazolo[1,5-a]pyrimidine-3-carbonitrile | 81 |
| 4-[[4-[2-(Dimethylamino)ethyl]-1-piperazinyl]acetyl]-4,5-dihydro-7-[3-(trifluoromethyl)phenyl]pyrazolo[1,5-a]pyrimidine-3-carbonitrile, dihydrochloride | 72 |
| 4-[[4-[3-(Dimethylamino)propyl]-1-piperazinyl]acetyl]-4,5-dihydro-7-[3-(trifluoromethyl)phenyl]pyrazolo[1,5-a]pyrimidine-3-carbonitrile | 89 |
| 4-[3-[4-[(4-Chlorophenyl)methyl]-1-piperazinyl]-1-oxopropyl]-4,5-dihydro-7-[3-trifluoromethyl)phenyl)pyrazolo[1,5-a]pyrimidine-3-carbonitrile, dihydrochloride | 85 |

TABLE XI (continued)

| Compound | % Inhibition |
|---|---|
| 4-[[4-[(4-Chlorophenyl)methyl]-1-piperazinyl]acetyl]-7-[3-(trifluoromethyl)phenyl]-pyrazolo[1,5-a]pyrimidine-3-carbonitrile | 84 |
| 4,5-Dihydro-4-[1-oxo-3-[4-(phenylmethyl)-1-piperazinyl]propyl]-7-[3-(trifluoromethyl)phenyl]pyrazolo[1,5-a]pyrimidine-3-carbonitrile | 80 |
| 4,5,6,7-Tetrahydro-4-[[4-(3-phenoxypropyl)-1-piperazinyl]acetyl]-7-[3-(trifluoromethyl)phenyl]pyrazolo[1,5-a]pyrimidine-3-carbonitrile | 57 |
| 7-(2,5-Dichlorophenyl)-4,5-dihydro-4-[1-oxo-3-[4-(phenylmethyl)-1-piperazinyl]propyl]pryazolo[1,5-a]pyrimidine-3-carbonitrile | 79 |

The hypoxic survival test is a useful *in vivo* test for measuring the effectiveness of central nervous system-acting drugs in enhancing survival in an hypoxic environment relative to the known parasympathomimetic agent physostigmine. This assay shows the enhanced survival of animals in an hypoxic environment after treatment with drug as compared to saline-treated control animals.

Extensive testing has demonstrated that under conditions of 10% oxygen, only 5-20% of control mice survive after 5 minutes, whereas 60-80% of the physostigimine-treated mice survive. Drugs are tested by intraperitoneal injection of groups of mice 30 minutes prior to placing them in an hypoxic environment and measuring survival. The rationale of this test is that drugs which enhance survival under hypoxic conditions without concomitant depression or sedative side effects, do so by enhancing brain metabolism, i.e., by improving energy supply relative to demand and so preserving normal brain function under conditions of reduced energy metabolism. Considering the dependence of the brain on a constant supply of energy, drugs which have this property may have many far-reaching therapeutic indications, including aiding recovery from stroke and closed head injury and reducing the deleterious effects of age-related central nervous system deficits. For example, deficiencies in energy metabolism are thought to contribute significantly to the neurochemical and neurophysiological dysfunctions of aging and senile dementia.

In the hypoxic survival test, groups of 20 Royal Hart mice (6 weeks of age) were injected intraperitoneally with a test compound in saline at various doses ranging from 1 to 200 mg/kg, 30 minutes prior to placing them in a hypoxic mixture (10% oxygen, 90% carbon dioxide) and measuring survival after 5 minutes.

A control group of 20 mice were injected intraperitoneally with saline (0.01 ml/g of body weight) and processed as described above.

Another group of 20 mice was injected intraperitoneally with 0.125 mg/kg of physostimine in saline and processed as described above.

Results of this test on representative compounds of this invention appear in Table XII.

TABLE XII

Hypoxic Survial Test

| Compound | Dose (mg/kg) | % Survivors |
|---|---|---|
| 4,5-Dihydro-4-[[4-(phenylmethyl)-1-piperazinyl]acetyl]-7-[3-(trifluoromethyl)phenyl]pyrazolo[1,5-a]pyrimidine-3-carbonitrile | 10<br>100 | 40<br>48 |
| 7-(4-Chlorophenyl)-4,5-dihydro-4-[[4-(phenylmethyl)-1-piperazinyl]acetyl]pyrazolo[1,5-a]pyrimidine-3-carbonitrile | 10<br>100 | 50<br>82.5 |
| 7-(4-Chlorophenyl)-4,5-dihydro-4-[[4-[[2-(trifluoromethyl)phenyl]methyl]-1-piperazinyl]acetyl]pyrazolo[1,5-a]pyrimidine-3-carbonitrile | 10<br>100 | 55<br>90 |
| 7-(2,5-Dichlorophenyl)-4-[[4-(2,5-dimethylphenyl)-1-piperazinyl]-acetyl]-4,5-dihydropyrazolo[1,5-a]pyrimidine-3-carbonitrile | 100<br>200 | 45<br>80 |
| 4,5-Dihydro-4-[[4-[4-(2-oxo-1-pyrrolidinyl)-2-butynyl]-1-piperazinyl]acetyl]-7-[3-(trifluoromethyl)phenyl]pyrazolo[1,5-a]pyrimidine-3-carbontrile | 100 | 68 |
| 4,5-Dihydro-5-methyl-4[[4-(phenylmethyl)-1-piperazinyl]acetyl]-7-[3(trifluoromethyl)phenyl]pyrazolo[1,5-a] pyrimidine-3-carbonitrile, dihydrochloride | 100 | 43 |

TABLE XII

Hypoxic Survial Test
(Continued)

| Compound | Dose (mg/kg) | % Survivors |
|---|---|---|
| 4,5-Dihydro-6-methyl-4[[4-(phenylmethyl)-1-piperazinyl]-acetyl]-7-[3-(trifluoromethyl)-phenyl]pyrazolo[1,5-a] pyrimidine-3-carbonitrile, dihydrochloride | 100 | 73 |
| 4,5,6,7-Tetrahydro-4-[[4-(phenylmethyl)-1-piperazinyl]acetyl]-7-[3-(trifluoromethyl)phenyl]pyrazolo-[1,5-a]pyridimine-3-carbonitrile | 100 | 80 |
| 4,5-Dihydro-4-[[4-(phenylmethyl)--1-piperazinyl]acetyl]-7-[3-(trifluoromethyl)phenyl]pyrazolo-[1,5-a]pyrimidine-3-carbonitrile, hydrochloride | 100 | 83 |
| 4,5-Dihydro-4-[[4-[(3-trifluoromethylphenyl)methyl]-1-piperazinyl]acetyl]-7-[3-(trifluoromethyl)phenyl]pyrazolo[1,5-a]-pyrimidine-3-carbonitrile | 100 | 40 |
| 4,5-Dihydro-4-[[4-[(3-trifluoromethyl-phenyl)methyl]-1-piperazinyl]acetyl]-7-[3-(trifluoromethyl)phenyl]pyrazolo[1,5-a]-pyrimidine-3-carbonitrile, dihydrochloride | 100 | 85 |
| 7-(3-Chlorophenyl)-4-[[4-[(3-chlorophenyl)methyl-1-piperazinyl]acetyl]-4,5-dihydro-6-methyl-pyrazolo-[1,5-a]pyrimidine-3-carbonitrile, dihydrochloride | 100<br>50 | 77<br>73 |
| 7-(4-Chlorophenyl)-4-[[4-[(3-chlorophenyl)methyl]-1-piperazinyl]acetyl]-4,5-dihydro-6-methylpyrazolo-[1,5-a]pyrimidine-3-carbonitrile, dihydrochloride | 200<br>100 | 73<br>62 |

TABLE XII

Hypoxic Survial Test
(Continued)

| Compound | Dose (mg/kg) | % Survivors |
|---|---|---|
| 7-(3-Chlorophenyl)-4,5-dihydro-6-methyl-4-[[4-[[3-(triflouromethyl)-phenyl]methyl]-1-piper-azinyl]acetyl]pyrazolo-[1,5-a]pyrimidine-3-carbonitrile | 200 | 73 |
| 4-[(4-Benzoyl-1-piper-azinyl)acetyl]-4,5-dihydro-7-[3-(trifluoromethyl)phenyl]pyrazolo-[1,5-a]pyrimidine-3-carbonitrile | 100 | 95 |
| 4,5-Dihydro-4-[[4-(2-phenylethyl)-1-piperazinyl]acetyl]-7-[3-(trifluoromethyl)phenyl]-pyrazolo[1,5-a]pryimidine--3-carbonitrile, dihydro-chloride | 100 | 60 |
| 4-[[4-[Bis(4-fluoro-phenyl)methyl)-1-piper-azinyl)acetyl]-4,5-dihydro-7-[3-(trifluoromethyl)phenyl]pyrazolo-[1,5-a]pyrimidine-3-carbonitrile, dihydro-chloride | 100 | 55 |
| 7-(2,5-Dichlorophenyl)-4,5-dihydro-4-[(4-phenyl-1-piperidinyl)acetyl]pyra-zolo[1,5-a]pyrimidine-3-carbonitrile | 100 | 70 |
| 7-(2,5-Dichlorophenyl)-4,5-dihydro-4-[[4-(phenyl-methyl)-1-piperidinyl]-acetyl]pyrazolo[1,5-a]-pyrimidine-3-carbonitrile | 100 | 50 |

Those novel compounds of the present invention that are effective for the treatment of depression and/or for the alleviation of anxiety in warm-blooded animals are administered in amounts ranging from about 0.1 mg to about 35.0 mg/kg of body weight per day. A preferred dosage regimen for optimum results would be from about 0.5 mg to about 20.0 mg/kg of body weight per day and such dosage units are employed that a total of from about 35 mg to about 1.4 g of the active compound for a subject of about 70 kg of body weight are administered in a 24 hour period.

Certain of the compounds of the present invention have been found to be useful as agents for the treatment of cognitive and related neural behavioral problems in mammals when administered in amounts ranging from about 5 mg to about 200 mg/kg of body weight per day. A preferred dosage regimen for optimum results would be from about 1 mg to about 50 mg/kg of body weight per day and such dosage units are employed that a total of from about 700 mg to about 3.5 g of the active compound for a subject of about 70 kg of body weight are administered in a 24 hour period.

The 4-[(substituted)alkylcarbonyl]-4,5-dihydro and -4,5,6,7-tetrahydro-7-[(substituted)-phenyl]-pyrazolo-[1,5-a]pyrimidine-3-carbonitriles of the invention will be described in greater detail in conjunction with the following non-limiting Examples 64-199.

Example 64

4-(Chloroacetyl)-4,5-dihydro-7-[3-trifluoromethyl)-phenyl]pyrazol[1,5-a]pyrimidine-3-carbonitrile

An 8.3 g portion of sodium cyanoborohydride was added portionwise to a solution of 20 g of 7-[3-(trifluoromethyl)phenyl]pyrazolo[1,5-a]pyrimidine-3-carbonitrile (U. S. Patent 4,178,449) in 400 ml of acetic acid at ice bath temperature with stirring under nitrogen. The reaction was stirred at ice bath temperature for 45 minutes, then at room temperature for 3 hours, 1.7 g of sodium cyanoborohydride was added and stirring was continued for 2 more hours.

The precipitate was collected, washed with water and saved.

The acetic acid solution was concentrated to 1/2 volume and diluted with 250 ml of water. The resulting precipitate was collected, washed twice with water, combined with the above precipitate and dissolved in 600 ml of dichloromethane. This solution was washed twice with saturated aqueous sodium bicarbonate, dried, filtered and concentrated to a pale yellow solid. This solid was recrystallized from hot isopropanol, giving 14.8 g of 4,5-dihydro-7-[3-(trifluoromethyl)phenyl]pyrazolo[1,5-a]pyrimidine-3-carbonitrile.

A mixture of 2.9 g of the above compound and 3.4 g of chloroacetic anhydride in 100 ml of toluene was heated with stirring at reflux for 5 hours. The reaction was cooled, 1 g of chloroacetic anhydride was added, the mixture was stirred 48 hours at room temperature, then refluxed for 5 hours and allowed to stand overnight at room temperature. The mixture was then concentrated in vacuo on a steam bath. The residue was slurried in ether and the white crystals collected, washed with ether and dried, giving 1.1 g of the desired intermediate, mp 179-181°C.

Example 65

4-(3-Chloro-1-oxopropyl)-4,5-dihydro-7-[3-(trifluoromethyl)phenyl]pyrazolo[1,5-a]pyrimidine-3-carbonitrile

To a stirred mixture of 4.5 g of 4,5-dihydro-7-[3-trifluoromethyl)phenyl]pyrazolo[1,5-a]pyrimidine-3-carbonitrile and 3.6 g of 1,8-bis(dimethylamino-naphthalene, N,N,N',N',-tetramethyl-1,8-naphthalenediamine in 120 ml of dry tetrahydrofuran, under nitrogen, was added dropwise, a solution of 3.9 g of 3-chloropropionyl chloride in 30 ml of tetrahydrofuran. The mixture was stirred for one hour, heated at reflux for 4 hours, then allowed to stand overnight. The mixture was filtered and the filtrate evaporated in vacuo on a steam bath. The residue was triturated with ether, filtered, washed with ether, dried and the solid was then heated to solution in 25 ml of acetonitrile and filtered. Refrigeration produced a solid which was collected, washed with ether and dried, giving 3 g of the desired product, mp 183-185°C.

Example 66

4-(Chloroacetyl)-7-(3-chlorophenyl)-4,5-dihydropyrazolo[1,5-a]pyrimidine-3-carbonitrile

A 23.4 g portion of 7-(3-chlorophenyl)-pyrazolo[1,5-a]pyrimidine-3-carbonitrile and 11.7 g of sodium cyanoborohydride in 500 ml of acetic acid were reacted as described in Example 1, giving 18.4 g of 4,5-dihydro-7-(3-chlorophenyl)pyrazolo[1,5-a]pyrimidine-3-carbonitrile.

An 18.1 g portion of the above compound and 15 g of chloracetic anhydride in 200 ml of toluene were reacted as described in Example 1, giving 15 g of the desired intermediate, mp 196-198°C.

Example 67

4,5-Dihydro-4-(iodoacetyl)-7-[3-(trifluoromethyl)phenyl]pyrazolo[1,5-a]pyrimidine-3-carbonitrile

A mixture of 1.8 g of 4-(chloroacetyl)-4,5-dihydro-7-[3-(trifluoromethyl)phenyl]pyrazolo[1,5-a]pyrimidine-3-carbonitrile and 825 mg of sodium iodide in 25 ml of acetone, under argon, was stirred and refluxed for 2 hours, then cooled and filtered. The filtrate was evaporated, the residue triturated with ether, filtered, washed with ether and dried. The solid was heated to solution in 10 ml of ethyl acetate, filtered, cooled, and after one hour 20 ml of hexane was added and the reaction recooled. The solid was collected, washed with hexane and dried, giving 1.3 g of the desired intermediate, mp 158-160°C.

Example 68

4-(Chloroacetyl)-4,5,6,7-tetrahydro-7-[3-(trifluoromethyl)phenyl]pyrazolo[1,5-a]pyrimidine-3-carbonitrile

A 9.0 g portion of 4,5-dihydro-7-[3-(trifluoromethyl)phenyl]pyrazolo[1,5-a]pyrimidine-3-carbonitrile in 90 ml of trifluoroacetic acid was stirred under argon and heated to 60-65°C. A 10.8 ml portion of triethylsilane was added and the mixture was stirred at 60-65°C for 5.5 hours, then allowed to stand at room temperature overnight. The mixture was poured into 300 ml of 25% aqueous potassium hydroxide containing cracked ice and extracted three times with chloroform. The extracts were combined, washed with saturated sodium chloride, dried, concentrated to 200 ml and filtered through hydrous magnesium silicate. The solvent was evaporated and the residue triturated with ether and dried, giving 5.9 g of 4,5,6,7-tetrahydro-7-[3-(trifluoromethyl)phenyl]pyrazolo[1,5-a]pyrimidine-3-carbonitrile.

A mixture of 5.9 g of the above compound and 10.2 g of chloroacetric anhydride in 60 ml of toluene, under argon, was stirred at reflux for 18 hours, then the solvent was removed in vacuo on a steam bath. The residual oil was dissolved in 25 ml of chloroform, filtered through hydrous magnesium silicate and concentrated to an oil. This oil was added to ether and the solid collected, giving the desired intermediate, mp 157-159°C.

Example 69

4-(Chloroacetyl)-7-(3-fluorophenyl)-4,5-dihydropyrazolo[1,5-a]pyrimidine-3-carbonitrile

7-(3-Fluorophenyl)pyrazolo[1,5-a]pyrimidine-3-carbonitrile was reacted as described in Example 66, giving 6 g of the desired intermediate, mp 190-193°C.

Example 70

4-(Chloroacetyl)-4,5-dihydro-7-(3-nitrophenyl)pyrazolo[1,5-a]pyrimidine-3-carbonitrile

7-(3-Nitrophenyl)pyrazolo[1,5-a]pyrimidine-3-carbonitrile was reacted as described in Example 66, giving 0.9 g of the desired intermediate, mp 164-167°C.

Example 71

4-(Chloroacetyl)-7-(4-fluorophenyl)-4,5-dihydropyrazolo[1,5-a]pyrimidine-3-carbonitrile

7-(4-Chlorophenyl)pyrazolo[1,5-a]pyrimidine-3-carbonitrile was reacted as described in Example 66, giving 12.44 g of the desired intermediate, mp 198-199°C.

Example 72

4-(Chloroacetyl)-7-(2,5-dichlorophenyl)-4,5-dihydropyrazolo[1,5-a]pyrimidine-3-carbonitrile

7-(2,5-Dichlorophenyl)pyrazolo[1,5-a]pyrimidine-3-carbonitrile was reacted as described in Example 66, giving 39.3 g of the desired intermediate, mp 235-236°C.

Example 73

4-(Chloroacetyl)-4,5-dihydro-6-methyl-7-[3-(trifluoromethyl)phenyl]pyrazolo[1,5-a]pyrimidine-3-carbonitrile

6-Methyl-7-[3-(trifluoromethyl)phenyl]pyrazolo[1,5-a]pyrimidine-3-carbonitrile was reacted as described in Example 66, giving 39.3 g of the desired intermediate, mp 178-182°C.

Example 74

4-(Chloroacetyl)-4,5-dihydro-5-methyl-7-[3-(trifluoromethyl)phenyl]pyrazolo[1,5-a]pyrimidine-3-carbonitrile

5-Methyl-7-[3-(fluoromethyl)phenyl]pyrazolo[1,5-a]pyrimidine-3-carbonitrile was reacted as described in Example 66, giving 6.2 g of the desired intermediate, mp 160-163°C.

Example 75

4-(Chloroacetyl)-7-(3-fluorophenyl)-4,5-dihydro-6-methylpyrazolo[1,5-a]pyrimidine-3-carbonitrile

7-(Chloroacetyl)-7-(3-chlorophenyl)-4,5-dihydro-6-methylpyrazolo[1,5a ]pyrimidine-3-carbonitrile was reacted as described in Example 66, giving 12.5 g of the desired intermediate, mp 132-135°C.

Example 76

4-(Chloroacetyl)-4,5-dihydro-7-[3-(trifluoromethyl)phenyl]pyrazolo[1,5-a]pyrimidine-3-carboxylic acid, ethyl ester

A 100 g portion of ethyl (ethoxymethylene)cyanaoacetate was carfully poured into a solution of 50 ml of 85% hydrazine hydrate in 115 ml of water. The mixture was stirred for one hour, then cooled in an ice bath. The crystals which formed were collected, dissolved in dichloromethane, filtered through hydrous magnesium silicate, the filtrate cooled in an ice bath and hexane added. The resulting crystals were collected (40.9 g).

The above crystals and 62.7 g of 3-dimethylamino-3′-(trifluoromethyl)acrylophenone in 250 ml of acetic acid were stirred and heated at reflux for 16 hours and then evaporated to an oil. The oil was dissolved in 500 ml of dichloromethane and washed with aqueous sodium bicarbonate. Then the organic layer was dried, filtered and evaporated to an oil. This oil was dissolved in isopropanol on a steam bath then placed in an ice bath overnight. Filtration gave 67.1 g of white crystals.

The above 67.1 g of crystals were dissolved in 250 ml of glacial acetic acid and 35.5 g of sodium cyanoborohydride were added under nitrogen. The mixture was stirred for 3 hours and then evaporated to an oil. The oil was dissolved in dichloromethane, washed with aqueous sodium bicarbonate and on standing a solid formed. The solid was dissolved in 500 ml of isopropanol with heating, then chilled giving 50.3 g of solid.

To 6.8 g of the above solid and 4.0 g of 1,8-bis(dimethylamino)naphthalene, N,N,N',N',-tetramethyl-1,8-naphthalenediamine in 100 ml of dry tetrahydrofuran, was added with stirring, 4.50 g of chloroacetyl chloride in 100 ml of dry tetrahydrofuran, dropwise over 2 hours under a nitrogen atmosphere. The mixture was stirred overnight and then filtered. The filtrate was evaporated to a semi-solid and this was crystallized from ether to give 5.0 g of the desired intermediate, mp 141-142°C.

Example 77

4-(Chloroacetyl)-7-(4-chlorophenyl)-4,5-dihydro-6-methylpyrazolo[1,5-a]pyrimidine-3-carbonitrile

A 10 g portion of 7-(7-chlorophenyl)-4,5-dihydro-6-methylpyrazolo[1,5-a]pyrimidine-3-carbonitrile and 9 g of 1,8-bis(dimethylamino)naphthalene, N,N,N', N'-tetramethyl-1,8-naphthalenediamine in 300 ml of dry tetrahydrofuran under nitrogen was mixed and 6 ml of chloroacetyl chloride in 70 ml of dry tetrahydrofuran was added over 15 minutes with stirring. The mixture was stirred at reflux for 5.5 hours, then at room temperature overnight and then filtered. The filtrated was concentrated to an oil. This oil was triturated with ether, the solid collected and dried, giving 10 g of the desired intermediate, mp 138-140°C.

Example 78

7-(4-Chlorophenyl)-4-(iodoacetyl)-4,5-dihydropyrazolo[1,5-a]pyrimidine-3-carbonitrile

A mixture of 15,67 g of 4-(chloroacetyl)-7-(4-chlorophenyl)-4,5-dihydropyrazolo[1,5-a]pyrimidine-3-carbonitrile and 15.61 g of potassium iodine in 150 ml of acetone was heated and stirred at reflux for 6.5 hours and then filtered. The filtrate was evaporated and the residue triturated with ether, giving 4.51 g of the desired intermediate.

Example 79

4,5-Dihydro-4-[(4-methyl-1-piperazinyl)acetyl]-7-[3-(trifluoromethyl)phenyl]pyrazolo[1,5-a]pyrimidine-3-carbonitrile

A mixture of 1.3 g of N-methylpiperazine, 4.4 g of 4-(chloroacetyl)-4,5-dihydro-7-[3-(trifluoromethyl)-phenyl]pyrazolo[1,5-a]pyrimidine-3-carbonitrile and 1.5 g of sodium carbonate in 75 ml of toluene was refluxed for 4 hours, then allowed to stand at room temperature overnight. The mixture was treated with 30 ml of 1N sodium hydroxide and the phases were separated. The aqueous phase was extracted twice with chloroform. The organic phases were combined, washed with water, dried, filtered and concentrated. The residue was heated to solution in 50 ml of ethyl acetate, filtered and cooled. The crystalline solid was collected, washed with ethyl acetate and hexane and dried, giving 1.8 g of the desired product, mp 178-180°C.

Following the general procedure of Example 79, using the intermediates of Examples 64-78 and the indicated piperazines, the products of Examples 80-140 found in Table XIII were obtained.

TABLE XIII

| Example | Piperazine | Product | MP°C |
|---|---|---|---|
| 80 | N-benzyl | 4,5-Dihydro-4-[[4-(phenylmethyl)-1-piperazinyl]-acetyl]-7-[3-(trifluoromethyl)phenyl]pyrazolo[1,5-a]-pyrimidine-3-carbonitrile | 200-202 |
| 81 | 3-chlorobenzyl | 4-[[4-[(3-Chlorophenyl)methyl]-1-piperazinyl]acetyl]-4,5-dihydro-7-[3-(trifluoromethyl)phenyl]pyrazolo-[1,5-a]pyrimidine-3-carbonitrile | 185-187 |
| 82 | 2,6-dichlorobenzyl | 4-[[4-[(2,6-Dichlorophenyl)methyl]-1-piperazinyl]-acetyl]-4,5-dihydro-7-[3-(trifluoromethyl)phenyl]-pyrazolo[1,5-a]pyrimidine-3-carbonitrile | 179-181 |
| 83 | 3-methoxybenzyl | 4,5-Dihydro-4-[[4-[(3-methoxyphenyl)methyl]-1-piperazinyl]acetyl]-7-[3-(trifluoromethyl)phenyl]pyrazolo-[1,5-a]pyrimidine-3-carbonitrile | 160-162 |
| 84 | 4-chlorophenyl-benzyl | 4-[[4-[(4-Chlorophenyl)phenylmethyl]-1-piperazinyl]-acetyl]-4,5-dihydro-7-[3-(trifluoromethyl)phenyl]-pyrazolo[1,5-a]pyrimidine-3-carbonitrile | 177-179 |
| 85 | 3,4-dichlorophenyl | 4-[[4-(3,4-Dichlorophenyl)-1-piperazinyl]acetyl]-4,5-dihydro-7-[3-(trifluoromethyl)phenyl]pyrazolo[1,5-a]-pyrimidine-3-carbonitrile | 180-182 |

TABLE XIII (continued)

| Example | Piperazine | Product | MP°C |
|---|---|---|---|
| 86 | 1,3-benzodioxol-5-ylmethyl | 4-[[4-(1,3-Benzodioxol-5-ylmethyl)-1-piperazinyl]-acetyl]-4,5-dihydro-7-[3-(trifluoromethyl)phenyl]-pyrazolo[1,5-a]pyrimidine-3-carbonitrile | 173-175 |
| 87 | N-cyclohexyl | 4-[(4-Cyclohexyl-1-piperazinyl)acetyl]-4,5-dihydro-7-[3-(trifluoromethyl)phenyl]pyrazolo[1,5-a]pyrimidine-3-carbonitrile | 210-212 |
| 88 | N-phenyl | 4,5-Dihydro-4-[(4-phenyl-1-piperazinyl)acetyl]-7-[3-(trifluoromethyl)phenyl]pyrazolo[1,5-a]pyrimidine-3-carbonitrile | 179-180 |
| 89 | 3-chlorophenyl | 4-[[4-(3-Chlorophenyl)-1-piperazinyl]acetyl]-4,5-dihydro-7-[3-(trifluoromethyl)phenyl]pyrazolo[1,5-a]-pyrimidine-3-carbonitrile | 157-158 |
| 90 | N-benzyl | 4,5-Dihydro-4-[1-oxo-3-[4-(phenylmethyl)-1-piperazinyl]propyl]-7-[3-(trifluoromethyl)phenyl]pyrazolo-[1,5-a]pyrimidine-3-carbonitrile | 106-108 |
| 91 | 1-(2-pyrimidinyl) | 4,5-Dihydro-4-[[4-(2-pyrimidinyl)-1-piperazinyl]-acetyl]-7-[3-(trifluoromethyl)phenyl]pyrazolo[1,5-a]-pyrimidine-3-carbonitrile | 169-171 |

TABLE XIII (continued)

| Example | Piperazine | Product | MP°C |
|---|---|---|---|
| 92 | 1-(2-pyridinyl) | 4,5-Dihydro-4-[[4-(2-pyridinyl)-1-piperazinyl]acetyl]-7-[3-(trifluoromethyl)phenyl]pyrazolo[1,5-a]pyrimidine-3-carbonitrile | 168-170 |
| 93 | 3-(trifluoromethyl)phenyl | 4,5-Dihydro-7-[3-(trifluoromethyl)phenyl]-4-[[4-[3-(trifluoromethyl)phenyl]-1-piperazinyl]acetyl]pyrazolo[1,5-a]pyrimidine-3-carbonitrile | 145-146 |
| 94 | 4-fluorophenyl | 4-[[4-(4-Fluorophenyl)-1-piperazinyl]acetyl]-4,5-dihydro-7-[3-(trifluoromethyl)phenyl]pyrazolo[1,5-a]-pyrimidine-3-carbonitrile | 183-184 |
| 95 | 2-methoxyphenyl | 4,5-Dihydro-4-[[4-(2-methoxyphenyl)-1-piperazinyl]-acetyl]-7-[3-(trifluoromethyl)phenyl]pyrazolo[1,5-a]-pyrimidine-3-carbonitrile | 164-165 |
| 96 | 3-dimethylaminopropyl | 4-[[4-[3-(Dimethylamino)propyl]-1-piperazinyl]acetyl]-4,5-dihydro-7-[3-(trifluoromethyl)phenyl]pyrazolo-[1,5-a]pyrimidine-3-carbonitrile | 146-148 |
| 97 | 2-propynyl | 4,5-Dihydro-4-[[4-(2-propynyl)-1-piperazinyl]acetyl]-7-[3-(trifluoromethyl)phenyl]pyrazolo[1,5-a]pyrimidine-3-carbonitrile | 143-145 |

TABLE XIII (continued)

| Example | Piperazine | Product | MP°C |
|---|---|---|---|
| 98 | 4-chlorobenzyl | 4-[3-[4-[(Chlorophenyl)methyl]-1-piperazinyl]-1-oxopropyl]-4,5-dihydro-7-[3-(trifluoromethyl)phenyl]-pyrazolo[1,5-a]pyrimidine-3-carbonitrile | 99-101 |
| 99 | 4-morpholinyl-2-oxoethyl | 4,5-Dihydro-4-[[4-[2-(4-morpholinyl)-2-oxoethyl]-1-piperazinyl]acetyl]-7-[3-(trifluoromethyl)phenyl]-pyrazolo[1,5-a]pyrimidine-3-carbonitrile | 179-181 |
| 100 | N-(1-methylethyl)-2-oxoethyl | 4-[2-[3-Cyano-7-[3-(trifluoromethyl)phenyl]pyrazolo-[1,5-a]pyrimidin-4(5H)-yl]-2-oxoethyl-N-(1-methylethyl)-1-piperazineacetamide | 117-119 |
| 101 | (1-pyrrolidinyl)-2-oxoethyl | 4,5-Dihydro-4-[[4-[2-oxo-2-(1-pyrrolidinyl)ethyl]-1-piperazinyl]acetyl]-7-[3-(trifluoromethyl)phenyl]-pyrazolo[1,5-a]pyrimidine-3-carbonitrile | 159-162 |
| 102 | 4-chlorobenzyl | 4-[[4-[(4-Chlorophenyl)methyl]-1-piperazinyl]acetyl]-7-[3-(trifluoromethyl)phenyl]pyrazolo[1,5-a]pyrimidine-3-carbonitrile | 205-207 |
| 103 | bis(4-fluorophenyl)methyl | 4-[[4-[Bis(4-fluorophenyl)methyl]-1-piperazinyl]-acetyl]-4,5-dihydro-7-[3-(trifluoromethyl)phenyl]-pyrazolo[1,5-a]pyrimidine-3-carbonitrile | 177-179 |

TABLE XIII (continued)

| Example | Piperazine | Product | MP°C |
|---|---|---|---|
| 104 | 2-cyclohexylethyl | 4-[[4-(2-Cyclohexylethyl)-1-piperazinyl]acetyl]-4,5-dihydro-7-[3-(trifluoromethyl)phenyl]pyrazolo-[1,5-a]pyrimidine-3-carbonitrile | 208-210 |
| 105 | carboxylic acid, ethyl ester | 4-[2-[3-Cyano-7-[3-(trifluoromethyl)phenyl]pyrazolo-[1,5-a]pyrimidin-4(5H)-yl]-2-oxoethyl]-1-piperazine-carboxylic acid, ethyl ester | 75-78 |
| 106 | 3-chlorobenzyl | 7-(3-Chlorophenyl)-4-[[4-[(3-chlorophenyl)methyl]-1-piperazinyl]acetyl]-4,5-dihydropyrazolo[1,5-a]pyrimi-dine-3-carbonitrile | 160-162 |
| 107 | benzyl | 7-(3-Chlorophenyl)-4,5-dihydro-4-[[4-(phenylmethyl)-1-piperazinyl]acetyl]pyrazolo[1,5-a]pyrimidine-3-carbonitrile | 165-166 |
| 108 | 4-chlorophenyl-benzyl | 7-(3-Chlorophenyl)-4-[[4-[(4-chlorophenyl)phenyl-methyl]-1-piperazinyl]acetyl]-4,5-dihydropyrazolo-[1,5-a]pyrimidine-3-carbonitrile | 159-161 |
| 109 | N-benzyl | 4,5,6,7-Tetrahydro-4-[[4-(phenylmethyl)-1-piperazinyl]acetyl]-7-[3-(trifluoromethyl)phenyl]pyrazolo-[1,5-a]pyrimidine-3-carbonitrile | 164-166 |

TABLE XIII (continued)

| Example | Piperazine | Product | MP°C |
|---|---|---|---|
| 110 | 2-propenyl | 4,5-Dihydro-4-[[4-(2-propenyl)-1-piperazinyl]acetyl]-7-[3-(trifluoromethyl)phenyl]pyrazolo[1,5-a]pyrimidine-3-carbonitrile | 167-169 |
| 111 | N-benzyl | 7-(3-Fluorophenyl)-4,5-dihydro-4-[[4-(phenylmethyl)-1-piperazinyl]acetyl]pyrazolo[1,5-a]pyrimidine-3-carbonitrile | 138-140 |
| 112 | 4-chlorobenzyl | 4-[[4-[(4-Chlorophenyl)methyl]-1-piperazinyl]acetyl]-7-(3-fluorophenyl)-4,5-dihydropyrazolo[1,5-a]pyrimidine-3-carbonitrile | 160-165 |
| 113 | N-benzyl | 4,5-Dihydro-7-(3-nitrophenyl)-4-[[4-(phenylmethyl)-1-piperazinyl]acetyl]pyrazolo[1,5-a]pyrimidine-3-carbonitrile | 155-157 |
| 114 | 1-methylethyl | 4,5-Dihydro-4-[[4-(1-methylethyl)-1-piperazinyl]-acetyl]-7-[3-(trifluoromethyl)phenyl]pyrazolo[1,5-a]-pyrimidine-3-carbonitrile | 187-189 |
| 115 | N-cyclobutyl | 4-[(4-Cyclobutyl-1-piperazinyl)acetyl]-4,5-dihydro-7-[3-(trifluoromethyl)phenyl]pyrazolo[1,5-a]pyrimidine-3-carbonitrile | 188-190 |

TABLE XIII (continued)

| Example | Piperazine | Product | MP°C |
|---|---|---|---|
| 116 | 3-chlorobenzyl | 4-[[4-[(3-Chlorophenyl)methyl]-1-piperazinyl]acetyl]-4,5,6,7-tetrahydro-7-[3-(trifluoromethyl)phenyl]-pyrazolo[1,5-a]pyrimidine-3-carbonitrile | 160-162 |
| 117 | 2,6-dichloro-benzyl | 4-[[4-[(2,6-Dichlorophenyl)methyl]-1-piperazinyl]-acetyl]-4,5,6,7-tetrahydro-7-[3-(trifluoromethyl)-phenyl]pyrazolo[1,5-a]pyrimidine-3-carbonitrile | 140-142 |
| 118 | 3-phenoxypropyl | 4,5-Dihydro-4-[[4-(3-phenoxypropyl)-1-piperazinyl]-acetyl]-7-[3-(trifluoromethyl)phenyl]pyrazolo[1,5-a]-pyrimidine-3-carbonitrile | 139-141 |
| 119 | 4-chlorobenzyl | 4-[[4-[(4-Chlorophenyl)methyl]-1-piperazinyl]acetyl]-4,5,6,7-tetrahydro-7-[3-(trifluoromethyl)phenyl]-pyrazolo[1,5-a]pyrimidine-3-carbonitrile | 165-167 |
| 120 | 3-phenoxypropyl | 4,5,6,7-Tetrahydro-4-[[4-(3-phenoxypropyl)-1-pipera-zinyl]acetyl]-7-[3-(trifluoromethyl)phenyl]pyrazolo-[1,5-a]pyrimidine-3-carbonitrile | 113-115 |
| 121 | N-benzyl | 7-(4-Chlorophenyl)-4,5-dihydro-4-[[4-(phenylmethyl)-1-piperazinyl]acetyl]pyrazolo[1,5-a]pyrimidine-3-carbonitrile | 182-183 |

TABLE XIII (continued)

| Example | Piperazine | Product | MP°C |
|---|---|---|---|
| 122 | 2-(trifluoromethyl)benzyl | 7-(4-Chlorophenyl)-4,5-dihydro-4-[[4-[[2-(trifluoromethyl)phenyl]methyl]-1-piperazinyl]acetyl]pyrazolo[1,5-a]pyrimidine-3-carbonitrile | 144-145 |
| 123 | 2-methoxyphenyl | 7-(4-Chlorophenyl)-4,5-dihydro-4-[[4-(2-methoxyphenyl)-1-piperazinyl]acetyl]pyrazolo[1,5-a]pyrimidine-3-carbonitrile | 179-180 |
| 124 | bis(4-fluorophenyl)methyl | 4-[[4-[Bis(4-fluorophenyl)methyl]-1-piperazinyl]acetyl]-7-(4-chlorophenyl)-4,5-dihydropyrazolo[1,5-a]pyrimidine-3-carbonitrile | 108-110 |
| 125 | N-benzyl | 7-(2,5-Dichlorophenyl)-4,5-dihydro-4-[[4-(phenylmethyl)-1-piperazinyl]acetyl]pyrazolo[1,5-a]pyrimidine-3-carbonitrile | 173-174 |
| 126 | 3-phenoxypropyl | 7-(4-Chlorophenyl)-4,5-dihydro-4-[[4-(3-phenoxypropyl)-1-piperazinyl]acetyl]pyrazolo[1,5-a]pyrimidine-3-carbonitrile | 175-177 |
| 127 | 4-chlorobenzyl | 7-(4-Chlorophenyl)-4-[[4-[(4-chlorophenyl)methyl]-1-piperazinyl]acetyl]-4,5-dihydropyrazolo[1,5-a]pyrimidine-3-carbonitrile | 178-179 |

TABLE XIII (continued)

| Example | Piperazine | Product | MP°C |
|---|---|---|---|
| 128 | 1,3-benzodioxol-5-yl | 4-[[4-(1,3-Benzodioxol-5-yl)-1-piperazinyl]acetyl]-7-(2,5-dichlorophenyl)-4,5-dihydropyrazolo[1,5-a]-pyrimidine-3-carbonitrile | 173-174 |
| 129 | 2,5-dimethylphenyl | 7-(2,5-Dichlorophenyl)-4-[[4-(2,5-dimethylphenyl)-1-piperazinyl]acetyl]-4,5-dihydropyrazolo[1,5-a]pyrimidine-3-carbonitrile | 166-167 |
| 130 | N-methyl | 7-(2,5-Dichlorophenyl))-4,5-dihydro-4-[(4-methyl-1-piperazinyl)acetyl]pyrazolo[1,5-a]pyrimidine-3-carbonitrile | 182-185 |
| 131 | N-benzoyl | 4-[(4-Benzoyl-1-piperazinyl)acetyl]-4,5-dihydro-7-[3-(trifluoromethyl)phenyl]pyrazolo[1,5-a]pyrimidine-3-carbonitrile | 183-185 |
| 132 | 2-methoxyphenyl | 7-(2,5-Dichlorophenyl)-4,5-dihydro-4-[[4-(2-methoxyphenyl)-1-piperazinyl]acetyl]pyrazolo[1,5-a]pyrimidine-3-carbonitrile | 121-122 |
| 134 | 2-furanylcarbonyl | 7-(2,5-Dichlorophenyl)-4,5-dihydro-4-[[4-(2-furanylcarbonyl)-1-piperazinyl]acetyl]pyrazolo[1,5-a]pyrimidine-3-carbonitrile | 142-144 |

TABLE XIII (continued)

| Example | Piperazine | Product | MP°C |
|---|---|---|---|
| 135 | N-benzyl | 7-(2,5-Dichlorophenyl)-4,5-dihydro-4-[1-oxo-3-[4-(phenylmethyl)-1-piperazinyl]propyl]pyrazolo[1,5-a]-pyrimidine-3-carbonitrile | 94-95 |
| 136 | N-benzyl | 4,5-Dihydro-5-methyl-4-[[4-(phenylmethyl)-1-piperazinyl]acetyl]-7-[3-(trifluoromethyl)phenyl]pyrazolo-[1,5-a]pyrimidine-3-carbonitrile | 129-131 |
| 137 | N-hydroxyethyl | 4,5-Dihydro-4-[[4-(2-hydroxyethyl)-1-piperazinyl]-acetyl]-6-methyl-7-[3-(trifluoromethyl)phenyl]pyrazolo[1,5-a]pyrimidine-3-carbonitrile | 178-179 |
| 138 | N-methyl | 4,5-Dihydro-4-[(4-methyl-1-piperazinyl)acetyl]-7-[3-(trifluoromethyl)phenyl]pyrazolo[1,5-a]pyrimidine-3-carboxamide | 164-166 |
| 139 | N-benzyl | 4,5-Dihydro-4-[[4-(phenylmethyl)-1-piperazinyl]-acetyl]-7-[3-(trifluoromethyl)phenyl]pyrazolo[1,5-a]-pyrimidine-3-carboxamide | 152-154 |
| 140 | N-benzyl | 3-Bromo-4,5-dihydro-4-[[4-(phenylmethyl)-1-piperazinyl]acetyl]-7-[3-(trifluoromethyl)phenyl]pyrazolo-[1,5-a]pyrimidine | 127-129 |

Example 141

4,5-Dihydro-4-[(4-methyl-1-piperazinyl)acetyl]-7-[3-(trifluoromethyl)phenyl]pyrazolo[1,5-a]pyrimidine-3-carbonitrile, dihydrochloride

4,5-Dihydro-4-[(4-methyl-1-piperazinyl)acetyl]-7-[3-(trifluoromethyl)phenyl]pyrazolo[1,5-a]pyrimidine-3-carbonitrile was dissolved in hot ethyl acetate and treated with dilute alcoholic hydrochloric acid. Cooling produced a solid which was collected, washed with ether and dried. This solid was heated to boiling in 60 ml of acetonitrile, filtered and the filtrate diluted with 60 ml of ether and refrigerated. The solid was collected, washed with ether and dried, giving 600 mg of the desired product, mp 198°C (eff.).

Following the general procedure of Example 141 using various organic and inorganic solvents, the products of Examples 142 through 159 found in Table XIV were obtained.

TABLE XIV

| Example | Base Example | Product | MP°C |
|---|---|---|---|
| 142 | 17 | 4,5-Dihydro-4-[[4-(phenylmethyl)-1-piperazinyl]acetyl]-7-[3-(trifluoromethyl)phenyl]pyrazolo[1,5-a]pyrimidine-3-carbonitrile, dihydrochloride | 224-226 |
| 143 | 20 | 4,5-Dihydro-4-[[4-[(3-methoxyphenyl)methyl]-1-piperazinyl]-acetyl]-7-[3-(trifluoromethyl)phenyl]pyrazolo[1,5-a]pyrimidine-3-carbonitrile, dihydrochloride | 226-228 |
| 144 | 19 | 4-[[4-[(2,6-Dichlorophenyl)methyl]-1-piperazinyl]acetyl]-4,5-dihydro-7-[3-(trifluoromethyl)phenyl]pyrazolo[1,5-a]pyrimidine-3-carbonitrile, dihydrochloride | 220-222 |
| 145 | 18 | 4-[[4-[(3-Chlorophenyl)methyl]-1-piperazinyl]acetyl]-4,5-dihydro-7-[3-(trifluoromethyl)phenyl]pyrazolo[1,5-a]pyrimidine-3-carbonitrile, dihydrochloride | 220-222 |
| 146 | 22 | 4-[[4-(3,4-Dichlorophenyl)-1-piperazinyl]acetyl]-4,5-dihydro-7-[3-(trifluoromethyl)phenyl]pyrazolo[1,5-a]pyrimidine-3-carbonitrile, hydrochloride | 260-262 |
| 147 | 24 | 4-[(4-Cyclohexyl-1-piperazinyl)acetyl]-4,5-dihydro-7-[3-(trifluoromethyl)phenyl]pyrazolo[1,5-a]pyrimidine-3-carbonitrile, dihydrochloride | 241-243 |

TABLE XIV (continued)

| Example | Base Example | Product | MP°C |
|---|---|---|---|
| 148 | 28 | 4,5-Dihydro-4-[[4-(2-pyrimidinyl)-1-piperazinyl]acetyl]-7-[3-(trifluoromethyl)phenyl]pyrazolo[1,5-a]pyrimidine-3-carbonitrile, hydrochloride | 235-237 |
| 149 | 35 | 4-[3-[(4-Chlorophenyl)methyl]-1-piperazinyl]-1-oxopropyl]-4,5-dihydro-7-[3-(trifluoromethyl)phenyl]pyrazolo[1,5-a]pyrimidine-3-carbonitrile, dihydrochloride | 254-256 |
| 150 | 41 | 4-[[4-(2-(Cyclohexylethyl)-1-piperazinyl]acetyl]-4,5-dihydro-7-[3-(trifluoromethyl)phenyl]pyrazolo[1,5-a]pyrimidine-3-carbonitrile, dihydrochloride | 235-237 |
| 151 | 39 | 4-[[4-[(4-Chlorophenyl)methyl]-1-piperazinyl]acetyl]-4,5-dihydro-7-[3-(trifluoromethyl)phenyl]pyrazolo[1,5-a]pyrimidine-3-carbonitrile, dihydrochloride | 219-221 |
| 152 | 40 | 4-[[4-[Bis(4-Fluorophenyl)methyl]-1-piperazinyl]acetyl]-4,5-dihydro-7-[3-(trifluoromethyl)phenyl]pyrazolo[1,5-a]pyrimidine-3-carbonitrile, dihydrochloride | 255-258 |
| 153 | 46 | 4,5,6,7-Tetrahydro-4-[[4-(phenylmethyl)-1-piperazinyl]acetyl]-7-[3-(trifluoromethyl)phenyl]pyrazolo[1,5-a]pyrimidine-3-carbonitrile, dihydrochloride | 195-197 |

TABLE XIV (continued)

| Example | Base Example | Product | MP°C |
|---|---|---|---|
| 154 | 51 | 4,5-Dihydro-4-[[4-(1-methylethyl)-1-piperazinyl]acetyl]-7-[3-(trifluoromethyl)phenyl]pyrazolo[1,5-a]pyrimidine-3-carbonitrile, dihydrochloride | 205-208 |
| 155 | 52 | 4-[(4-Cyclobutyl-1-piperazinyl)acetyl]-4,5-dihydro-7-[3-(trifluoromethyl)phenyl]pyrazolo[1,5-a]pyrimidine-3-carbonitrile, dihydrochloride | 227-229 |
| 156 | 67 | 7-(2,5-Dichlorophenyl)-4,5-dihydro-4-[(4-methyl-1-piperazinyl)-acetyl]pyrazolo[1,5-a]pyrimidine-3-carbonitrile, dihydrochloride | 248-250 |
| 157 | 68 | 4-[(4-Benzoyl-1-piperazinyl)acetyl]-4,5-dihydro-7-[3-(trifluoromethyl)phenyl]pyrazolo[1,5-a]pyrimidine-3-carbonitrile, hydrochloride | 243-245 |
| 158 | 69 | 4,5-Dihydro-4-[[4-(phenylmethyl)-1-piperazinyl]acetyl]-7-[3-(trifluoromethyl)phenyl]pyrazolo[1,5-a]pyrimidine-3-carbonitrile, hydrochloride | 160-162 |
| 159 | 72 | 4,5-Dihydro-5-methyl-4-[[4-(phenylmethyl)-1-piperazinyl]acetyl]-7-[3-(trifluoromethyl)phenyl]pyrazolo[1,5-a]pyrimidine-3-carbonitrile, dihydrochloride | 190-192 |

Example 160

4-[[4-(2-Furanylcarbonyl-1-piperazinyl]acetyl]-4,5-dihydro-7-[(3-trifluoromethyl)phenyl]pyrazolo[1,5-a]-pyrimidine-3-carbonitrile, hydrochloride

A 2.2 g portion of 2-furanylcarbonyl piperazine, 4 g of 4-(chloroacetyl)-4,5-dihydro-7-[3-(trifluoromethyl)-phenyl]pyrazolo[1,5-a]pyrimidine-3-carbonitrile and 1.6 g of sodium carbonate in 60 ml of toluene was reacted as described in Example 79, giving 3.3 g of the base as a hygroscopic solid. This solid was dissolved in ethanol, 10 ml of 3N ethanolic hydrogen chloride was added and the mixture was stirred. The solid was collected, washed with ethanol, ether, and then dried to give 1.2 g of the desired product, mp 240-243°C.

Example 161

4-[[4-[2-(Dimethylamino)ethyl]-1-piperazinyl]acetyl]4,5-dihydro-7-[3-(trifluoromethyl)phenyl]pyrazolo[1,5-a]-pyrimidine-3-carbonitrile, dihydrochloride

A mixture of 1.7 g of 2-dimethylaminoethyl piperazine, 3.8 g of 4-(chloroacetyl)-4,5-dihydro-7-[3-trifluoromethyl)phenyl]pyrazolo[1,5-a ]pyrimidine-3-carbonitrile, 1.2 g of sodium carbonate and 65 ml of toluene was reacted as described in Example 79, giving 3.2 g of solid. The solid was reacted with ethanolic hydrogen chloride, giving 170 mg of the desired product, mp 229-231°C.

Example 162

4,5-dihydro-4-(1-piperazinylacetyl)-7-[3-(trifluoromethyl)phenyl]pyrazolo[1,5-a]pyrimidine-3-carbonitrile

A mixture of 1.56 g of 4,5-dihydro-4-(iodoacetyl)-7-[3-(trifluoromethyl)phenyl]pyrazolo[1,5-a ]pyrimidine-3-carbonitrile and 1 g of piperazine in 50 ml of dioxane, under argon, was heated and stirred at reflux for 18 hours. The reaction was cooled, the solid collected, removed by filtration and the filtrate evaporated. The residue was crystallized by trituration with ether giving 1.3 g of crystals. These crystals were heated to solution in 10 ml of ethyl acetate, diluted with 10 ml of hexane and filtered. The filtrated was cooled giving a solid which was collected, washed with hexane and dried, giving 400 mg of the desired product, mp 151-154°C.

Example 163

4-[2-[3-Cyano-7-[3-(trifluoromethyl)phenyl]pyrazolo-[1,5-a]pyrimidin-4(5H)-yl]-2-oxoethyl]-N-phenyl-1-piperazinecarboxamide

A mixture of 3.5 g of 4,5-dihydro-4-(1-piperazinylacetyl)-7-[3-(trifluoromethyl)phenyl]pyrazolo[1,5-a]-pyrimidine-3-carbonitrile and 1 g of phenyl isocyanate in 50 ml of acetone was stirred and refluxed for 18 hours, then cooled and filtered. The filtrate was evaporated on a steam bath and the residue repeatedly triturated with ether. The solid was dissolved with warming in 15 ml of chloroform, then filtered and 15 ml of hexane was added. The crystals were collected, washed with hexane and dried, giving 2.1 g of the desired product, mp 184-187°C.

Example 164

N-(5-Chloro-2-methoxyphenyl)-4-[2-[3-cyanao-7-[3-(trifluoromethyl)phenyl]pyrazolo[1,5-a]pyrimidin-4(5H)-yl]-2-oxoethyl]-1-piperazineacetamide

A mixture of 3.5 g of 4,5-dihydro-4-(1-piperazinylacetyl)-7-[3-(trifluoromethyl)phenyl]pyrazolo[1,5-A]-pyrimidine-3-carbonitrile, 2 g of 2-chloro-N-(5-chloro-2-methoxy phenyl)acetamide and 900 mg of sodium carbonate in 50 ml of toluene was refluxed with stirring for 18 hours, then cooled and made basic with 15 ml of 1N sodium hydroxide. The aqueous phase was separated and extracted twice with chloroform. The organic phases were combined, washed with water, dried, filtered and concentrated to dryness. The residue was triturated with ether, the solid collected, washed with ether and dried. This solid was heated to solution in 40 ml of acetonitrile, filtered and cooled. The solid was collected, washed with acetonitrile and ether and dried, giving 2.3 g of the desired product, mp 166-168°C.

Example 165

4-[2-[3-Cyano-7-[3-(trifluoromethyl)phenyl]pyrazolo-[1,5-a]pyrimidin-4-(5H)-yl]-2-oxoethyl]-N-[3-(trifluoromethyl)phenyl]-1-piperazinecarboxamide

A mixture of 2.6 g of 3-(trifluoromethyl)phenyl isocyanate, 5.7 g of 4,5-dihydro-4-(1-piperazinylacetyl)-7-[3-(trifluoromethyl)phenyl]pyrazolo[1,5-a]pyrimidine-3-carbonitrile and 80 ml of acetone was heated at reflux for 18 hours, then concentrated to dryness. The residue was triturated with 20 ml of ether and the solid collected. The solid was dissolved with heating in 10 ml of ethyl acetate, diluted with 10 ml of hexane and then cooled. The solid was collected, washed with hexane and dried, giving 780 mg of the desired product, mp 147-149°C.

Example 166

4,5-Dihydro-4-[[4-(1-oxotetradecyl)-1-piperazinyl]acetyl]-7-[3-(trifluoromethyl)phenyl]pyrazolo[1,5-a]-pyrimidine-3-carbonitrile

To a mixture of 3 g of 4,5-dihydro-4-(1-piperazinylacetyl)-7-[3-(trifluoromethyl)phenyl]pyrazolo[1,5-a]-pyrimidine-3-carbonitrile and 0.8 g of 1,8-bis(dimethylamino)naphthalene, N,N,N',N'-tetramethyl-1,8-naphthalenediamine in 30 ml of dry tetrahydrofuran, under nitrogen, was added a solution of 1.75 g of myristoyl chloride in 10 ml of dry tetrahydrofuran. The mixture was stirred at reflux overnight, then at room temperature for 2 days. The solid was collected, washed with ether, then water and dried, giving 3 g of the desired product, mp 260-263°C.

Example 167

4,5-Dihydro-4-[[4-(tricyclo[3•3•1•1]dec-1-ylcarbonyl)-1-piperazinyl]acetyl]-7-[3-(trifluoromethyl)phenyl]-pyrazolo[1,5-a]pyrimidine-3-carbonitrile

The procedure of Example 166 was followed using 1.5 of adamantanecarbonyl chloride, giving 2.3 g of the desired product, mp 194-200°C.

Example 168

4-[2-[3-Cyano-7-[3-(trifluoromethyl)phenyl]pyrazolo[1,5-a]pyrimidin-4(5H)-yl]-2-oxoethyl]-N-(3,4-dichlorophenyl-1-piperazinecarboxamide

The procedure of Example 165 was followed, using 12.3 g of 3,4-dichlorophenyl isocyanate, givine 1.4 g of the desired product, mp 204-206°C.

Example 169

N-(3-Chlorophenyl)-4-[2-[3-cyano-7-[3-(trifluoromethyl)phenyl]pyrazolo[1,5-a]pyrimidin-4(5H)-yl]-2-oxoethyl]-1-piperazinecarboxamide

The procedure of Example 165 was followed, using 1.8 of 3-chlorophenyl isocyanate, giving 900 mg of the desired product, mp 182-185°C.

Example 170

N-[4-Chloro-3-(trifluoromethyl)phenyl]-4-[2-[3-cyano-7-[3-(trifluoromethyl)phenyl]pyrazolo[1,5-a]pyrimidin-4-(5H)-yl]-2-oxoethyl]-1-piperazinecarboxamide

The procedure of Example 165 was followed, using 2.7 g of 4-chloro-3-(trifluoromethyl)phenyl isocyanate, giving 1.4 g of the desired compound, mp 214-216°C.

Example 171

4-[2-[3-cyano-7-[3-(trifluoromethyl)phenyl]pyrazolo[1.5-a]pyrimidin-4(5H)-yl]-2,4-difluorophenyl)-1-piperazinecarboxamide

The procedure of Example 165 was followed, using 1.4 g of 2,4-difluorophenyl isocyanate, giving 2.6 g of the desired product, mp 142-145°C.

Example 172

4-[2-[3-cyano-7-[3-(trifluoromethyl)phenyl]pyrazolo[1,5-a]pyrimidin-4(5H)-yl]-2-oxoethyl]-N-(3-methylphenyl)-1-piperazinecarboxamide

The procedure of Example 165 was followed, using 1.2 g of 3-methylphenyl isocyanate, giving 2.5 g of the desired product, mp 138-140°C.

Example 173

4,5-Dihydro-4-[[4-[4-(2-oxo-1-pyrrolidinyl)-2-butynyl]-1-]piperazinyl]acetyl]-7-[3-(trifluoromethyl)phenyl]-pyrazolo[1,5-a]pyrimidine-3-carbonitrile

A mixture of 3.1 g of 4,5-dihydro-4-(1-piperazinylacetyl)-7-[3-(trifluoromethyl)phenyl]pyrazolo[1,5-a]-pyrimidine-3-carbonitrile, 255 mg of paraformaldehyde, 815 mg of 1-(2-propargyl)pyrrolidine-2-one and 50 mg of cupric chloride in 30 ml of dioxane was refluxed under nitrogen, with stirring for 2 hours then stirred overnight at room temperature. The mixture was concentrated and the residue acidified with 3 ml of 6N hydrochloric acid. A 10 ml portion of water was added and the mixture was shaken with ether until the oil was dissolved. The aqueous phase was separated, rewashed with ether, then made basic with 6 ml of 5N sodium hydroxide and extracted twice with chloroform. The chloroform extracts were combined, washed with saturated salt solution, dried, filtered and concentrated to dryness. The residue was triturated with ether and the solid collected. This solid was dissolved with heating in 25 ml of ethyl acetate, filtered and cooled. The solid was collected, washed with hexane and dried, giving 2 g of the desired product mp 124-126°C.

Example 174

4,5-Dihydro-4-[[4-(phenylmethyl)-1-piperazinyl]acetyl]-7-[3-(trifluoromethyl)phenyl]pyrazolo[1,5-a]pyrimidine-3-carboxylic acid, ethyl ester, dihydrochloride

A mixture of 2.065 g of 4-(chloroacetyl)-4,5-dihydro-7-[3-(trifluoromethyl)phenyl]pyrazolo[1,5-a]-pyrimidine-3-carboxylic acid, ethyl ester, 535 mg of 1,8-bis(dimethylamino)naphthalene, N,N,N' ,N'-tetramethyl-1,8-naphthalenediamine 2.88 g of N-benzylpiperazine and 100 ml of dry ether was stirred at room temperature overnight and then filtered. The filtrate was evaporated, the residue dissolved in 300 ml of ether and 1 ml of concentrated hydrochloric acid in 50 ml of water was added. The solid was collected, giving 845 mg of the desired product, mp 228-230°C.

Example 175

4,5-Dihydro-6-methyl-4-[[4-(phenylmethyl)-1-piperazinyl]acetyl]-7-[3-(trifluoromethyl)phenyl]pyrazolo[1,5-a]-pyrimidine-3-carbonitrile, dihydrochloride

A mixture of 3.4 g of 4-(chloroacetyl)-4,5-dihydro-6-methyl-7-[3-(trifluoromethyl)phenyl]pyrazolo[1,5-a]-pyrimidine-3-carbonitrile, 1.1 g of sodium carbonate, 1.7 g of N-benzylpiperazine and 115 ml of toluene was reacted as described in Example 79, giving the base form of the product which was converted to the dihydrochloride salt by the procedure of Example 161, giving 2.4 g, mp 204-206°C.

4,5-Dihydro-6-methyl-4-[[4-(2-pyridinyl)-1-piperazinyl]acetyl]-7-[3-(trifluoromethyl)phenyl]pyrazolo[1,5-a]-pyrimidine-3-carbonitrile

A mixture of 2 g of 1-(2-pyridinyl)piperazine, 2 g of 4-(chloroacetyl)-4,5-dihydro-6-methyl-7-[3-(trifluoromethyl)phenyl]pyrazolo[1,5-a]pyrimidine-3-carbonitrile and 60 ml of acetone was refluxed on a steam bath for 8 hours and then evaporated. The residue was stirred in water and the solid collected. The solid was dissolved in dilute hydrochloric acid, filtered and the filtrated basified with dilute ammonium hydroxide. The solid was collected, washed with water and dried in vacuo , giving 2.67 g of the desired product, mp 132-134°C.

Example 177

7-(3-Chlorophenyl)-4,5-dihydro-6-methyl-4-[[4-(phenylmethyl)piperazinyl]acetyl]pyrazolo[1,5-a]pyrimidine-3-carbonitrile, dihydrochloride

A mixture of 3.1 g of 4-(Chloroacetyl)-7-(3-chlorophenyl)-4,5-dihydro-6-methyl-pyrazolo[1,5-a]pyrimidine-3-carbonitrile, 1.1 g of sodium carbonate, 1.7 g of N-benzylpiperazine and 115 ml of toluene was reacted as described in Example 16, giving the base form of the product which was converted to the dihydrochloride salt by the procedure of Example 161, giving 1.3 g, mp 201-203°C.

Example 178

7-(4-Chlorophenyl)-4,5-dihydro-6-methyl-4-[[4-(phenylmethyl)piperazinyl]acetyl]pyrazolo[1,5-a]pyrimidine-3-carbonitrile, dihydrochloride

A mixture of 3.1 g of 4-(chloroacetyl)-7-(4-chlorophenyl)-4,5-dihydro-6-methylpyrazolo[1,5-a]pyrimidine-3-carbonitrile, 1.3 g of sodium carbonate, 2 g of N-benzylpiperazine and 130 ml of toluene was reacted as described in Example 79, giving the base form of the product which was converted to the dihydrochloride salt, by the procedure of Example 161, giving 1.5 g, mp 238-240°C.

Example 179

7-(4-Chlorophenyl)-4,5-dihydro-4-(1-piperazinylacetyl)pyrazolo[1,5-a]pyrimidine-3-carbonitrile, hydroiodide

A mixture of 5 g of 7-(4-chlorophenyl)-4-(iodoacetyl)-4,5-dihydropyrazolo[1,5-a]pyrimidine-3-carbonitrile, and 3.15 g of piperazine in acetone was heated at reflux for 6 hours, then at room temperature overnight and evaporated. The residue was taken up in water, evaporated and recrystallized from ethanol, giving 3.27 g of the desired product, mp 231-232°C.

Example 180

4,5-Dihydro-6-methyl-7-[3-(trifluoromethyl)-4-[[4[[3-(trifluoromethyl)phenyl]methyl]piperazinyl]acetyl]-pyrazolo[1,5-a]pyrimidine-3-carbonitrile, hydrochloride

A mixture of 1.83 g of 4-(chloroacetyl)-7-[3(trifluoromethyl)phenyl]-4,5-dihydro-6-methylpyrazolo[1,5-a]-pyrimidine-3-carbonitrile, 1.5 g of 3-(trifluoromethyl)benzylpiperazine, 2 g of potassium carbonate and 60 ml of toluene were reacted as described in Example 79, giving the base form of the product, which was converted to the hydrochloride salt by the procedure of Example 161, giving 1.78 g, mp 195-198°.

Example 181

4-[[4-(2,6-Dichlorophenyl)methyl]-1-piperazinyl]acetyl]-4,5-dihydro-6-methyl-7-[3-(trifluoromethyl)phenyl]-pyrazolo[1,5-a]pyrimidine-3-carbonitrile, hydrochloride

A mixture of 1 g of 4-(chloroacetyl)-7-[3-(trifluoromethyl)phenyl]-4,5-dihydro-6-methylpyrazolo[1,5-a]-pyrimidine-3-carbonitrile, 1 g of 2,6-dichlorobenzylpiperazine, 600 mg of potassium carbonate and 100 ml of toluene was reacted as described in Example 79, giving the base form of the product, which was converted to the hydrochloride salt by the procedure of Example 161, giving 1.66 g, mp 252-254°C.

Example 182

4-(Chloroacetyl)4,5-dihydro-7-phenylpyrazolo[1,5-a]pyrimidine-3-carbonitrile

The above compound is prepared from 7-phenyl-pyrazolo[1,5-a]pyrimidine-3-carbonitrile by the procedure of Example 64.

Example 183

4,5-Dihydro-4[[4-(phenylmethyl)-1-piperazinyl]acetyl]-7-phenylpyrazolo[1,5-a]pyrimidine-3-carbonitrile

The above compound is obtained from N-benzylpiperazine and 4-(chloroacetyl)-4,5-dihydro-7-phenylpyrazolo[1,5-a]pyrimidine-3-carbonitrile by the procedure of Example 79.

Example 184

4-(Chloroacetyl)-4,5-dihydro-7-(3-methoxyphenyl)pyrazolo[1,5-a]pyrimidine-3-carbonitrile

The above compound is prepared from 7-(3-methoxyphenyl)pyrazolo[1,5-a]pyrimidine-3-carbonitrile by the procedure of Example 64.

Example 185

4,5-Dihydro-4[[4-(phenylmethyl)-1-piperazinyl]acetyl]-7-(3-methoxyphenyl)pyrazolo[1,5-a]pyrimidine-3-carbonitrile

The above compound is obtained from N-benzylpiperazine and 4-(chloroacetyl-4,5-dihydro-7-(3-methoxyphenyl)pyrazolo[1,5-a]pyrimidine-3-carbonitrile by the procedure of Example 79.

Example 186

3-Chloro-4-(chloroacetyl)-4,5-dihydro-7-[3-(trifluoromethyl)phenyl]pyrazolo[1,5-a]pyrimidine

A 13.3 g portion of 3-chloro-7[3-(trifluoromethyl)phenyl)pyrazolo[1,5-a]pyrimidine and 7 g of sodium cyanoborohydride in 150 ml of acetic acid, were reacted as described in Example 64 giving 6.7 g of 3-chloro-4,5-dihydro-7-[3-(trifluoromethyl)phenyl]pyrazolo[1,5-a]pyrimidine.

A 4.3 portion of the above compound and 5.8 g of chloroacetic anhydride in 35 ml of toluene were reacted as described in Example 64 giving 1.5 g of the desired intermediate, mp 116-119°C.

Example 187

4-(Chloroacetyl-4,5-dihydro-7-(3-methylphenyl)pyrazolo[1,5-a]pyrimidine-3-carbonitrile

This compound is obtained from 7-(3-methylphenyl)pyrazolo[1,5-a]pyrimidine-3-carbonitrile by the procedure of Example 64.

Example 188

4,5-Dihydro-4[[4-(phenylmethyl)-1-piperazinyl]acetyl]-7-(3-methylphenyl)pyrazolo[1,5-a]pyrimidine

By the method of Example 79, N-benzyl piperazine is reacted with 4-(chloroacetyl)-4,5-dihydro-7-(3-methylphenyl)pyrazolo[1,5-a]pyrimidine-3-carbonitrile. The title compound is obtained.

Example 189

4-(Chloroacetyl-4,5-dihydro-7-[3-(trifluoromethyl)phenyl]pyrazolo[1,5-a]pyrimidine-3-carboxamide

A mixture of 2 g of 4-(chloroacetyl)-4,5-dihydro-7-[3-(trifluoromethyl)phenyl]pyrazolo[1,5-a]pyrimidine-3-carbonitrile and 20 ml of concentrated sulfuric acid was stirred at room temperature for 2 hours, then poured into ice water, stirred, and filtered. The product was washed with water and dried giving 2 g of the desired intermediate, mp 174-176°C.

Example 190

3-Bromo-4-(Chloroacetyl)4,5-dihydro-7-[3-(trifluoromethyl)phenyl]pyrazolo[1,5-a]pyrimidine

The reaction of 10.0 g of 3-bromo-7-[3-(trifluoromethyl)phenyl]pyrazolo[1,5-a]pyrimidine (U.S. Patent 4,178,449) with 4.59 g of sodium cyanoborohydride in 130 ml of acetic acid at 25°C for 5 hours gave after workup, 4.4 g of 3-bromo-4,5-dihydro-7-[3-trifluoromethyl)phenyl]pyrazolo[1,5-a]pyrimidine as yellow crystals, mp 101-103°C.

A 2.5 g portion of the above compound and 3.0 g of chloracetic anhydride in 40 ml of toluene were reacted as described in Example 64, to give 0.8 g of the title compound, m.p. 128-130°C.

Example 191

7-(3-Chlorophenyl)-4,5-dihydro-6-methyl-4[[4-[[3-(trifluoromethyl)phenyl]methyl]-1-piperazinyl]acetyl]-pyrazolo[1,5-a]pyrimidine-3-carbonitrile

The above compound was prepared by the reaction of 4-(3-trifluoromethyl)phenylmethylpiperazine with the compound of Example 75 by the procedure of Example 79, mp 133-135°C.

Example 192

4,5-Dihydro-4[[4-[(3-trifluoromethylphenylmethyl]-1-piperazinyl]acetyl]-7-[3-(trifluoromethyl)phenyl]pyrazolo-[1,5-a]pyrimidine-3-carbonitrile

The above compound was prepared by the reaction of 4-(3-trifluoromethyl)phenylmethylpiperazine with the compound of Example 64 by the method of Example 79, mp 178-180°C.

Example 193

7-(2,5-Dichlorophenyl)-4,5-dihydro-4[(4-phenyl-1-piperazinyl)acetyl]pyrazolo[1,5-a]pyrimidine-3-carbonitrile

The above compound was prepared by the reaction of 4-phenylpiperidine with the compound of Example 72 by the method of Example 79, mp 199-200°C.

Example 194

7-(2,5-Dichlorophenyl)-4,5-dihydro-4-[[4-(phenylmethyl)-1-piperazinyl]acetyl]pyrazolo[1,5-a]pyrimidine-3-carbonitrile

The above compound was prepared by the reaction of 4-benzylpiperidine with the compound of Example 72 by the procedure of Example 79, mp 157-158°C.

Example 195

7-(3-Chlorophenyl)-4-[[4-[(3-Chlorophenyl)methyl-1-piperazinyl]acetyl]4,5-dihydro-6-methylpyrazolo[1,5-a]-pyrimidine-3-carbonitrile, dihydrochloride

The above compound was prepared by the reaction of 4-(3-chlorophenyl)methylpiperazine with the compound of Example 75 by the procedure of Examples 79 then 141, mp 208-210°C.

Example 196

4,5-Dihydro-4[[4-(2-phenylethyl)-1-piperazinyl]acetyl]-7-[3-(trifluoromethyl)phenyl]pyrazolo[1,5-a]pyrimidine-3-carbonitrile, dihydrochloride

The above compound was prepared by the reaction of 4-(2-phenylethyl)piperazine with the compound of Example 64 by the methods of Examples 79 then 141, mp 235-237°C.

Example 197

7-(4-Chlorophenyl)-4[[4-(3-chlorophenyl)methyl]-1-piperazinyl]acetyl]-4,5-dihydro-6-methylpyrazolo[1,5-a]-pyrimidine-3-carbonitrile, dihydrochloride

The above compound was prepared by the reaction of 4-(3-chlorophenyl)methylpiperazine with the compound of Example 77, by the methods of Examples 79 then 141, mp 250°C (with sintering).

Example 198

4[[4-[Bis(4-fluorophenyl)methyl-1-piperazinyl]acetyl]-4,5-dihydro-7-[3-(trifluoromethyl)phenyl]pyrazolo[1,5-a]-pyrimidine-3-carbonitrile, dihydrochloride

The above compound was prepared by the reaction of 4-bis(4-fluorophenyl)methylpiperazine with the compound of Example 1 by the procedures of Examples 79 then 141, mp 255-258°C.

Example 199

4,5-Dihydro-6-methyl-4[[4-(phenylmethyl)-1-piperazinyl]acetyl]-7-[3-(trifluoromethyl)phenyl]pyrazolo[1,5-a]-pyrimidine-3-carbonitrile, dihydrochloride

The above compound was prepared by the reaction of N-benzylpiperazine with the compound of Example 73 by the methods of Example 79 then 141, mp 204-206°C.

Example 200

N-[3-[4-(Chloroacetyl)-(3-cyano-4,5-dihydropyrazolo[1,5-a]pyrimidin-7-yl]phenyl]acetamide

A mixture of 7.0 g of 3'-actamido-3-(N,N-dimethylamino)acrylophenone and 3.3 g. of 3-amino-4-cyanopyrazole in 100 ml of acetic acid was heated at reflux for 8 hours. Crystals that formed on cooling were filtered and dried to give 7.4 g. of N-[3-(3-cyanopyrazolo-[1,5-a]pyrimidin-7-yl]phenyl] acetamide, m.p. 254-256°C. This was reacted with 14.8 g of sodium cyanoborohydride in 50 ml of acetic acid to give 3.0 g of N-[3-(3-cyano-4,5-dihydropyrazolo-[1,5-a]pyrimidin-7-yl)phenyl]acetamide, m.p. 251-254°C. This is reacted with chloroacetic anhydride and sodium carbonate in toluene as in Example 66 to give the title compound.

Example 201

N-[3-[4-(Chloroacetyl)-(3-cyano-4,5-dihydropyrazolo[1,5-a]pyrimidinyl)phenyl]-N-methylacetamide

To 33.0 g of 3'-acetamido-3-dimethylaminoacrylophenone in 170 ml of dimethylformamide was added 6.8 g of 60% sodium hydride followed by 40 g of methyl iodide to give, after workup, 10.5 g of 3'-(N-methylacetamido)-3-( N,N-dimethylamino)acrylophenone, m.p. 129-131°C. This was reacted with 4.60 g of 3-amino-4-cyanaopyrazole in 150 ml of acetic acid as reflux for 16 hrs. to give 7.4 g of N-[3-(3-cyanopyrazolo[1,5-A]pyrimidinyl)phenyl]-N-methylactamide as yellow crystals, m.p. 201-203°C. This was reacted with sodium cyanoborohydride to give N[3-(3-cyano-4,5-dihydropyrazolo[1,5-a]pyrimidin-7-yl)-phenyl]-N-methylacetamide as yellow crystals, m.p. 114-120°C. This is reacted with chloroacetic anhydride and sodium carbonate in toluene as in Example 66 to give the title compound.

Example 202

N-[3-[[4-(Phenylmethyl)-1-piperazinyl]acetyl(3-cyano-4,5-dihydropyrazolo-[1,5-a]pyrimidin-7-yl]phenyl]-acetamide

The above compound is obtained from N-benzyl piperazine and N-[3-[4-(Chloroacetyl)-(3-cyano-4,5-dihydropyrazolo-[1,5-a]pyrimidin-7-yl]phenyl]acetamide by the method of Example 79.

Example 203

N-[3-[4-(Phenylmethyl)-1-piperazinyl-acetyl-(3-cyano-4,5-dihydropyrazolo-[1,5-a]pyrimidin-7-yl]phenyl]-N-methylacetamide

The above compound is obtained from N-benzyl piperazine and N-[3-[4-(chloroacetyl)-(3-cyano-4,5-dihydropyrazolo-[1,5-a]pyrimidin-7-yl]phenyl]-N-methylacetamide by the method of Example 79.

Example 204

4,5-Dihydro-6-ethyl-4[[4-(phenylmethyl)-1-piperazinyl]acetyl]7-[3-(trifluoromethyl)phenyl]pyrazolo[1,5-a]-pyrimidine-3-carbonitrile

A mixture of 3.3g of 4-(chloroacetyl-4,5-dihydro-6-ethyl-7-[3-(trifluoromethyl)phenyl]pyrazolo[1,5-a]-pyrimidine-3-carbonitrile, 1.6 ml of 1-benzylpiperazine and 1g. of sodium carbonate in 110 ml of toluene was stirred and refluxed for 18 hours. The mixture was treated with 10 ml of 5N sodium hydroxide and the organic phase was separated, dried over sodium sulfate and evaporated to give a dark oil. This was dissolved in ether and combined with ethanolic hydrogen chloride to give a white solid and this solid was recrystallized from acetonitrile to give 3.4g of the title compound, m.p. 184-186°C.

The reactants were prepared as follows.

Using the method of Example 65, 4,5-dihydro-6-ethyl-7-[3-(trifluoromethyl)phenyl]pyrazolo[1,5-a]-pyrimidine-3-carbonitrile was reacted with chloroacetyl chloride to give 4-(chloroacetyl)-4,5-dihydro-6-ethyl-7-[3-(trifluoromethyl)phenyl]pyrazolo[1,5-a]pyrimidine-3-carbonitrile, m.p. 150-152°C.

By the method of Example 64 , 6-ethyl-7-[3-(trifluoromethyl)phenyl]pyrazolo [1,5-a]pyrimidine-3-carbonitrile was reacted with sodium cyanoborohydride in acetic acid to give the 4,5-dihydro derivative, m.p. 185-187°C.

A mixture of 4.0g of 3-dimethylamino-2-ethyl-3'-trifluoromethylacrylophenone and 1.5g of 3-amino-4-cyanopyrazole in 75 ml of glacial acetic acid was refluxed for two hours to give 6-ethyl-7-[3-(trifluoromethyl)phenyl]pyrazolo[1,5-a]pyrimidine-3-carbonitrile, m.p. 135-137°C.

The reaction of 3'-trifluoromethylbutyrophenone and dimethylformamide dimethylacetal gave the above acrylophenone,as a liquid, b.p. 90-95°C (0.05 mm Hg).

To 30.0g. of 2-morpholinyl-3'-trifluoromethylphenylacetonitrile in 250 ml of N,N-dimethylformamide at 10°C was added 5.2g of 50% sodium hydride-mineral oil and to this was added 18.7g of iodopropane. The reaction mixture was stirred for 18 hours and then was poured into water and the resultant oil was collected to give 33.2g of 2-morpholinyl-2-propyl-3'-trifluoromethylphenylacetonitrile. This was hydrolyzed by heating in 70% acetic acid, followed by neutralization with sodium hydroxide to give 15.3g. of 3'-trifluoromethylbutyrophenone as a yellow liquid, b.p. 60-65°C (0.05 mm Hg).

## Claims

1. A compound of the formula:

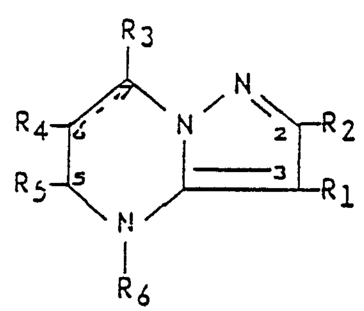

Ia or Ib

wherein ---may represent the presence of a double bond between the $C_6$ and $C_7$ position, Ia, or the absence of a double bond between the $C_6$ and $C_7$ position, Ib; $R_1$ is hydrogen, bromo, chloro, carbamoyl, carboxamido, ethylcarboxylate, carboxyl, carboxyalkoxyl where alkoxyl is ($C_1$-$C_3$), cyano, -CO-$CF_3$, COONa,

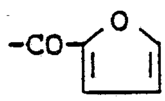

,

-CO-C($CH_3$)$_3$, or

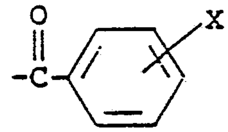

where X is hydrogen, cyano, halogen or nitro; $R_2$, $R_4$ and $R_5$ are hydrogen or lower alkyl($C_1$-$C_3$); $R_3$ is hydrogen, alkyl($C_1$-$C_3$),

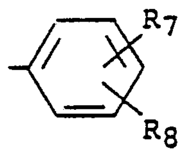

where $R_7$ and $R_8$ may be the same or different and are hydrogen, halogen, alkyl($C_1$-$C_3$), nitro, alkoxy($C_1$-$C_3$), trifluoromethyl, N-alkyl($C_1$-$C_3$)-N-alkanoxyl($C_1$-$C_3$)-amino, acetylamino or N-alkylacetylamino where alkyl is ($C_1$-$C_3$), and $R_3$ may be a monovalent radical of 3-thienyl, 2-pyridinyl, 3-pyridinyl or 4-pyridinyl, either of said pyridinyl radicals being optionally substituted with an alkyl radical $R_9$, where alkyl is ($C_1$-$C_4$), and the structures of the monovalent 2-pyridinyl, 3-pyridinyl and 4-pyridinyl moieties are depicted respectively as:

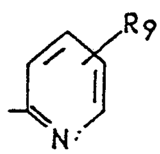 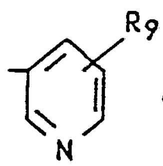 and 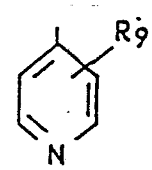 ;

$R_6$ is hydrogen, alkyl($C_1$-$C_3$) or

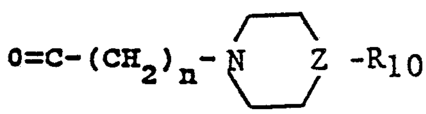

, where n is an integer from 1 to 4 inclusive; and Z is CR-, R-and -O-such that when Z is -CH, $R_{10}$ is hydrogen, ethoxycarbonyl, phenyl, phenylmethyl, phenylmethyl($C_1$-$C_6$)alkylenyl, when Z is -O-, $R_{10}$ is nil and when Z is N-, $R_{10}$ is hydrogen, alkyl-($C_1$-$C_3$), alkenyl($C_2$-$C_3$), alkynyl($C_2$-$C_3$), cycloalkyl($C_3$-$C_6$), dimethylaminoalkyl($C_1$-$C_3$), hydroxyalkyl($C_1$-$C_3$), ethylcarboxylate, alkyl($C_1$-$C_3$)carbonyl, phenyl, benzyl, mono-or disubstituted benzyl [wherein the substituents are halogen, alkoxy($C_1$-$C_3$), alkyl($C_1$-$C_3$), and trifluoromethyl], benzoyl, 4-chlorophenylmethyl, 1,3-benzodioxol-5-yl-methyl, 1,3-benzodioxl-4-yl, 2-furanyl-carboxyl, 2-pyrimidinyl, 2-pyridinyl, 4-morpholinyl-2-oxoethyl, N-(1-methylethyl)-2-oxoethyl, 1-pyrrolidinyl-2-oxoethyl, bis(4-fluorophenyl)methyl, 2-cyclohexylethyl, phenylcarboxamido, mono-and disubstituted phenylcarboxamido [wherein the substituents are trifluoromethyl, halogen and alkyl ($C_1$-$C_3$)], adamantanoyl, 3-phenoxypropyl, mono-and disubstituted phenyl [wherein the substituents are halogen, trifluoromethyl, alkoxy($C_1$-$C_3$) and alkyl($C_1$-$C_3$)], 5-chloro-2-methoxy phenylacetamide and (2-oxo-1-pyrrolidinyl)-2-butynyl and the pharmacologically acceptable acid addition salts thereof.

2. A compound according to claim 1 of the formula:

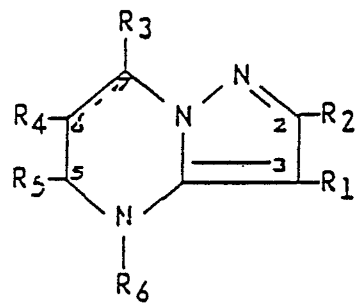

Ia or Ib

wherein ---may represent the presence of a double bond between the $C_6$ and $C_7$ position, Ia, or the absence of a double bond between the $C_6$ and $C_7$ position, Ib; $R_1$ is hydrogen, bromo, chloro, carbamoyl, carboxyl, carboxyalkoxyl where alkoxyl is ($C_1$-$C_3$), cyano, -CO-$CF_3$, COONa,

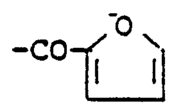 ,

-CO-$C(CH_3)_3$, or

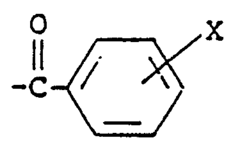

where X is hydrogen, cyano, halogen or nitro; $R_2$, $R_4$ and $R_5$ may be hydrogen or lower alkyl($C_1$-$C_3$); $R_3$ is hydrogen, alkyl($C_1$-$C_3$),

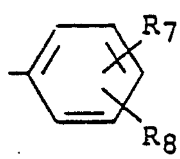

where $R_7$ and $R_8$ may be the same or different and are hydrogen, halogen, alkyl($C_1$-$C_3$), nitro, alkoxy($C_1$-$C_3$), trifluoromethyl, acetylamino or N-alkylacetylamino where alkyl is ($C_1$-$C_3$), and $R_3$ may also be a monovalent radical of 3-thienyl, 2-pyridinyl, 3-pyridinyl or 4-pyridinyl, either of said pyridinyl radicals being optionally substituted with an alkyl radical $R_9$, where alkyl is ($C_1$-$C_4$), and the structures of the monovalent 2-pyridinyl, 3-pyridinyl and 4-pryidinyl moieties are depicted respectively as:

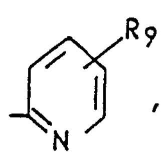 , 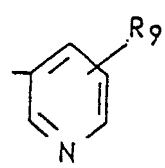 and 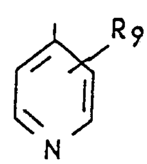

; $R_6$ is hydrogen or alkyl($C_1$-$C_3$) and the pharmacologically acceptable acid addition salts thereof.

3. A compound according to claim 1 of the formula:

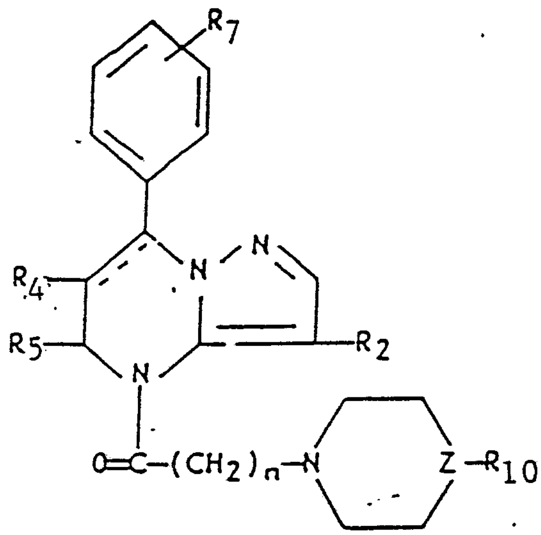

wherein n is an integer from 1 to 4 inclusive; $R_7$ is a mono-or disubstituent of hydrogen, lower alkyl($C_1$-$C_3$), lower alkoxy($C_1$-$C_3$), halogen, nitro, N -alkyl($C_1$-$C_3$)-N-alkanoyl($C_1$-$C_3$)amino, trifluoromethyl; $R_2$ is cyano, carboxamido, ethyl carboxylate or halogen; Z is CH-, N-or -O-such that when Z is CH-, $R_{10}$ is hydrogen, ethoxy carbonyl, phenyl, phenylmethyl or phenylmethyl($C_1$-$C_6$)alkylenyl, and when Z is -O-, $R_3$ is nil, and when Z is N-, $R_3$ is alkyl($C_1$-$C_3$), alkenyl($C_2$-$C_3$), alkynyl($C_2$-$C_3$), cycloalkyl($C_3$-$C_6$, dimethylaminoalkyl($C_1$-$C_3$), hydroxyalkyl($C_1$-$C_3$), ethylcarboxylate, alkyl($C_1$-$C_3$)carbonyl, phenyl, benzyl, mono-or disubstituted benzyl [wherein the substituents halogen, alkoxy($C_1$-$C_3$), alkyl($C_1$-$C_3$), or trifluoromethyl], benzoyl, 4-chlorophenyl-phenylmethyl, 1,3-benzodioxol-5-ylmethyl, 1,3-benzodioxol-5-yl, 2-furanylcarbonyl, 2-pyrimidinyl, 2-pyridinyl, 4-morpholinyl-2-oxoethyl, N-(1-methylethyl)-2-oxoethyl, 1-pyrrolidinyl-2-oxoethyl, bis(4-fluorophenyl)methyl, 2-cyclohexylethyl, phenylcarboxamido, mono-or disubstituted phenylcarboxamido [wherein the substituents are trifluoromethyl, halogen or alkyl($C_1$-$C_3$)], adamantanoyl, 3-phenoxypropyl, mono-and disubstituted phenyl [wherein the substituents are halogen, trifluoromethyl, alkoxy($C_1$-$C_3$) or alkyl($C_1$-$C_3$)], 5-chloro-2-methoxyphenylacetamide or (2-oxo-1-pyrrolidinyl)-2-butynyl; $R_4$ and $R_5$ are each hydrogen or alkyl($C_1$-$C_3$); and the pharmacologically acceptable acid addition salts thereof.

4. A compound according to claim 3 selected from those of the following formula:

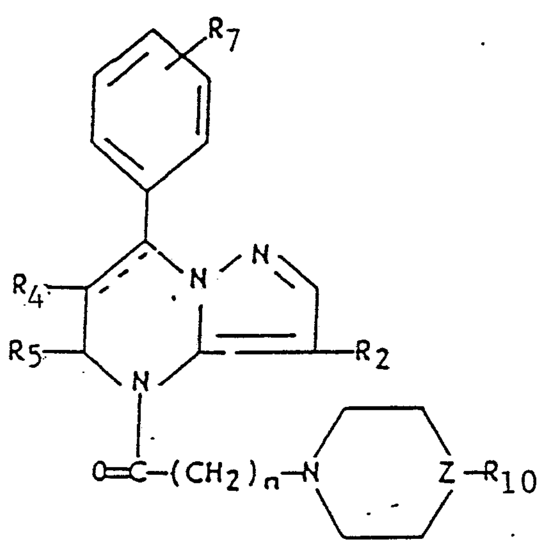

wherein n is an integer from 1 to 4 inclusive; $R_7$ represents a mono-or disubstitutent of hydrogen, lower alkyl($C_1$-$C_3$), lower alkoxy ($C_1$-$C_3$), halogen, nitro or trifluoromethyl; $R_2$ is cyano, carboxamido, ethyl carboxylate or halogen; $R_{10}$ is hydrogen, alkyl($C_1$-$C_3$), alkenyl($C_2$-$C_3$), alkynyl($C_2$-$C_3$), cycloalkyl($C_3$-$C_6$), dimethylaminoalkyl($C_1$-$C_3$), hydroxyalkyl($C_1$-$C_3$), ethylcarboxylate, alkyl($C_1$-$C_3$)carbonyl, phenyl, benzyl, mono-or disubstituted benzyl [wherein the substitutents are halogen, alkoxy($C_1$-$C_3$) or trifluoromethyl], benzoyl, 4-chlorophenylphenylmethyl, 1,3-benzodioxol-5-yl-methyl, 1,3-benzodioxol-5-yl, 2-furanylcarbonyl, 2-pyrimidinyl, 2-pyridinyl, 4-morpholin-yl-2-oxoethyl, N-(1-methylethyl)-2-oxoethyl, 1-pyrrolidinyl-2-oxoethyl, bis(4-fluorophenyl)methyl, 2-cyclohexylethyl, phenylcarboxamido [wherein the substituents are trifluoromethyl, halogen or alkyl($C_1$-$C_3$)], adamantanoyl, 3-phenoxypropyl, mono-or disubstituted phenyl [wherein the substituents are halogen, trifluoromethyl, alkoxy($C_1$-$C_3$)], 5-chloro-2-methoxyphenylacetamide or (2-oxo-1-pyrrolidinyl)-2-butynyl; $R_4$ and $R_5$ are hydrogen or alkyl($C_1$-$C_3$); and the pharmacologically acceptable acid addition salts thereof.

5. A compound according to Claim 1 selected from those of the following formula:

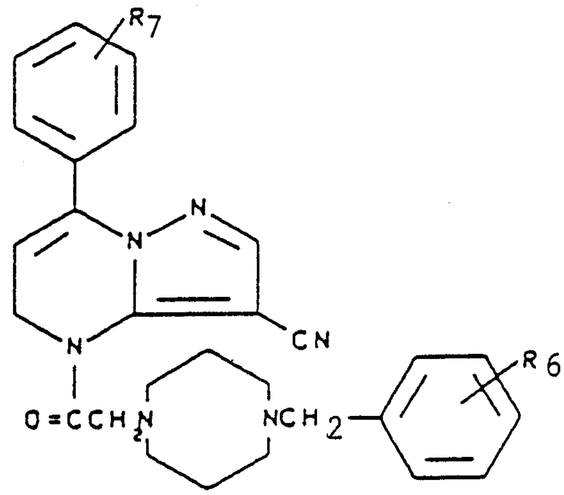

wherein $R_1$ represents a mono-or disubstituent of hydrogen, alkyl($C_1$-$C_3$), Alkoxy($C_1$-$C_3$), halogen, nitro or trifluoromethyl; and $R_6$ is hydrogen, alkyl($C_1$-$C_3$), alkoxy($C_1$-$C_3$), chloro, fluoro or trifluoromethyl.

6. A therapeutic composition of matter in dosage unit form comprising from about 5 to about 200 mg of a compound of Claim 2 in association with a pharmaceutically acceptable carrier or diluent.

7. A process for preparing a compound of Claim 2, which comprises reacting a compound of the formula:

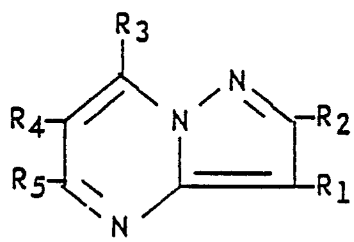

wherein $R_1$, $R_2$, $R_3$, $R_4$, and $R_5$ are as defined in Claim 2, with a suitable reducing agent such as an alkali metal borohydride, such as lithium borohydride, sodium borohydride, or sodium cyanoborohydride, or hydrogen in the presence of a catalyst such as palladium, platinum, activated nickel, activated cobalt, or the like; or by the use of a trialkylsilane such as triethylsilane in the presence of a proton donor such as trifluoroacetic acid when the compound of the hereinabove formula is in the 4,5-dihydroform.

8. A process for preparing a compound of Claim 2, wherein $R_1$, $R_2$, $R_3$, $R_4$, $R_5$ and $R_6$ are as defined in Claim 2, which comprises reacting a compound of the formula:

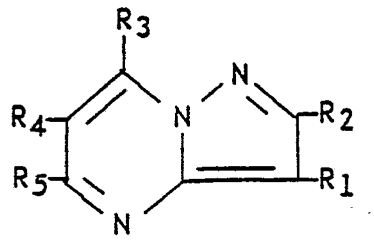

with a compound of the formula: $NaBH_3CN$ in the presence of glacial acetic acid, under nitrogen, in an ice bath for about one hour, then at room temperature for from 1-12 hours, the resulting precipitate being washed with water, dissolved in an inert solvent such as dichloromethane or acetonitrile and the like, neutralized with aqueous saturated sodium bicarbonate and then recovering the dihydro product Ia of Claim 2 from the organic phase;

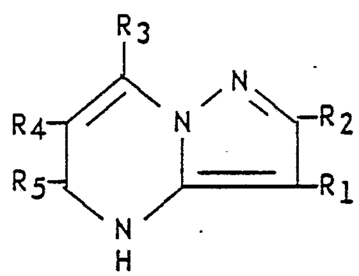

Ia

then further reducing the dihydro product Ia by reacting it with a compound of the formula: $(C_2H_5)_3SiH$ in trifluoroacetic acid at 60°C for 1-24 hours; and precipitating the tetrahydro product Ib with aqueous potassium hydroxide at pH 9.0 and recovering the product Ib therefrom;

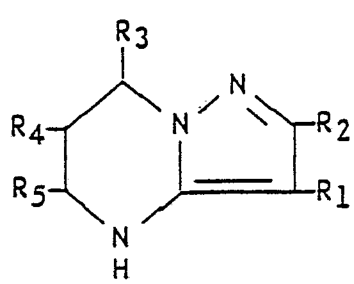

Ib

then dissolving the dihydro product Ia or the tetrahydro product Ib in a solvent such as N, H - dimethylformamide and reacting with an alkylating agent such as methyl iodide or dimethyl sulfate or the like in the presence of sodium hydride provides the product where $R_6$ is alkyl.

9. A therapeutic composition of matter in dosage unit form comprising form about 10 mg to about 500 mg per dosage unit of a compound of Claim 3, in association with a pharmaceutically acceptable carrier or diluent.

10. A process for producing a compound of the formula:

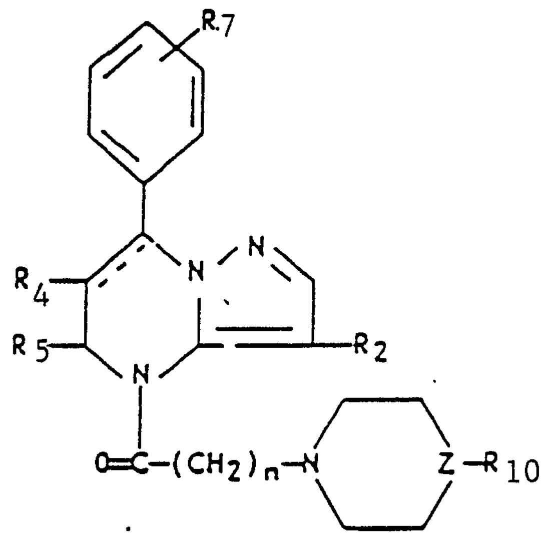

wherein n, $R_2$, $R_4$, $R_5$, $R_{10}$ and Z are as defined in Claim 3 which comprises reacting an amine of the formula:

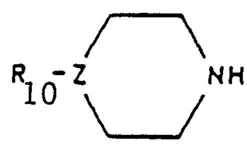

wherein $R_{10}$ and z are as described in Claim 3 with a compound of the formula:

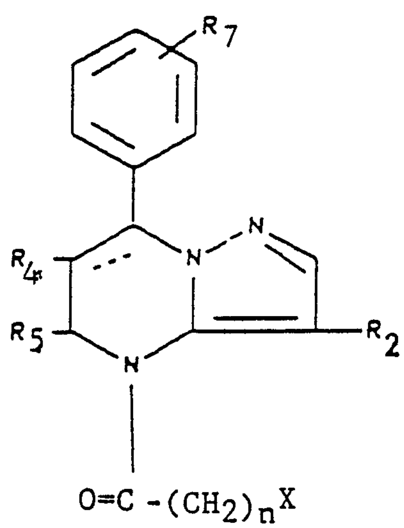

wherein $R_7$, $R_2$, $R_4$, $R_5$ and n are as described in Claim 3 and X is chloro or iodo.

European Patent Office

# EUROPEAN SEARCH REPORT

Application Number

EP 87 11 4904

## DOCUMENTS CONSIDERED TO BE RELEVANT

| Category | Citation of document with indication, where appropriate, of relevant passages | Relevant to claim | CLASSIFICATION OF THE APPLICATION (Int. Cl.3) |
|---|---|---|---|
| A | EP-A-0 129 847 (AMERICAN CYANAMID CO.) * claim 1 * --- | 1 | C 07 D 487/04<br>A 61 K 31/495//<br>(C 07 D 487/04<br>C 07 D 239:00<br>C 07 D 231:00 ) |
| A | EP-A-0 025 819 (AMERICAN CYANAMID CO.) * claim 1 * & US - A - 42 81000 (Cat. D,A) --- | 1 | |
| A | US-A-4 521 422 (J.P. DUSZA et al.) * claim 1 * --- | 1 | |
| D,A | US-A-4 236 005 (J.P. DUSZA et al.) * claim 1 * --- | 1 | |
| A | CHEMICAL ABSTRACTS, vol. 95, no. 24, 14th December 1981, page 554, abstract no. 211886t, Columbus, Ohio, US; C. BELLEC et al.: "Deaminative electrochemical reduction of pyrazolo[1,5-a]pyrimidine-7-amines", & CAN. J. CHEM. 1981, 59(19), 2826-2832 --- | 1 | TECHNICAL FIELDS SEARCHED (Int. Cl.3) |
| A | CHEMICAL ABSTRACTS, vol. 67, no. 19, 6th November 1967, pages 8556-8557, abstract no. 90833j, Columbus, Ohio, US; & JP - A - 67 3172 (SHIONOGI & CO., LTD.) 10-02-1967 --- | 1 | C 07 D 487/00 |
| D,A | JOURNAL OF MEDICINAL CHEMISTRY, vol. 20, no. 3, 1977, pages 386-393; W.E. KIRKPATRICK et al.: "3-Halo-5,7-dimethylpyrazolo[1,5-a]pyrimidines, a nonbenzodiazepinoid class of antianxiety agents devoid of potentiation of central nervous system depressant effects of Ethanol or barbiturates" --- -/- | | |

The present search report has been drawn up for all claims

| Place of search | Date of completion of the search | Examiner |
|---|---|---|
| BERLIN | 12-01-1988 | HASS C V F |

CATEGORY OF CITED DOCUMENTS

X : particularly relevant if taken alone
Y : particularly relevant if combined with another document of the same category
A : technological background
O : non-written disclosure
P : intermediate document

T : theory or principle underlying the invention
E : earlier patent document, but published on, or after the filing date
D : document cited in the application
L : document cited for other reasons

& : member of the same patent family, corresponding document

EPO FORM 1503 03.82 (P0401)

European Patent Office

# EUROPEAN SEARCH REPORT

Application Number

EP 87 11 4904

## DOCUMENTS CONSIDERED TO BE RELEVANT

| Category | Citation of document with indication, where appropriate, of relevant passages | Relevant to claim | CLASSIFICATION OF THE APPLICATION (Int. Cl.3) |
|---|---|---|---|
| D,A | SYNTHESIS, August 1982, pages 673-677, Stuttgart, New York; G. MÜHMEL et al.: "Pyrazolo[1,5-a]pyrimidine aus 3-Alkoxy-acroleinen und 5-Amino-1H-pyrazolen" --- | | |
| D,A | JOURNAL OF ORGANIC CHEMISTRY, vol. 44, no. 26, 1979, pages 4809-4813; A.E. LANZILOTTI et al.: "Stereoselective reduction of some indoles with triethylsilane-trifluoracetic acid" ----- | 7 | |
| | | | TECHNICAL FIELDS SEARCHED (Int. Cl.3) |

The present search report has been drawn up for all claims

| Place of search | Date of completion of the search | Examiner |
|---|---|---|
| BERLIN | 12-01-1988 | HASS C V F |

CATEGORY OF CITED DOCUMENTS

X : particularly relevant if taken alone
Y : particularly relevant if combined with another document of the same category
A : technological background
O : non-written disclosure
P : intermediate document

T : theory or principle underlying the invention
E : earlier patent document, but published on, or after the filing date
D : document cited in the application
L : document cited for other reasons

& : member of the same patent family, corresponding document

EPO FORM 1503 03.82 (P0401)